# EUROPEAN PATENT SPECIFICATION

(11) **EP 1 857 466 B1**
(45) Date of publication and mention of the grant of the patent: **20.10.2010**
(21) Application number: 07012370.8
(22) Date of filing: 22.12.2000
(51) Int. Cl.: C07K 14/715, C12N 15/62, C12N 15/09, C07K 16/00, A61P 17/06

(54) **Soluble interleukin-20 receptor**
Löslicher Interleukin-20-Rezeptor
Récepteur d'interleukin-20

(30) Priority: 23.12.1999 US 471774; 22.06.2000 US 213416 P
(43) Date of publication of application: 21.11.2007
(62) Divisional of application: 00986743.3
(73) Proprietor: ZymoGenetics, Inc., Seattle, WA 98102 (US)
(72) Inventor: Foster, Donald C., Lake Forest Park, WA 98155 (US); Xu, Wenfeng, Seattle, WA 98115 (US); Madden, Karen L., Bellevue, WA 98004 (US); Kelly, James D., Mercer Island, WA 98040 (US); Sprecher, Cindy A., Hereford, AZ 85615 (US); Brandt, Cameron S., Seattle, WA 98115 (US); Rixon, Mark W., Issaquah. WA 98029 (US); Presnell, Scott R., Tacoma, WA 98407 (US); Fox, Brian A., Seattle, Washington 98105 (US)
(74) Representative: MacLean, Martin Robert

(56) References cited:
- WO-A-00/39161
- WO-A-01/46232
- WO-A-01/46261
- WO-A-99/03982
- WO-A-99/37772
- WO-A-99/46281
- WO-A-99/46379
- WO-A-99/61630
- US-A- 5 945 511
- BLUMBERG H ET AL: "Interleukin 20: Discovery, receptor identification, and role in epidermal function" CELL, CELL PRESS, CAMBRIDGE, NA, US, vol. 104, no. 1, 12 January 2001 (2001-01-12), pages 9-19, XP002210052 ISSN: 0092-8674
- PARRIS-NOVAK J ET AL: "INTERLEUKINS 19, 20, AND 24 SIGNAL THROUGH TWO DISTINCT RECEPTOR COMPLEXES" JOURNAL OF BIOLOGICAL CHEMISTRY, AMERICAN SOCIETY OF BIOLOCHEMICAL BIOLOGISTS, BIRMINGHAM,, US, vol. 277, no. 49, 6 December 2002 (2002-12-06), pages 47517-47523, XP001167074 ISSN: 0021-9258

## Description

Cytokines are soluble proteins that influence the growth and differentiation of many cell types. Their receptors are composed of one or more integral membrane proteins that bind the cytokine with high affinity and transduce this binding event to the cell through the cytoplasmic portions of the certain receptor subunits. Cytokine receptors have been grouped into several classes on the basis of similarities in their extracellular ligand binding domains. For example, the receptor chains responsible for binding and/or transducing the effect of interferons (IFNs) are members of the type II cytokine receptor family (CRF2), based upon a characteristic 200 residue extracellular domain. The demonstrated *in vivo* activities of these interferons illustrate the enormous clinical potential of, and need for, other cytokines, cytokine agonists, and cytokine antagonists. Some cytokines are involved in the inflammatory cascade and can promote such diseases as rheumatoid arthritis, Crohn's disease, psoriasis, heart disease etc. Thus, there is a need to discover cytokines and their receptors that are involved in inflammation. One can then use the isolated soluble receptors of the cytokine to inhibit the cytokine-mediated inflammation.

WO 99/03982 describes IL-20. WO 01/46232 describes a soluble IL-20 receptor. WO 99/37772 describes IL-18 receptors.

WO 00/39161 describes Class II cytokine receptor-like proteins and nucleic acids encoding them. US 5945511 describes a Class II cytokine receptor. WO 99/46281 describes a polypeptide and nucleic acids encoding the same. WO 99/46379 describes human receptor proteins, related reagents and methods. WO 99/61630 describes chimeric molecules comprising an extracellular ligand binding domain of a receptor and an IgE Fc or constant region, and their use in an assay system. WO 01/46261 describes a method for treating inflammation.

Blumberg H et al., Cell, Vol. 104, No. 1, 12 January 2001, pages 9-19, describes the IL-20, its receptor and its role in epidermal function. Parrish-Novak J. et al., J. Biol. Chem., Vol. 277, No. 49, 6 December 2002, pages 47517-47523 reports that IL-19, IL-20 and IL-24 signal through two distinct receptor complexes.

The present invention provides a polynucleotide that encodes a receptor comprising an IL-20RA subunit and an IL-20RB subunit, wherein the IL-20RA subunit comprises a polypeptide comprising an amino acid sequence selected from the group consisting of SEQ ID NOs: 12, 38, 55, 63, and 65, and the IL-20RB subunit comprises a polypeptide comprising an amino acid sequence selected from the group consisting of SEQ ID NOs: 15, 59, 61, 67, 68 and 69, and wherein the IL-20RA subunit and the IL-20RB subunit are covalently linked together by a linkage selected from one or more polypeptide linkers and one or more disulphide bonds.

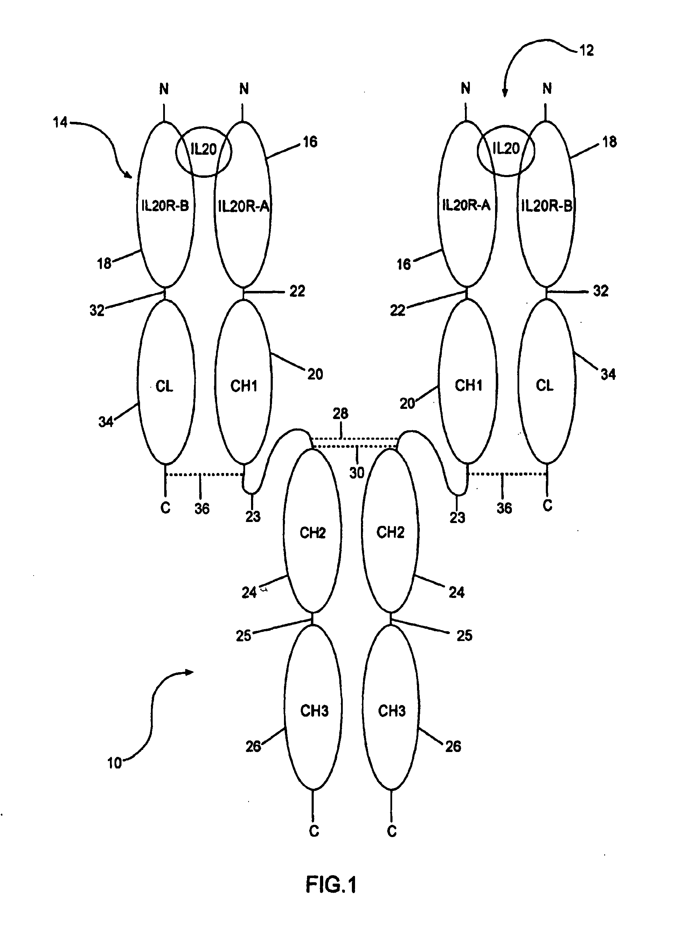

Figures 1-8 are schematic representations of different embodiments of the soluble receptor of the present invention.

The present invention thus provides means for the expression and production of a newly discovered soluble receptor to Interleukin-20 (IL-20). The soluble receptor can be used to downregulate IL-20 and thus treat inflammatory diseases such as psoriasis and inflammatory lung diseases.

IL-20 was formally called 'Zcyto10' (International Patent Publication No. WO/9927103) and has the amino acid sequences of SEQ ID NOs: 1-9. The receptor to IL-20 is comprised of two chains, an alpha chain and a beta chain. The alpha chain, hereinafter referred to as IL-20RA, was formally called ZcytoR7. See U.S. Patent No. 5,945,511. The beta chain, hereinafter referred to as IL-20RB, was formally called DIRS1. See International Patent Application No. PCT/US99/03735. The soluble receptor described herein is comprised of the extracellular domain of IL-20RA and the extracellular domain of IL-20RB.

In one embodiment the IL-20RA subunit is fused to the constant region of the heavy chain of an immunoglobulin (Ig) molecule or a portion thereof and the IL-20RB subunit is fused to the constant region of the light chain of an Ig molecule such that the constant region of the light chain is disulfide bonded to the constant region of the heavy chain, generally to a cysteine residue on the hinge region of the heavy chain. Also the opposite can occur, the IL-20RA subunit can be fused to the constant region of the light chain of an Ig molecule and the IL-20RB subunit can be fused to the constant region of the heavy chain of an Ig molecule.

In one embodiment of the soluble receptor, the IL-20RA subunit fused to the constant region of the heavy chain is comprised of an amino acid sequence selected from the group consisting of SEQ ID NOs: 23, 53, 54 and 62, and the IL-20RB subunit fused to the constant region of the light chain of the Ig molecule is comprised of an amino acid sequence selected from the group consisting of SEQ ID NOs: 21, 57, 58, and 60.

Also disclosed is a protein having a first polypeptide and a second polypeptide wherein the first polypeptide is comprised of an amino acid sequence of SEQ ID NO: 66 and the second polypeptide is comprised of an amino acid sequence selected from the group consisting of SEQ ID NOs: 70 and 71. The resultant protein can be used to generate antibodies to the IL-20RA subunit and the IL-20RB subunit. **Definitions**

Prior to setting forth the invention in more detail, it may be helpful to the understanding thereof to define the following terms.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

As used herein, the term "antibody fusion protein" refers to a recombinant molecule that comprises an antibody component and a therapeutic agent. Examples of therapeutic agents suitable for such fusion proteins include immunomodulators ("antibody-immunomodulator fusion protein") and toxins ("antibody-toxin fusion protein").

The term "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like. Where subsequent dissociation of the complement/anti-complement pair is desirable, the complement/anti-complement pair preferably has a binding affinity of <10⁹ M⁻¹.

The term "complements of a polynucleotide molecule" is a polynucleotide molecule having a complementary base sequence and reverse orientation as compared to a reference sequence. For example, the sequence 5' ATGCACGGG 3' is complementary to 5' CCCGTGCAT 3'.

The term "contig" denotes a polynucleotide that has a contiguous stretch of identical or complementary sequence to another polynucleotide. Contiguous sequences are said to "overlap" a given stretch of polynucleotide sequence either in their entirety or along a partial stretch of the polynucleotide. For example, representative contigs to the polynucleotide sequence 5'-ATGGCTTAGCTT-3' are 5'-TAGCTTgagtct-3' and 3'-gtcgacTACCGA-5'.

The term "degenerate nucleotide sequence" denotes a sequence of nucleotides that includes one or more degenerate codons (as compared to a reference polynucleotide molecule that encodes a polypeptide). Degenerate codons contain different triplets of nucleotides, but encode the same amino acid residue (i.e., GAU and GAC triplets each encode Asp).

The term "expression vector" is used to denote a DNA molecule, linear or circular, that comprises a segment encoding a polypeptide of interest operably linked to additional segments that provide for its transcription. Such additional segments include promoter and terminator sequences, and may also include one or more origins of replication, one or more selectable markers, an enhancer, a polyadenylation signal, etc. Expression vectors are generally derived from plasmid or viral DNA, or may contain elements of both.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, Nature 316:774-78 (1985).

An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. When used in this context, the term "isolated" does not exclude the presence of the same polypeptide in alternative physical forms, such as dimers or alternatively glycosylated or derivatized forms.

The term "operably linked", when referring to DNA segments, indicates that the segments are arranged so that they function in concert for their intended purposes, *e.g.*, transcription initiates in the promoter and proceeds through the coding segment to the terminator.

A "polynucleotide" is a single- or double-stranded polymer of deoxyribonucleotide or ribonucleotide bases read from the 5' to the 3' end. Polynucleotides include RNA and DNA, and may be isolated from natural sources, synthesized *in vitro,* or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired. Such unpaired ends will in general not exceed 20 nucleotides in length.

A "polypeptide" is a polymer of amino acid residues joined by peptide bonds, whether produced naturally or synthetically. Polypeptides of less than about 10 amino acid residues are commonly referred to as "peptides".

The term "promoter" is used herein for its art-recognized meaning to denote a portion of a gene containing DNA sequences that provide for the binding of RNA polymerase and initiation of transcription. Promoter sequences are commonly, but not always, found in the 5' non-coding regions of genes.

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

The term "receptor" denotes a cell-associated protein that binds to a bioactive molecule (i.e., a ligand) and mediates the effect of the ligand on the cell. Membrane-bound receptors are characterized by a multi-domain structure comprising an extracellular ligand-binding domain and an intracellular effector domain that is typically involved in signal transduction. Binding of ligand to receptor results in a conformational change in the receptor that causes an interaction between the effector domain and other molecule(s) in the cell. This interaction in turn leads to an alteration in the metabolism of the cell. Metabolic events that are linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids. In general, receptors can be membrane bound, cytosolic or nuclear, monomeric (*e.g*., thyroid stimulating hormone receptor, beta-adrenergic receptor) or multimeric (*e.g.*, PDGF receptor, growth hormone receptor, IL-3 receptor, GM-CSF receptor, G-CSF receptor, erythropoietin receptor and IL-6 receptor).

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

The term "splice variant" is used herein to denote alternative forms of RNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a protein encoded by a splice variant of an mRNA transcribed from a gene.

Molecular weights and lengths of polymers determined by imprecise analytical methods (*e.g.*, gel electrophoresis) will be understood to be approximate values. When such a value is expressed as "about" X or "approximately" X, the stated value of X will be understood to be accurate to ±10%.

As was stated above, IL-20 (formally called Zcyto10) is defined and methods for producing it and antibodies to IL-20 are contained in International Patent Application No. PCT/US98/25228, publication no. WO 99/27103,published November 25, 1998 and U.S. Patent Application No. 09/313,458 filed May 17, 1999. The polynucleotide and polypeptide of human IL-20 are represented by SEQ ID NOs: 1 - 4, and mouse IL-20 by SEQ ID NOs: 5-9.

The receptor to IL-20 has been discovered and is a heterodimer comprised of the polypeptide termed 'IL-20RA' (formally called Zcytor7) and a polypeptide termed 'IL-20RB'. The IL-20RA polypeptide, nucleic acid that encodes it, antibodies to IL-20RA, and methods for producing it are disclosed in U.S. Patent No. 5,945,511 issued August 31, 1999. SEQ ID NOs: 10 - 12 are the IL-20RA polynucleotides and polypeptides. The extracellular domain of the human IL-20RA is comprised of a polypeptide selected from the group consisting of SEQ ID NOs: 12, 55, 63 and 65, the full-length receptor subunit being comprised of SEQ ID NO: 11. The extracellular domain of mouse IL-20RA is SEQ ID NO: 38, SEQ ID NO: 37 being the entire mouse IL-20RA.

The extracellular domain of IL-20RB (SEQ ID NOs: 13-14, and a variant SEQ ID NOs: 18 and 19) is comprised of a polypeptide selected from the group consisting of SEQ ID NOs: 15, 59, 61, 67, 68 and 69. Preferably, the extracellular domain of the IL-20RA polypeptide and the extracellular domain of the IL-20RB polypeptide are covalently linked together. In a preferred embodiment one extracellular subunit polypeptide has a constant region of a heavy chain of an immunoglobulin fused to its carboxy terminus and the other extracellular subunit has a constant light chain of an immunoglobulin (Ig) fused to its carboxy terminus such that the two polypeptides come together to form a soluble receptor and a disulfide bond is formed between the heavy and the light Ig chains. In another method, a peptide linker could be fused to the two carboxy-termini of the polypeptides to form a covalently bonded soluble receptor.

SEQ ID NOs: 22 and 23 are constructs of the extracellular domain of IL-20RA fused to a mutated human immunoglobulin gamma 1 constant region produced according to the procedure set forth in example 5. SEQ ID NO: 62 is the predicted mature sequence without the signal sequence. SEQ ID NOs: 20 and 21 are constructs of the extracellular domain of IL-20RB fused to wild type human immunoglobulin kappa light chain constant region produced according to the procedure of example 5. SEQ ID NO: 60 is the predicted mature sequence without the signal sequence. Figure 1 depicts the heterotetramer produced by example 5.

SEQ ID NOs: 52 and 53 are constructs of the extracellular domain of IL-20RA fused to a mutated human immunoglobulin gamma 1 constant region produced according to the procedure set forth in example 12. SEQ ID NO: 54 is the predicted mature sequence without the signal sequence. SEQ ID NOs: 56 and 57 are constructs of the extracellular domain of IL-20RB fused to wild type human immunoglobulin kappa light chain constant region produced according to the procedure of example 12. SEQ ID NO: 58 is the predicted mature sequence without the signal sequence. The resultant heterotetramer is almost identical to that produced by example 5, the primary difference being the absence of a polypeptide linker between the extracellular domains and the beginning of the Ig constant regions, 22 in Figure 1. Hereinafter, the term "extracellular domain of a receptor" means the extracellular domain of the receptor or a portion of the extracellular domain that is necessary for binding to its ligand, in this case the ligand being IL-20.

One can link together the extracellular domains of IL-20RA and IL-20RB in a number of ways such that the resultant soluble receptor can bind to IL-20. Figures 1-8 illustrate a representative number of embodiments of the present invention. Common elements in each of the drawings are given the same number. Figure 1 represents the embodiment of the present invention produced according to example 5 below. The soluble receptor construct, designated 10, is comprised of two IL-20 binding site polypeptide chains designated 12 and 14. Each binding site is comprised of the extracellular domain of IL-20RA, designated 16, and the extracellular domain of IL-20RB designated 18.

The extracellular domain, 16, of IL-20RA is linked to the constant heavy one (CH1) domain, 20, of the human immunoglobulin gamma 1 heavy chain constant region via linker 22, which is SEQ ID NO:72. The CH1 domain, 20, is then linked to the CH2 domain, 24, via hinge region 23. The CH2 domain, 24, is linked to the CH3 domain, 26, via hinge region 25.

Comparing the construct of Figure 1 with SEQ ID NO:22, the extracellular domain, 16, of IL-20RA extends from amino acid residues 36, a valine, to and including amino acid residue 249, a glutamine of SEQ ID NO:22. Polypeptide linker, 22, extends from amino acid residue 250, a glycine to and including amino acid residue 264, a serine, of SEQ ID NO:22. The CH1 domain, 22 of Figure 1, extends from amino acid residue 265, an alanine, to and including amino acid residue 362, a valine, of SEQ ID NO:22. Hinge region 23 of Figure 1 extends from amino acid residue 363, a glutamic acid to and including amino acid residue 377, a proline, of SEQ ID NO: 22. Chains 12 and 14 are disulfide-bonded together by means of disulfide bonds 28 and 30. The disulfide bonds are formed between the heavy chains by the cysteine residues at positions 373 and 376 of SEQ ID NO: 22 of each of the two heavy chains.

Extracellular domain, 18, of IL-20RB is linked to the constant region of the human kappa light chain (CL), 34 of Figure 1 via polypeptide linker 32, which is the polypeptide SEQ ID NO: 72. The extracellular domain, 18, of IL-20RB extends from amino acid residue 30, a valine, to and including amino acid residue 230, an alanine, of SEQ ID NO: 20. Polypeptide linker, 32, extends from amino acid residue 231, a glycine, to and including amino acid residue 245, a serine, of SEQ ID NO:20. The kappa constant light region, 34, extends from amino acid residue 246, an arginine, to and including the final amino acid residue 352, a cysteine, of SEQ ID NO:20. The cysteine at position 352 of SEQ ID NO: 20 forms a disulfide bond, 36 in Figure 1, with the cysteine at position 367 of SEQ ID NO: 22. The constant light chain 34 is thus linked to the hinge region, 23, by disulfide bond, 36. In this way, the extracellular domain, 16, of IL-20RA is linked to the extracellular domain, 18, of IL-20RB to form a soluble receptor.

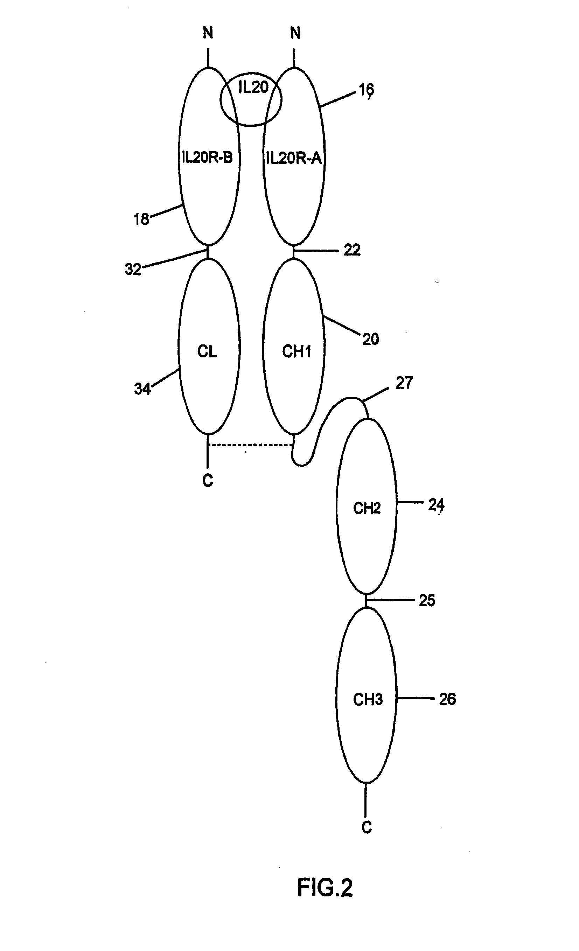

If the cysteine residues at positions 373 and 376 of SEQ ID NO:22 were changed to different amino acid residues, the two IL-20 binding polypeptides, 12 and 14, would not be disulfide bonded together and would form a construct shown in Figure 2. having hinge region, 27.

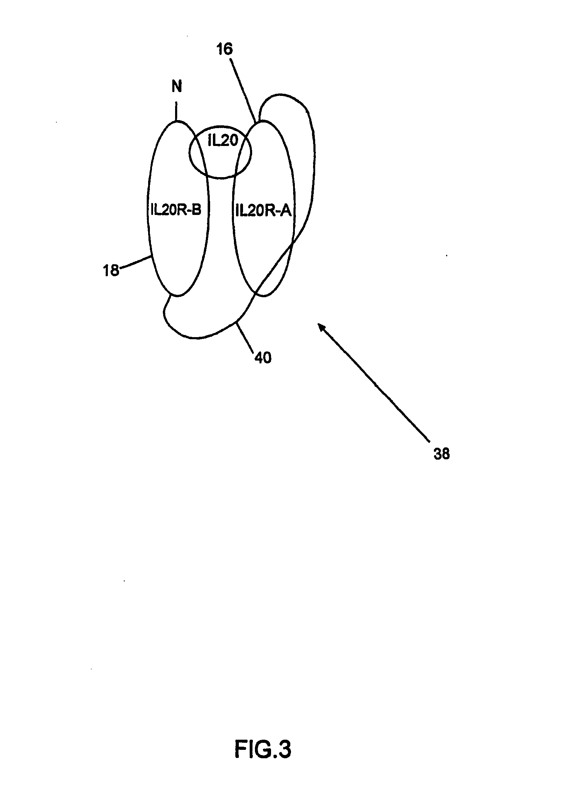

Figure 3 shows a very simple soluble receptor 38 of the present invention wherein extracellular domain, 16, of IL-20RA is connected to the extracellular domain, 18, of IL-20RB by means of a polypeptide linker, 40. The polypeptide linker extends from the amino terminus of extracellular domain, 16, of IL-20RA and is connected to the carboxyl terminus of the extracellular domain, 18, of IL-20RB. The polypeptide linker should be between 100-240 amino acids in length, preferably about 170 amino acid residues in length. A suitable linker would be comprised of glycine and serine residues. A possible linker would be multiple units of SEQ ID NO: 72, preferably about 12.

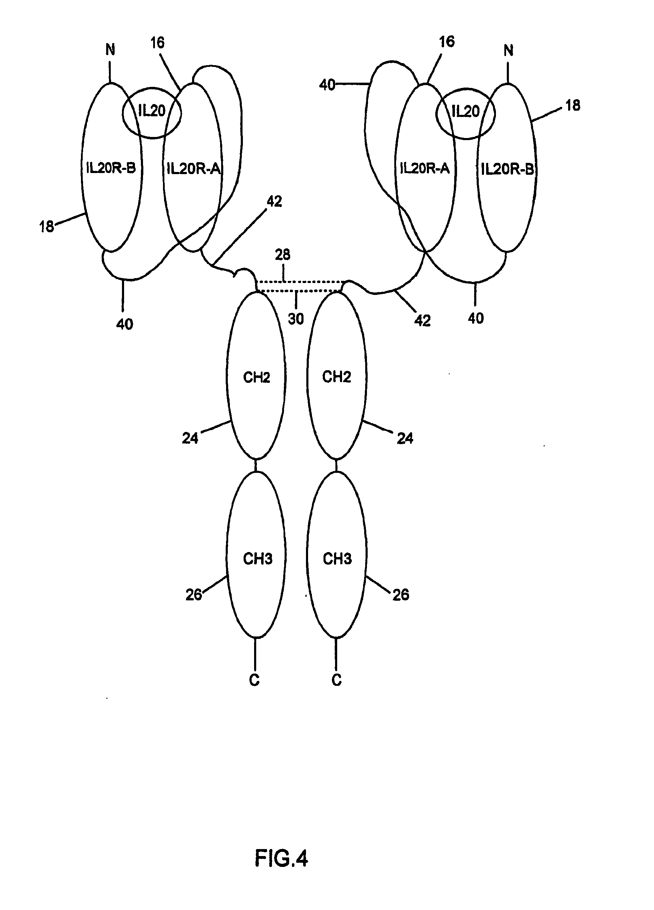

Figure 4 shows an embodiment that has the extracellular domain, 16, of IL-20RA linked to the extracellular domain, 18, of IL-20RB by means of linker 40, as in Figure 3. While the extracellular domain, 16, of IL-20RA is linked to the CH1 domain, 20, as in Figure 1 by means of polypeptide linker 42, which should be about 30 amino acid residues in length. An ideal linker would be comprised of glycine and serine as in SEQ ID NO: 72, and the hinge sequence, 23 of Figure 1.

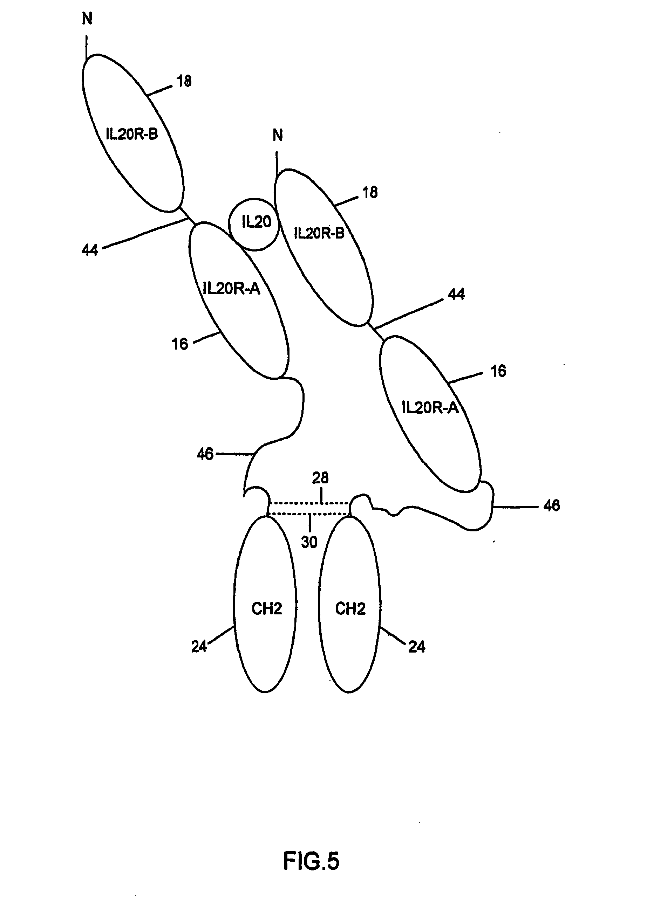

Figure 5 shows another possible embodiment of the present invention. In this embodiment, a polypeptide linker 44 of about 15 amino acid residue, *e.g.* SEQ ID NO: 72, links the carboxyl terminus of the extracellular domain, 18, of IL-20RB with the amino terminus of the extracellular domain, 16, of IL-20RA. A polypeptide linker 46 of about 30 amino acid residues extends from the carboxy terminus of the extracellular domain, 16, of IL-20RA to the CH2 domain. The carboxyl terminus of linker 46 would preferably be comprised of the hinge region extending from amino acid residue 363, a glutamic acid to and including amino acid residue 377, a proline, of SEQ ID NO: 22. Nonetheless, polypeptide linker 46 would ideally have at least one cysteine residue at its carboxyl terminus so a disulfide bond could be formed.

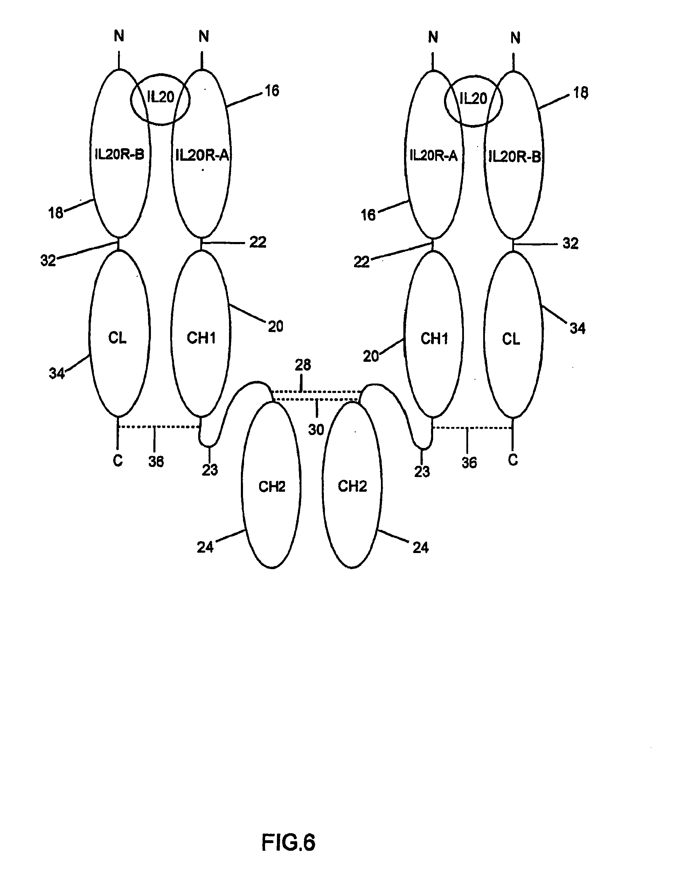

The soluble IL-20 receptor of Figure 6 is identical to that of Figure 1 except for the CH3 domain, 26 of Figure 1, is not present on the embodiment of Figure 6. The CH3 region begins at amino acid residue 488, a glycine, and extends to the last residue 594 of SEQ ID NO: 22.

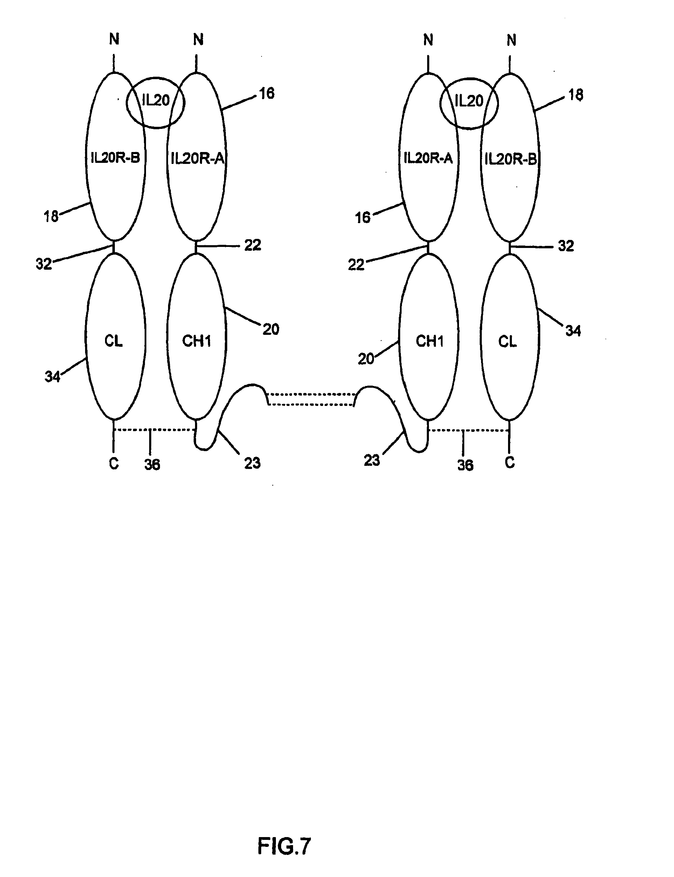

Figure 7 shows a soluble IL-20 receptor construct that is identical to the construct of Figure 1 except both the CH2, and CH3 domains are absent. The CH2 and CH3 domains run from amino acid residue 378, an alanine, to the end of the polypeptide sequence of SEQ ID NO: 22.

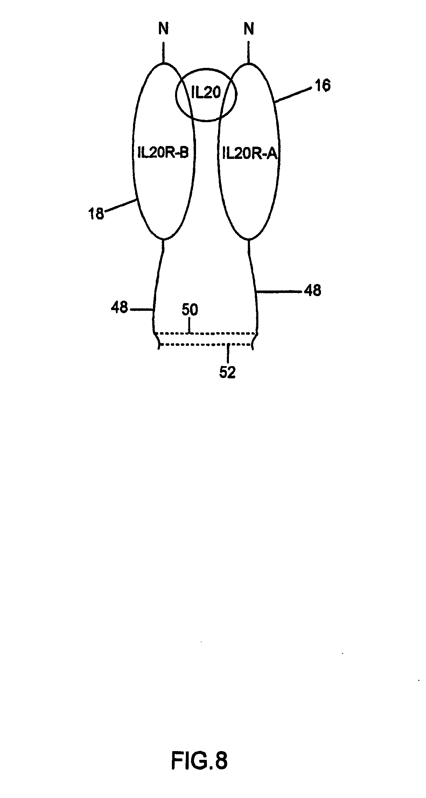

Figure 8 shows a construct wherein both IL-20RA, 16, and IL-20RB have a polypeptide linker, 48, fused to their respective carboxyl termini. Each polypeptide linker has two cysteine residues such that when they are expressed the cysteines form two disulfide bonds, 50 and 52. In this case the polypeptide linker is comprised of the hinge region, 23 in Figure 1. The hinge region is comprised of amino acid residues 363, a glutamine, to and including amino acid residue 377 of SEQ ID NO: 22.

In another aspect of the invention, a method is provided for producing a soluble receptor comprised of extracellular domains of IL-20RA and IL-20RB comprising (a) introducing into a host cell a first DNA sequence comprised of a transcriptional promoter operatively linked to a first secretory signal sequence followed downstream by and in proper reading frame the DNA that encodes the extracellular portion of IL-20RA and the DNA that encodes an immunoglobulin light chain constant region;(b) introducing into the host cell a second DNA construct comprised of a transcriptional promoter operatively linked to a second secretory signal followed downstream by and in proper reading frame a DNA sequence that encodes the extracellular portion of IL-20RB and a DNA sequence that encodes an immunoglobulin heavy chain constant region domain selected from the group consisting of C_{H}1, C_{H}2, C_{H}3 and C_{H}4; (c) growing the host cell in an appropriate growth medium under physiological conditions to allow the secretion of a fusion protein comprised of the extracellular domain of IL-20RA and IL-20RB; and (d) isolating the polypeptide from the host cell. In one embodiment, the second DNA sequence further encodes an immunoglobulin heavy chain hinge region wherein the hinge region is joined to the heavy chain constant region domain. In another embodiment, the second DNA sequence further encodes an immunoglobulin variable region joined upstream of and in proper reading frame with the immunoglobulin heavy chain constant region.

In an alternative embodiment, a method is provided for producing a soluble receptor comprised of the extracellular domains of IL-20RA and IL-20RB comprising (a) introducing into a host cell a first DNA sequence comprised of a transcriptional promoter operatively linked to a first secretory signal sequence followed downstream by and in proper reading frame the DNA that encodes the extracellular portion of IL-20RB and the DNA that encodes an immunoglobulin light chain constant region;(b) introducing into the host cell a second DNA construct comprised of a transcriptional promoter operatively linked to a second secretory signal followed downstream by and in proper reading frame a DNA sequence that encodes the extracellular portion of IL-20RA and a DNA sequence that encodes an immunoglobulin heavy chain constant region domain selected from the group consisting of C_{H}1, C_{H}2, C_{H}3 and C_{H}4; (c) growing the host cell in an appropriate growth medium under physiological conditions to allow the secretion of a dimerized heterodimeric fusion protein comprised of the extracellular domain of IL-20RA and IL-20RB; and (d) isolating the dimerized polypeptide from the host cell. In one embodiment, the second DNA sequence further encodes an immunoglobulin heavy chain hinge region wherein the hinge region is joined to the heavy chain constant region domain. In another embodiment, the second DNA sequence further encodes an immunoglobulin variable region joined upstream of and in proper reading frame with the immunoglobulin heavy chain constant region. (See U.S. Patent No. 5,843,725.)

A polynucleotide, generally a cDNA sequence, encodes the described polypeptides herein. A cDNA sequence that encodes a polypeptide is comprised of a series of codons, each amino acid residue of the polypeptide being encoded by a codon and each codon being comprised of three nucleotides. The amino acid residues are encoded by their respective codons as follows.
Alanine (Ala) is encoded by GCA, GCC, GCG or GCT.
Cysteine (Cys) is encoded by TGC or TGT.
Aspartic acid (Asp) is encoded by GAC or GAT.
Glutamic acid (Glu) is encoded by GAA or GAG.
Phenylalanine (Phe) is encoded by TTC or TTT.
Glycine (Gly) is encoded by GGA, GGC, GGG or GGT.
Histidine (His) is encoded by CAC or CAT.
Isoleucine (Ile) is encoded by ATA, ATC or ATT.
Lysine (Lys) is encoded by AAA, or AAG.
Leucine (Leu) is encoded by TTA, TTG, CTA, CTC, CTG or CTT.
Methionine (Met) is encoded by ATG.
Asparagine (Asn) is encoded by AAC or AAT.
Proline (Pro) is encoded by CCA, CCC, CCG or CCT.
Glutamine (Gln) is encoded by CAA or CAG.
Arginine (Arg) is encoded by AGA, AGG, CGA, CGC, CGG or CGT.
Serine (Ser) is encoded by AGC, AGT, TCA, TCC, TCG or TCT.
Threonine (Thr) is encoded by ACA, ACC, ACG or ACT.
Valine (Val) is encoded by GTA, GTC, GTG or GTT.
Tryptophan (Trp) is encoded by TGG.
Tyrosine (Tyr) is encoded by TAC or TAT.

It is to be recognized that according to the present invention, when a polynucleotide is claimed as described herein, it is understood that what is claimed are both the sense strand, the anti-sense strand, and the DNA as double-stranded having both the sense and anti-sense strand annealed together by their respective hydrogen bonds. Also claimed is the messenger RNA (mRNA) that encodes the polypeptides, and which mRNA is encoded by the cDNA described herein. Messenger RNA (mRNA) will encode a polypeptide using the same codons as those defined herein, with the exception that each thymine nucleotide (T) is replaced by a uracil nucleotide (U).

One of ordinary skill in the art will also appreciate that different species can exhibit "preferential codon usage." In general, see, Grantham, et al., Nuc. Acids Res. 8:1893-1912 (1980); Haas, et al. Curr. Biol. 6:315-324 (1996); Wain-Hobson, et al., Gene 13:355-364 (1981); Grosjean and Fiers, Gene 18:199-209 (1982); Holm, Nuc. Acids Res. 14:3075-3087 (1986); Ikemura, J. Mol. Biol. 158:573-597 (1982). As used herein, the term "preferential codon usage" or "preferential codons" is a term of art referring to protein translation codons that are most frequently used in cells of a certain species, thus favoring one or a few representatives of the possible codons encoding each amino acid. For example, the amino acid Threonine (Thr) may be encoded by ACA, ACC, ACG, or ACT, but in mammalian cells ACC is the most commonly used codon; in other species, for example, insect cells, yeast, viruses or bacteria, different Thr codons may be preferential. Preferential codons for a particular species can be introduced into the polynucleotides of the present invention by a variety of methods known in the art. Introduction of preferential codon sequences into recombinant DNA can, for example, enhance production of the protein by making protein translation more efficient within a particular cell type or species. Sequences containing preferential codons can be tested and optimized for expression in various species, and tested for functionality as disclosed herein.

Methods for synthesizing amino acids and aminoacylating tRNA are known in the art. Transcription and translation of plasmids containing nonsense mutations is carried out in a cell-free system comprising an *E. coli* S30 extract and commercially available enzymes and other reagents. Proteins are purified by chromatography. See, for example, Robertson et al., J. Am. Chem. Soc. 113:2722 (1991); Ellman et al., Methods Enzymol. 202:301 (1991; Chung et al., Science 259:806-809 (1993); and Chung et al., Proc. Natl. Acad. Sci. USA 90:10145-1019 (1993). In a second method, translation is carried out *in Xenopus* oocytes by microinjection of mutated mRNA and chemically aminoacylated suppressor tRNAs, Turcatti et al., J. Biol. Chem. 271:19991-19998 (1996). Within a third method, *E. coli* cells are cultured in the absence of a natural amino acid that is to be replaced (*e.g.*, phenylalanine) and in the presence of the desired non-naturally occurring amino acid(s) (*e.g.*, 2-azaphenylalanine, 3-azaphenylalanine, 4-azaphenylalanine, or 4-fluorophenylalanine). The non-naturally occurring amino acid is incorporated into the protein in place of its natural counterpart. See, Koide et al., Biochem. 33:7470-7476 (1994). Naturally occurring amino acid residues can be converted to non-naturally occurring species by *in vitro* chemical modification. Chemical modification can be combined with site-directed mutagenesis to further expand the range of substitutions, Wynn and Richards, Protein Sci. 2:395-403 (1993).

A limited number of non-conservative amino acids, amino acids that are not encoded by the genetic code, non-naturally occurring amino acids, and unnatural amino acids may be substituted for amino acid residues.

Essential amino acids in the polypeptides described herein can be identified according to procedures known in the art, such as site-directed mutagenesis or alanine-scanning mutagenesis, Cunningham and Wells, Science 244: 1081-1085 (1989); Bass et al., Proc. Natl. Acad. Sci. USA 88:4498-502 (1991). In the latter technique, single alanine mutations are introduced at every residue in the molecule, and the resultant mutant molecules are tested for biological activity as disclosed below to identify amino acid residues that are critical to the activity of the molecule. See also, Hilton et al., J. Biol. Chem. 271:4699-708, 1996. Sites of ligand-receptor interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-312 (1992); Smith et al., J. Mol. Biol. 224:899-904 (1992); Wlodaver et al., FEBS Lett. 309:59-64 (1992).

Multiple amino acid substitutions can be made and tested using known methods of mutagenesis and screening, such as those disclosed by Reidhaar-Olson and Sauer, Science 241:53-57 (1988) or Bowie and Sauer, Proc. Natl. Acad. Sci. USA 86:2152-2156 (1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display, *e.g.,* Lowman et al., Biochem. 30:10832-10837 (1991); Ladner et al., U.S. Patent No. 5,223,409; Huse, WIPO Publication WO 92/06204) and region-directed mutagenesis, Derbyshire et al., Gene 46:145 (1986); Ner et al., DNA 7:127 (1988).

Variants of the disclosed *IL-20,* IL-20RA and IL-20RB DNA and polypeptide sequences can be generated through DNA shuffling as disclosed by Stemmer, Nature 370:389-391, (1994), Stemmer, Proc. Natl. Acad. Sci. USA 91:10747-10751 (1994) and WIPO Publication WO 97/20078. Briefly, variant DNAs are generated by *in vitro* homologous recombination by random fragmentation of a parent DNA followed by reassembly using PCR, resulting in randomly introduced point mutations. This technique can be modified by using a family of parent DNAs, such as allelic variants or DNAs from different species, to introduce additional variability into the process. Selection or screening for the desired activity, followed by additional iterations of mutagenesis and assay provides for rapid "evolution" of sequences by selecting for desirable mutations while simultaneously selecting against detrimental changes.

Mutagenesis methods as disclosed herein can be combined with high-throughput, automated screening methods to detect activity of cloned, mutagenized polypeptides in host cells. Mutagenized DNA molecules that encode active polypeptides can be recovered from the host cells and rapidly sequenced using modem equipment. These methods allow the rapid determination of the importance of individual amino acid residues in a polypeptide of interest, and can be applied to polypeptides of unknown structure.

### PROTEIN PRODUCTION

Polypeptides can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., Molecular Cloning: A Laboratory Manual, 2nd ed., (Cold Spring Harbor Laboratory Press, Cold Spring Harbor, NY, 1989), and Ausubel et al., eds., Current Protocols in Molecular Biology (John Wiley and Sons, Inc., NY, 1987).

In general, a DNA sequence encoding a polypeptide is operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers.

To direct a polypeptide into the secretory pathway of a host cell, a secretory signal sequence (also known as a leader sequence, prepro sequence or pre sequence) is provided in the expression vector. The secretory signal sequence may be that of the native polypeptides, or may be derived from another secreted protein (*e.g.*, t-PA) or synthesized *de novo.* The secretory signal sequence is operably linked to the DNA sequence, i.e., the two sequences are joined in the correct reading frame and positioned to direct the newly synthesized polypeptide into the secretory pathway of the host cell. Secretory signal sequences are commonly positioned 5' to the DNA sequence encoding the polypeptide of interest, although certain secretory signal sequences may be positioned elsewhere in the DNA sequence of interest (see, *e.g.*, Welch et al., U.S. Patent No. 5,037,743; Holland et al., U.S. Patent No. 5,143,830).

Alternatively, the secretory signal sequence contained in the polypeptides described herein is used to direct other polypeptides into the secretory pathway. The present invention provides for such fusion polypeptides. The secretory signal sequence contained in the fusion polypeptides of the present invention is preferably fused amino-terminally to an additional peptide to direct the additional peptide into the secretory pathway. Such constructs have numerous applications known in the art. For example, these novel secretory signal sequence fusion constructs can direct the secretion of an active component of a normally non-secreted protein, such as a receptor. Such fusions may be used *in vivo* or *in vitro* to direct peptides through the secretory pathway.

Cultured mammalian cells are suitable hosts within the present invention. Methods for introducing exogenous DNA into mammalian host cells include calcium phosphate-mediated transfection, Wigler et al., Cell 14:725 (1978), Corsaro and Pearson, Somatic Cell Genetics 7:603 (1981); Graham and Van der Eb, Virology 52:456 (1973), electroporation, Neumann et al., EMBO J. 1:841-845 (1982), DEAE-dextran mediated transfection (Ausubel *et al., ibid.,* and liposome-mediated transfection, Hawley-Nelson et al., Focus 15:73 (1993); Ciccarone et al., Focus 15:80 (1993), and viral vectors, Miller and Rosman, BioTechniques 7:980(1989); Wang and Finer, Nature Med. 2:714 (1996). The production of recombinant polypeptides in cultured mammalian cells is disclosed, for example, by Levinson et al., U.S. Patent No. 4,713,339; Hagen et al., U.S. Patent No. 4,784,950; Palmiter et al., U.S. Patent No. 4,579,821; and Ringold, U.S. Patent No. 4,656,134. Suitable cultured mammalian cells include the COS-1 (ATCC No. CRL 1650), COS-7 (ATCC No. CRL 1651), BHK (ATCC No. CRL 1632), BHK 570 (ATCC No. CRL 10314), 293 (ATCC No. CRL 1573; Graham et al., J. Gen. Virol. 36:59 (1977) and Chinese hamster ovary (*e.g.* CHO-K1; ATCC No. CCL 61) cell lines. Additional suitable cell lines are known in the art and available from public depositories such as the American Type Culture Collection, Rockville, Maryland. In general, strong transcription promoters are preferred, such as promoters from SV-40 or cytomegalovirus. See, *e.g.*, U.S. Patent No. 4,956,288. Other suitable promoters include those from metallothionein genes (U.S. Patent Nos. 4,579,821 and 4,601,978) and the adenovirus major late promoter.

Drug selection is generally used to select for cultured mammalian cells into which foreign DNA has been inserted. Such cells are commonly referred to as "transfectants". Cells that have been cultured in the presence of the selective agent and are able to pass the gene of interest to their progeny are referred to as "stable transfectants." A preferred selectable marker is a gene encoding resistance to the antibiotic neomycin. Selection is carried out in the presence of a neomycin-type drug, such as G-418 or the like. Selection systems can also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. A preferred amplifiable selectable marker is dihydrofolate reductase, which confers resistance to methotrexate. Other drug resistance genes (*e.g.* hygromycin resistance, multi-drug resistance, puromycin acetyltransferase) can also be used. Alternative markers that introduce an altered phenotype, such as green fluorescent protein, or cell surface proteins such as CD4, CD8, Class I MHC, placental alkaline phosphatase may be used to sort transfected cells from untransfected cells by such means as FACS sorting or magnetic bead separation technology.

Other higher eukaryotic cells can also be used as hosts, including plant cells, insect cells and avian cells. The use of *Agrobacterium rhizogenes* as a vector for expressing genes in plant cells has been reviewed by Sinkar et al., J. Biosci. (Bangalore) 11:47 (1987). Transformation of insect cells and production of foreign polypeptides therein is disclosed by Guarino et al., U.S. Patent No. 5,162,222 and WIPO publication WO 94/06463. Insect cells can be infected with recombinant baculovirus, commonly derived from Autographa califomica nuclear polyhedrosis virus (AcNPV). DNA encoding a polypeptide is inserted into the baculoviral genome in place of the AcNPV polyhedrin gene coding sequence by one of two methods. The first is the traditional method of homologous DNA recombination between wild-type AcNPV and a transfer vector containing the gene flanked by AcNPV sequences. Suitable insect cells, *e.g.* SF9 cells, are infected with wild-type AcNPV and transfected with a transfer vector comprising a polynucleotide operably linked to an AcNPV polyhedrin gene promoter, terminator, and flanking sequences. See, King, L.A. and Possee, R.D., The Baculovirus Expression System: A Laboratory Guide, (Chapman & Hall, London); O'Reilly, D.R. et al., Baculovirus Expression Vectors: A Laboratory Manual (Oxford University Press, New York, New York, 1994); and, Richardson, C. D., Ed., Baculovirus Expression Protocols. Methods in Molecular Biology, (Humana Press, Totowa, NJ 1995). Natural recombination within an insect cell will result in a recombinant baculovirus that contains coding sequences driven by the polyhedrin promoter. Recombinant viral stocks are made by methods commonly used in the art.

The second method of making recombinant baculovirus utilizes a transposon-based system described by Luckow, V.A, et al., J Virol 67:4566 (1993). This system is sold in the Bac-to-Bac kit (Life Technologies, Rockville, MD). This system utilizes a transfer vector, pFastBac1™ (Life Technologies) containing a Tn7 transposon to move the DNA encoding the polypeptide into a baculovirus genome maintained in *E. coli* as a large plasmid called a "bacmid." The pFastBacl™ transfer vector utilizes the AcNPV polyhedrin promoter to drive the expression of the gene of interest. However, pFastBac1™ can be modified to a considerable degree. The polyhedrin promoter can be removed and substituted with the baculovirus basic protein promoter (also known as *Pcor,* p6.9 or MP promoter), which is expressed earlier in the baculovirus infection, and has been shown to be advantageous for expressing secreted proteins. See, Hill-Perkins, M.S. and Possee, R.D., J Gen Virol 71:971 (1990); Bonning, B.C. et al., J Gen Virol 75:1551 (1994); and, Chazenbalk, G.D., and Rapoport, B., J Biol Chem 270:1543 (1995). In such transfer vector constructs, a short or long version of the basic protein promoter can be used. Moreover, transfer vectors can be constructed that replace the native secretory signal sequences with secretory signal sequences derived from insect proteins. For example, a secretory signal sequence from Ecdysteroid Glucosyltransferase (EGT), honey bee Melittin (Invitrogen, Carlsbad, CA), or baculovirus gp67 (PharMingen, San Diego, CA) can be used in constructs to replace the native secretory signal sequence. In addition, transfer vectors can include an in-frame fusion with DNA encoding an epitope tag at the C- or N-terminus of the expressed polypeptide, for example, a Glu-Glu epitope tag, Grussenmeyer, T. et al., Proc Natl Acad Sci. 82:7952 (1985). Using a technique known in the art, a transfer vector containing a recombinant gene is transformed into *E. coli,* and screened for bacmids that contain an interrupted lacZ gene indicative of recombinant baculovirus. The bacmid DNA containing the recombinant baculovirus genome is isolated, using common techniques, and used to transfect *Spodoptera frugiperda* cells, *e.g.* Sf9 cells. Recombinant virus that expresses the polypeptide is subsequently produced. Recombinant viral stocks are made by methods commonly used the art.

The recombinant virus is used to infect host cells, typically a cell line derived from the fall armyworm, *Spodoptera frugiperda.* See, in general, Glick and Pasternak, Molecular Biotechnology: Principles and Applications of Recombinant DNA (ASM Press, Washington, D.C., 1994). Another suitable cell line is the High FiveO™ cell line (Invitrogen) derived from *Trichoplusia ni* (U.S. Patent #5,300,435). Commercially available serum-free media are used to grow and maintain the cells. Suitable media are Sf900 II™ (Life Technologies) or ESF 921™ (Expression Systems) for the Sf9 cells; and Ex-ce11O405™ (JRH Biosciences, Lenexa, KS) or Express FiveO™ (Life Technologies) for the *T. ni* cells. The cells are grown up from an inoculation density of approximately 2-5 x 10⁵ cells to a density of 1-2 x 10⁶ cells at which time a recombinant viral stock is added at a multiplicity of infection (MOI) of 0.1 to 10, more typically near 3. The recombinant virus-infected cells typically produce the recombinant polypeptide at 12-72 hours post-infection and secrete it with varying efficiency into the medium. The culture is usually harvested 48 hours post-infection. Centrifugation is used to separate the cells from the medium (supernatant). The supernatant containing the polypeptide is filtered through micropore filters, usually 0.45 µm pore size. Procedures used are generally described in available laboratory manuals (King, L. A. and Possee, R.D., *ibid.,* O'Reilly, D.R. *et al., ibid*.; Richardson, C. D., *ibid.*). Subsequent purification of the polypeptide from the supernatant can be achieved using methods described herein.

Fungal cells, including yeast cells, can also be used within the present invention. Yeast species of particular interest in this regard include *Saccharomyces cerevisiae, Pichia pastoris,* and *Pichia methanolica.* Methods for transforming *S. cerevisiae* cells with exogenous DNA and producing recombinant polypeptides therefrom are disclosed by, for example, Kawasaki, U.S. Patent No. 4,599,311; Kawasaki et al., U.S. Patent No. 4,931,373; Brake, U.S. Patent No. 4,870,008; Welch et al., U.S. Patent No. 5,037,743; and Murray et al., U.S. Patent No. 4,845,075. Transformed cells are selected by phenotype determined by the selectable marker, commonly drug resistance or the ability to grow in the absence of a particular nutrient (*e.g.*, leucine). A preferred vector system for use in *Saccharomyces cerevisiae* is the *POT1* vector system disclosed by Kawasaki et al. (U.S. Patent No. 4,931,373), which allows transformed cells to be selected by growth in glucose-containing media. Suitable promoters and terminators for use in yeast include those from glycolytic enzyme genes (see, *e.g.*, Kawasaki, U.S. Patent No. 4,599,311; Kingsman et al., U.S. Patent No. 4,615,974; and Bitter, U.S. Patent No. 4,977,092) and alcohol dehydrogenase genes. See also U.S. Patents Nos. 4,990,446; 5,063,154; 5,139,936 and 4,661,454. Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillermondii* and *Candida maltosa* are known in the art. See, for example, Gleeson et al., J. Gen. Microbiol. 132:3459 (1986) and Cregg, U.S. Patent No. 4,882,279. *Aspergillus* cells may be utilized according to the methods of McKnight et al., U.S. Patent No. 4,935,349. Methods for transforming *Acremonium chrysogenum* are disclosed by Sumino et al., U.S. Patent No. 5,162,228. Methods for transforming *Neurospora* are disclosed by Lambowitz, U.S. Patent No. 4,486,533.

The use of *Pichia methanolica* as host for the production of recombinant proteins is disclosed in WIPO Publications WO 97/17450, WO 97/17451, WO 98/02536, and WO 98/02565. DNA molecules for use in transforming *P. methanolica* will commonly be prepared as double-stranded, circular plasmids, which are preferably linearized prior to transformation. For polypeptide production in *P. methanolica,* it is preferred that the promoter and terminator in the plasmid be that of a *P. methanolica* gene, such as a *P. methanolica* alcohol utilization gene (*AUG1* or *AUG2*). Other useful promoters include those of the dihydroxyacetone synthase (DHAS), formate dehydrogenase (FMD), and catalase (CAT) genes. To facilitate integration of the DNA into the host chromosome, it is preferred to have the entire expression segment of the plasmid flanked at both ends by host DNA sequences. A preferred selectable marker for use in *Pichia methanolica* is a *P. methanolica ADE2* gene, which encodes phosphoribosyl-5-aminoimidazole carboxylase (AIRC; EC 4.1.1.21), which allows *ade2* host cells to grow in the absence of adenine. For large-scale, industrial processes where it is desirable to minimize the use of methanol, it is preferred to use host cells in which both methanol utilization genes (*AUG1* and *AUG2*) are deleted. For production of secreted proteins, host cells deficient in vacuolar protease genes (*PEP4* and *PRB1*) are preferred. Electroporation is used to facilitate the introduction of a plasmid containing DNA encoding a polypeptide of interest into *P. methanolica* cells. It is preferred to transform *P. methanolica* cells by electroporation using an exponentially decaying, pulsed electric field having a field strength of from 2.5 to 4.5 kV/cm, preferably about 3.75 kV/cm, and a time constant (t) of from I to 40 milliseconds, most preferably about 20 milliseconds.

Prokaryotic host cells, including strains of the bacteria *Escherichia coli, Bacillus* and other genera are also useful host cells within the present invention. Techniques for transforming these hosts and expressing foreign DNA sequences cloned therein are well known in the art; see, *e.g.*, Sambrook *et al., ibid*.). When expressing a polypeptide in bacteria such as *E. coli,* the polypeptide may be retained in the cytoplasm, typically as insoluble granules, or may be directed to the periplasmic space by a bacterial secretion sequence. In the former case, the cells are lysed, and the granules are recovered and denatured using, for example, guanidine isothiocyanate or urea. The denatured polypeptide can then be refolded and dimerized by diluting the denaturant, such as by dialysis against a solution of urea and a combination of reduced and oxidized glutathione, followed by dialysis against a buffered saline solution. In the latter case, the polypeptide can be recovered from the periplasmic space in a soluble and functional form by disrupting the cells (by, for example, sonication or osmotic shock) to release the contents of the periplasmic space and recovering the protein, thereby obviating the need for denaturation and refolding.

Transformed or transfected host cells are cultured according to conventional procedures in a culture medium containing nutrients and other components required for the growth of the chosen host cells. A variety of suitable media, including defined media and complex media, are known in the art and generally include a carbon source, a nitrogen source, essential amino acids, vitamins and minerals. Media may also contain such components as growth factors or serum, as required. The growth medium will generally select for cells containing the exogenously added DNA by, for example, drug selection or deficiency in an essential nutrient, which is complemented by the selectable marker carried on the expression vector or co-transfected into the host cell. *P. methanolica* cells are cultured in a medium comprising adequate sources of carbon, nitrogen and trace nutrients at a temperature of about 25°C to 35°C. Liquid cultures are provided with sufficient aeration by conventional means, such as shaking of small flasks or sparging of fermentors. A preferred culture medium for *P. methanolica* is YEPD (2% D-glucose, 2% Bacto™ Peptone (Difco Laboratories, Detroit, MI), 1% Bacto™ yeast extract (Difco Laboratories), 0.004% adenine and 0.006% L-leucine).

### Protein Isolation

It is preferred to purify the polypeptides described herein to ≥80% purity, more preferably to ≥90% purity, even more preferably ≥95% purity, and particularly preferred is a pharmaceutically pure state, that is greater than 99.9% pure with respect to contaminating macromolecules, particularly other proteins and nucleic acids, and free of infectious and pyrogenic agents. Preferably, a purified polypeptide is substantially free of other polypeptides, particularly other polypeptides of animal origin.

Expressed recombinant polypeptides (or chimeric polypeptides) can be purified using fractionation and/or conventional purification methods and media. Ammonium sulfate precipitation and acid or chaotrope extraction may be used for fractionation of samples. Exemplary purification steps may include hydroxyapatite, size exclusion, FPLC and reverse-phase high performance liquid chromatography. Suitable chromatographic media include derivatized dextrans, agarose, cellulose, polyacrylamide, specialty silicas, and the like. PEI, DEAE, QAE and Q derivatives are preferred. Exemplary chromatographic media include those media derivatized with phenyl, butyl, or octyl groups, such as Phenyl-Sepharose FF (Pharmacia), Toyopearl butyl 650 (Toso Haas, Montgomeryville, PA), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups and/or carbohydrate moieties. Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Methods for binding receptor polypeptides to support media are well known in the art. Selection of a particular method is a matter of routine design and is determined in part by the properties of the chosen support. See, for example, Affinity Chromatography: Principles & Methods (Pharmacia LKB Biotechnology, Uppsala, Sweden, 1988).

Polypeptides can be isolated by exploitation of their properties. For example, immobilized metal ion adsorption (IMAC) chromatography can be used to purify histidine-rich proteins, including those comprising polyhistidine tags. Briefly, a gel is first charged with divalent metal ions to form a chelate, Sulkowski, Trends in Biochem. 3:1 (1985). Histidine-rich proteins will be adsorbed to this matrix with differing affinities, depending upon the metal ion used, and will be eluted by competitive elution, lowering the pH, or use of strong chelating agents. Other methods of purification include purification of glycosylated proteins by lectin affinity chromatography and ion exchange chromatography. A protein fused to the Fc portion of an immunoglobulin can be purified using a 'Protein A column'. Methods in Enzymol., Vol. 182, "Guide to Protein Purification", M. Deutscher, (ed.),page 529-539 (Acad. Press, San Diego, 1990). Within additional embodiments of the invention, a fusion of the polypeptide of interest and an affinity tag (*e.g.*, maltose-binding protein, an immunoglobulin domain) may be constructed to facilitate purification.

As used herein, the term "antibodies" includes polyclonal antibodies, affinity-purified polyclonal antibodies, monoclonal antibodies, and antigen-binding fragments, such as F(ab')₂ and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced.

A variety of assays known to those skilled in the art can be utilized to detect antibodies that bind to protein or peptide. Exemplary assays are described in detail in Antibodies: A Laboratory Manual, Harlow and Lane (Eds.) (Cold Spring Harbor Laboratory Press, 1988). Representative examples of such assays include: concurrent immunoelectrophoresis, radioimmunoassay, radioimmuno-precipitation, enzyme-linked immunosorbent assay (ELISA), dot blot or Western blot assay, inhibition or competition assay, and sandwich assay.

The soluble receptors described herein can be used to down-regulate IL-20, which has been shown to be involved in a number of inflammatory processes. Specifically, IL-20 has been shown to up-regulate IL-8. Inflammatory diseases in which IL-8 plays a significant role, and for which a decrease in IL-8 would be beneficial are, adult respiratory disease (ARD), septic shock, multiple organ failure, inflammatory lung injury such as asthma or bronchitis, bacterial pneumonia, psoriasis, eczema, atopic and contact dermatitis, and inflammatory bowel disease such as ulcerative colitis and Crohn's disease. Thus, the soluble receptor to IL-20 described herein can be administered to a patient to treat these diseases.

### Biology of IL-20, Its receptor and Its Role in Psoriasis

Two orphan class II cytokine receptors, both of which are expressed in skin, were identified as IL-20 receptor subunits. Both IL-20 receptor subunits are required for ligand binding, distinguishing their role from that of subunits in the four other known class II cytokine receptors. IL-20RA and IL-20RB are also coexpressed in a number of human tissues besides skin, including ovary, adrenal gland, testis, salivary gland, muscle, lung, kidney, heart and to a lesser degree the small intestine suggesting additional target tissues for IL-20 action. We conclude that the IL-20 heterodimeric receptor is structurally similar to other class II cytokine receptors and is expressed in skin where we have demonstrated activity of the IL-20 ligand.

Two lines of evidence indicate that a role IL-20 and its receptor are involved in psoriasis. This multigenic skin disease is characterized by increased keratinocyte proliferation, altered keratinocyte differentiation, and infiltration of immune cells into the skin. The first line of evidence for a role of IL-20 in psoriasis is that the observed hyperkeratosis and thickened epidermis in the transgenic mice that resemble human psoriatic abnormalities. Decreased numbers of tonofilaments, thought to be related to defective keratinization, are a striking feature of human psoriasis. Intramitochondrial inclusions have been found in both chemically induced and naturally occurring hyperplastic skin conditions in mice. The cause of the inclusions and their effects on mitochondrial function, if any, are unknown. We conclude that IL-20 transgenic mice exhibit many of the characteristics observed in human psoriasis.

A second line of evidence that implicates the IL-20 receptor in psoriasis is that both IL-20RA and IL-20RB mRNA are markedly upregulated in human psoriatic skin compared to normal skin. Both IL-20 receptor subunits are expressed in keratinocytes throughout the epidermis and are also expressed in a subset of immune and endothelial cells. We propose that increased expression of an activated IL-20 receptor may alter the interactions between endothelial cells, immune cells and keratinocytes, leading to dysregulation of keratinocyte proliferation and differentiation.

A crucial step in understanding the function of a novel cytokine is the identification and characterization of its cognate receptor. We have successfully used a structure-based approach to isolate a novel interleukin that ultimately led to the isolation of its receptor. IL-20 stimulates signal transduction in the human keratinocyte HaCaT cell line, supporting a direct action of this novel ligand in skin. In addition, IL-1β, EGF and TNF-α, proteins known to be active in keratinocytes and to be involved with proliferative and pro-inflammatory signals in skin, enhance the response to IL-20. In both HaCaT and BHK cells expressing the IL-20 receptor, IL-20 signals through STAT3. Thus, IL-20 binds its receptor on keratinocytes and stimulates a STAT3-containing signal transduction pathway.

### Use of Antagonist to IL-20 to Treat Psoriasis

As indicated in the discussion above and the examples below, IL-20 is involved in the pathology of psoriasis. Thus, the soluble receptors described herein can be administered to an individual to down-regulate IL-20 and thus treat psoriasis.

Psoriasis is one of the most common dermatologic diseases, affecting up to 1 to 2 percent of the world's population. It is a chronic inflammatory skin disorder characterized by erythematous, sharply demarcated papules and rounded plaques, covered by silvery micaceous scale. The skin lesions of psoriasis are variably pruritic. Traumatized areas often develop lesions of psoriasis. Additionally, other external factors may exacerbate psoriasis including infections, stress, and medications, *e.g.* lithium, beta blockers, and anti-malarials.

The most common variety of psoriasis is called plaque type. Patients with plaque-type psoriasis will have stable, slowly growing plaques, which remain basically unchanged for long periods of time. The most common areas for plaque psoriasis to occur are the elbows knees, gluteal cleft, and the scalp. Involvement tends to be symmetrical. Inverse psoriasis affects the intertriginous regions including the axilla, groin, submammary region, and navel, and it also tends to affect the scalp, palms, and soles. The individual lesions are sharply demarcated plaques but may be moist due to their location. Plaque-type psoriasis generally develops slowly and runs an indolent course. It rarely spontaneously remits.

Eruptive psoriasis (guttate psoriasis) is most common in children and young adults. It develops acutely in individuals without psoriasis or in those with chronic plaque psoriasis. Patients present with many small erythematous, scaling papules, frequently after upper respiratory tract infection with beta-hemolytic streptococci. Patients with psoriasis may also develop pustular lesions. These may be localized to the palms and soles or may be generalized and associated with fever, malaise, diarrhea, and arthralgias..

About half of all patients with psoriasis have fingernail involvement, appearing as punctate pitting, nail thickening or subungual hyperkeratosis. About 5 to 10 percent of patients with psoriasis have associated joint complaints, and these are most often found in patients with fingernail involvement. Although some have the coincident occurrence of classic Although some have the coincident occurrence of classic rheumatoid arthritis, many have joint disease that falls into one of five type associated with psoriasis: (1) disease limited to a single or a few small joints (70 percent of cases); (2) a seronegative rheumatoid arthritis-like disease; (3) involvement of the distal interphalangeal joints; (4) severe destructive arthritis with the development of "arthritis mutilans"; and (5) disease limited to the spine.

Psoriasis can be treated by administering antagonists to IL-20. The preferred antagonists are either a soluble receptor to IL-20 or antibodies, antibody fragments or single chain antibodies that bind to either the IL-20 receptor or to IL-20. The antagonists to IL-20 can be administered alone or in combination with other established therapies such as lubricants, keratolytics, topical corticosteroids, topical vitamin D derivatives, anthralin, systemic antimetabolites such as methotrexate, psoralen-ultraviolet-light therapy (PUVA), etretinate, isotretinoin, cyclosporine, and the topical vitamin D3 derivative calcipotriol. The antagonists, in particularly the soluble receptor or the antibodies that bind to IL-20 or the IL-20 receptor can be administered to individual subcutaneously, intravenously, or transdermally using a cream or transdermal patch that contains the antagonist of IL-20. If administered subcutaneously, the antagonist can be injected into one or more psoriatic plaques. If administered transdermally, the antagonists can be administered directly on the plaques using a cream containing the antagonist to IL-20.

### Use of Antagonists to IL-20 to Treat Inflammatory Conditions of the Lung.

The soluble receptor of IL-20 described herein can be administered to a person who has asthma, bronchitis or cystic fibrosis or other inflammatory lung disease to treat the disease. The antagonists can be administered by any suitable method including intravenous, subcutaneous, bronchial lavage, and the use of inhalant containing an antagonist to IL-20.

### Administration of the IL-20 Soluble Receptor

The quantities of the IL-20 soluble necessary for effective therapy will depend upon many different factors, including means of administration, target site, physiological state of the patient, and other medications administered. Thus, treatment dosages should be titrated to optimize safety and efficacy. Typically, dosages used *in vitro* may provide useful guidance in the amounts useful for *in vivo* administration of these reagents. Animal testing of effective doses for treatment of particular disorders will provide further predictive indication of human dosage. Methods for administration include oral, intravenous, peritoneal, intramuscular, transdermal or administration into the lung or trachea in spray form by means or a nebulizer or atomizer. Pharmaceutically acceptable carriers will include water, saline, buffers to name just a few. Dosage ranges would ordinarily be expected from 1µg to 1000µg per kilogram of body weight per day. A dosage for an average adult of the IL-20 soluble receptor would be about 25 mg given twice weekly as a subcutaneous injection. Injections could be given at the site of psoriatic lesions for the treatment of psoriasis. For subcutaneous or intravenous administration of the antagonist to IL-20, the antibody or soluble receptor can be in phosphate buffered saline. Also in skin diseases such as psoriasis, the antagonist to IL-20 can be administered via an ointment or transdermal patch. The doses by may be higher or lower as can be determined by a medical doctor with ordinary skill in the art. For a complete discussion of drug formulations and dosage ranges *see* Remington's Pharmaceutical Sciences, 18th Ed., (Mack Publishing Co., Easton, Penn., 1996), and Goodman and Gilman's: The Pharmacological Bases of Therapeutics, 9th Ed. (Pergamon Press 1996).

The invention is further illustrated by the following non-limiting examples.

### Example 1.

### Up-regulation of IL-8 by IL-20

### Methods:

Normal Human Epidermal neonatal keratinocytes (NHEK) (from Clonetics) at passage 2 were plated and grown to confluency in 12 well tissue culture plates. KGM (Keratinocyte growth media) was purchased from Clonetics. When cells reached confluency, they were washed with KGM media minus growth factors = KBM (keratinocyte basal media). Cells were serum starved in KBM for 72 hours prior to the addition of test compounds. Thrombin at 1 I.U./mL and trypsin at 25nM were used as positive controls. One mL of media/well was added. KBM only was used as the negative control.

IL-20 was made up in KBM media and added at varying concentrations, from 2.5µg/ml down to 618ng/mL in a first experiment and from 2.5µg/mL down to 3ng/mL in a second experiment.

Cells were incubated at 37° C, 5% CO₂ for 48 hours. Supernatants were removed and frozen at -80° C for several days prior to assaying for IL-8 and GM-CSF levels. Human IL-8 Immunoassay kit # D8050 (RandD Systems, Inc.) and human GM-CSF Immunoassay kit # HSGMO (RandD Systems, Inc.) were used to determine cytokine production following manufacturer's instructions.

### Results

The results indicated that the expression of IL-8 and GM-CSF were induced by IL-20.

### Example 2

### Cloning of IL-20RB

### Cloning of IL-20RB coding region

Two PCR primers were designed based on the sequence from International Patent Application No. PCT/US99/03735 (publication no. WO 99/46379) filed on March 8, 1999. SEQ ID NO: 16 contains the ATG (Met1) codon with an EcoRI restriction site, SEQ ID NO: 17 contains the stop codon (TAG) with an XhoI restriction site. The PCR amplification was carried out using a human keratinocyte (HaCaT) cDNA library DNA as a template and SEQ ID NO: 16 and SEQ ID NO: 17 as primers. The PCR reaction was performed as follows: incubation at 94°C for 1 min followed by 30 cycles of 94°C for 30 sec and 68°C for 2 min, after additional 68°C for 4 min, the reaction was stored at 4°C. The PCR products were run on 1% Agarose gel, and a 1 kb DNA band was observed. The PCR products were cut from the gel and the DNA was purified using a QIAquick Gel Extraction Kit (Qiagen). The purified DNA was digested with EcoRI and XhoI, and cloned into a pZP vector that was called pZP7N. A pZP plasmid is a mammalian expression vector containing an expression cassette having the mouse metallothionein-1 promoter, human tPA leader peptide, multiple restriction sites for insertion of coding sequences, a Glu-Glu tag, and a human growth hormone terminator. The plasmid also has an E. coli origin of replication, a mammalian selectable marker expression unit having an SV40 promoter, an enhancer and an origin of replication, as well as a DHFR gene, and the SV40 terminator. Several IL-20RB-pZP7N clones were sequenced. They all contain three non-conservative mutations compared with the sequence of IL-20RB in PCT/US99/03735: (sequence IL-20RB-pZP7N), 146 Pro (CCC) -- Thr (ACC), 148 His (CAT) -- Asp (GAT), and 171 Thr (ACG) -- Arg (AGG).

To verify the three substitutions in IL-20RB-pZP7N clone, PCR amplification was carried out using three difference cDNA sources -- fetal skin marathon cDNA, HaCaT cDNA library DNA, and prostate smooth muscle cDNA library DNA -- as templates. The PCR products were gel purified and sequenced. The sequence of each of the three PCR products was consistent with that of the IL-20RB-pZP7N clone. IL-20RB is SEQ ID NO: 13 and 14, and the mature extracellular domain is SEQ ID NO: 15.

### Example 3

### Binding of IL-20 to IL-20RB/ IL-20RA Heterodimer

A cell-based binding assay was used to verify IL-20 binds to IL-20RAIL-20RB heterodimer.

Expression vectors containing known and orphan Class II cytokine receptors (including IL-20RA and IL-20RB) were transiently transfected into COS cells in various combinations, which were then assayed for their ability to bind biotin-labeled IL-20 protein. The results show IL-20RB- IL-20RA heterodimer is a receptor for IL-20. The procedure used is described below.

The COS cell transfection was performed in a 12-well tissue culture plate as follows: 0.5 µg DNA was mixed with medium containing 5 µl lipofectamine in 92 µl serum free Dulbecco's modified Eagle's medium (DMEM) (55 mg sodium pyruvate, 146 mg L-glutamine, 5 mg transferrin, 2.5 mg insulin, 1 µg selenium and 5 mg fetuin in 500 ml DMEM), incubated at room temperature for 30 minutes and then added to 400 µl serum free DMEM media. This 500 µl mixture was then added to 1.5 x 10⁵ COS cells/well and incubated for 5 hours at 37°C. 500 µl 20% fetal bovine serum (FBS) DMEM media was added and incubated overnight.

The assay, a modification of the "secretion trap" (Davis, S., et al., Cell 87: 1161-1169 (1996), was performed as follows: cells were rinsed with PBS/1% bovine serum albumin (BSA) and blocked for 1 hour with TNB (0.1 M Tris-HCl, 0.15 M NaCl and 0.5% Blocking Reagent (NEN Renaissance TSA-Direct Kit Cat# NEL701) in water). This was followed by a one-hour incubation with 3 µg/ml biotinylated IL-20 protein in TNB. Cells were washed with PBS/1% BSA and incubated for another hour with 1:300 diluted streptavidin-HRP (NEN kit) in TNB. Following another wash, cells were fixed for 15 minutes with 1.8% Formaldehyde in phosphate-buffered saline (PBS). Cells were then washed with TNT (0.1 M Tris-HCL, 0.15 M NaCl, and 0.05% Tween-20 in water). Positive binding signals were detected following a five-minute incubation with fluorescein tyramide reagent diluted 1:50 in dilution buffer (NEN kit). Cells were washed with TNT, preserved with Vectashield Mounting Media (Vector Labs) diluted 1:5 in TNT, and visualized using an FITC filter on an inverted fluorescent microscope.

### Example 4

### Up-regulation of Inflammatory Cytokines by IL-20

### Cell Treatment

The human keratinocyte cell line, HaCaT was grown at 37°C to several days post-confluence in T-75 tissue culture flasks. At this point, normal growth media (DMEM + 10% FBS) was removed and replaced with serum-free media. Cells were then incubated for two days at 37°C. DMEM was then removed and four flasks of cells per treatment were treated with one of each of the following conditions for four hours at 37°C: recombinant human (rh) IL-1 alpha at 5 ng/mL, rh IL-1 alpha at 20 ng/mL, rh IL-1 alpha at 5 ng/mL + IL-20 at 1µg/mL, IL-20 at 1µg/mL, or rh IL-10 at 10 ng/mL.

### RNA Isolation

Following cytokine treatment, media was removed and cells were lysed using a guanidium thiocyanate solution. Total RNA was isolated from the cell lysate by an overnight spin on a cesium chloride gradient. The following day, the RNA pellet was resuspended in a TE/SDS solution and ethanol precipitated. RNA was then quantitated using a spectrophotometer, followed by a DNase treatment as per Section V.B. of Clontech's Atlas^{™} cDNA Expression Arrays User Manual (version PT3140-1/PR9X390, published 11/5/99). Quality of RNA samples was verified by purity calculations based on spec readings, and by visualization on agarose gel. Genomic contamination of the RNA samples was ruled out by PCR analysis of the beta-actin gene.

### Probe Synthesis

Clontech's protocols for polyA+ enrichment, probe synthesis and hybridization to Atlas^{™} arrays were followed (see above, plus Atlas^{™} Pure Total RNA Labeling System User Manual, PT3231-1/PR96157, published 6/22/99). Briefly, polyA+ RNA was isolated from 50 mg of total RNA using streptavidin coated magnetic beads (by Clontech, Paolo Alto, CA) and a magnetic particle separator. PolyA+ RNA was then labeled with ^{alpha32}P-dATP via RT-PCR. Clontech CDS primers specific to the 268 genes on the Atlas^{™} human cytokine/receptor array (Cat. #7744-1) were used in the reaction. Labeled probe was isolated using column chromatography and counted in scintillation fluid.

### Array membrane Hybridization

Atlas^{™} arrays were pre-hybridized with Clontech ExpressHyb plus 100 mg/mL heat denatured salmon sperm DNA for at least thirty minutes at 68°C with continuous agitation. Membranes were then hybridized with 1.9 x 10⁶ CPM/mL (a total of 1.14 x 10⁷ CPM) overnight at 68°C with continuous agitation. The following day, membranes were washed for thirty minutes x 4 in 2X SSC, 1% SDS at 68° C, plus for thirty minutes x 1 in 0.1X SSC, 0.5% SDS at 68°C, followed by one final room temperature wash for five minutes in 2X SSC. Array membranes were then placed in Kodak plastic pouches sealed and exposed to a phosphor imager screen overnight at room temperature. The next day, phosphor screens were scanned on a phosphor imager and analyzed using Clontech's AtlasImage^{™} 1.0 software.

### Results

### Genes Up-regulated by IL-20

1. Tumor necrosis factor (TNF) was up-regulated 1.9-2.4 fold by IL-20.
2. Placental growth factors 1 & 2 (PLGF) were up-regulated 1.9-2.0 fold by IL-20.
3. Coagulating factor II receptor was up-regulated 2.0-2.5 fold by IL-20.
4. Calcitonin receptor was up-regulated 2.2-2.3 fold by IL-20.
5. TNF-inducible hyaluronate-binding protein TSG-6 was up-regulated 2.1-2.2 fold by IL-20.
6. Vascular endothelial growth factor (VEGF) receptor-1 precursor, tyrosine-protein kinase receptor (FLT-1) (SFLT) was up-regulated 2.1-2.7 fold by IL-20.
7. MRP-8 (calcium binding protein in macrophages MIF- related) was up-regulated 2.9-4.1 fold by IL-20.
8. MRP-14 (calcium binding protein in macrophages MIF-related) was up-regulated 3.0-3.8 fold by IL-20.
9. Relaxin H2 was up-regulated 3.14 fold by IL-20.
10. Transforming growth factor beta (TGFβ) receptor III 300 kDa was up-regulated 2.4-3.6 fold by IL-20.

### Genes Showing Synergy with IL-20 + IL-1 Treatment

1. Bone morphogenic protein 2a was up-regulated 1.8 fold with IL-20 treatment alone, 2.5 fold with IL-1 treatment alone, and 8.2 fold with both IL-20 and IL-1 treatment together.
2. MRP-8 was up-regulated 2.9 fold with IL-20 treatment alone, 10.7 fold with IL-1 treatment alone and 18.0 fold with both IL-20 and IL-1 treatment together.
3. Erythroid differentiation protein (EDF) was up-regulated 1.9 fold with IL-20 treatment alone, 9.7 fold with IL-1 treatment alone and 19.0 fold with both IL-20 and IL-1 treatment together.
4. MRP-14 (calcium binding protein in macrophages, MIF related) was up-regulated 3.0 fold with IL-20 treatment alone, 12.2 fold with IL-1 treatment alone and 20.3 fold with both IL-20 and IL-1 treatment together.
5. Heparin-binding EGF-like growth factor was up-regulated 2.0 fold with IL-20 treatment alone, 14 fold with IL-1 treatment alone and 25.0 fold with both IL-20 and IL-1 treatment together.
6. Beta-thromboglobulin-like protein was up-regulated 1.5 fold with IL-20 treatment alone, 15 fold with IL-1 treatment alone and 27 fold with both IL-20 and IL-1 treatment together.
7. Brain-derived neurotrophic factor (BDNF) was up-regulated 1.7 fold with IL-20 treatment alone, 25 fold with IL-1 treatment alone and 48 fold with both IL-20 and IL-1 treatment together.
8. Monocyte chemotactic and activating factor MCAF was up-regulated 1.3 fold with IL-20 treatment alone, 32 fold with IL-1 treatment alone and 56 fold with both IL-20 and IL-1 treatment together.

### Example 5

### IL-20RA/RB Receptor-Ig fusion Heterotetramer

The expression vector pEZE3 was used to express the recombinant IL-20 receptor-Ig fusion protein. The plasmid pEZE3 is derived from pDC312. pDC312 was obtained through license from Immunex Corporation. The plasmids pDC312 and pEZE3 contain an EASE segment as described in WO 97/25420. The presence of the EASE segment in an expression vector can improve expression of recombinant proteins two to eight fold in stable cell pools.

The plasmid pEZE3 is a tricistronic expression vector that may be used to express up to three different proteins in mammalian cells, preferably Chinese Hamster Ovary (CHO) cells. The pEZE3 expression unit contains the cytomegalovirus (CMV) enhancer/promoter, the adenovirus tripartite leader sequence, a multiple cloning site for insertion of the coding region for the first recombinant protein, the poliovirus type 2 internal ribosome entry site, a second multiple cloning site for insertion of the coding region for the second recombinant protein, an encephalomyocarditis virus internal ribosome entry site, a coding segment for mouse dihydrofolate reductase, and the SV40 transcription terminator. In addition, pEZE3 contains an E. coli origin of replication and the bacterial beta lactamase gene.

The IL-20 receptor-Ig fusion protein is a disulfide linked heterotetramer consisting of two chains of the extracellular domain of the human IL-20RB fused to the wild type human immunoglobulin kappa light chain constant region and two chains of the human IL-20RA protein extracellular domain fused to a mutated human immunoglobulin gamma 1 constant region. The human immunoglobulin gamma 1 constant region contains amino acid substitutions to reduce FcγRI binding and C1q complement fixation.

The human IL-20RB extracellular domain human immunoglobulin kappa light chain constant region fusion construct was generated by overlap PCR. The IL-20RB coding segment consists of amino acids 1 to 230. The template used for the PCR amplification of the IL-20R segment was generated IL-20RB human kappa light chain constant region expression construct as described below in Example 12. Oligonucleotide primers SEQ ID NO: 24 and SEQ ID NO: 25 were used to amplify the IL-20RB segment. The entire wild type human immunoglobulin kappa light chain constant region was used. The template used for the PCR amplification of the wild type human immunoglobulin kappa light chain constant region segment was generated IL-20RB human kappa light chain constant region expression construct as described in Example 12. Oligonucleotide primers SEQ ID NO: 26 and SEQ ID NO: 27 were used to amplify the wild type human immunoglobulin kappa light chain constant region. The two protein coding domains were linked by overlap PCR using oligonucleotides SEQ ID NO: 24 and SEQ ID NO: 27. A (Gly₄Ser)₃ ( SEQ ID NO: 72) peptide linker was inserted between the two protein domains. The (Gly₄Ser)₃ peptide linker was encoded on the PCR primers SEQ ID NO: 26 and SEQ ID NO:25. The resultant IL-20RB extracellular domain/kappa light chain constant region fusion construct is shown by SEQ ID NOs: 20 and 21. The predicted mature polypeptide, minus the signal sequence, is SEQ ID NO: 60. The portion of the extracellular domain of IL-20RB that was actually used was comprised of the amino acid sequence of SEQ ID NO: 61. N-terminal sequencing resulted in the predicted amino acid sequence.

The human IL-20RA extracellular domain human immunoglobulin gamma 1 heavy chain constant region fusion construct was generated by overlap PCR of four separate DNA fragments, each generated by separate PCR amplification reactions. The first fragment contained an optimized tPA (tissue plasminogen activator) signal sequence. The tPA signal sequence was amplified using oligonucleotide primers SEQ ID NO: 28 and SEQ ID NO: 29 using an in-house previously generated expression vector as the template. The second fragment contained the IL-20RA extracellular domain-coding region consisting of amino acids 30 to 243 of SEQ ID NO: 11. Oligonucleotide primers SEQ ID NO: 30 and SEQ ID NO: 31 were used to amplify this IL-20RA segment using a previously generated clone of IL-20RA as the template.

The human gamma 1 heavy chain constant region was generated from 2 segments. The first segment containing the C_{H}1 domain was amplified using oligonucleotide primers SEQ ID NO: 32 and SEQ ID NO: 33 using a clone of the wild type human gamma 1 heavy chain constant region as the template. The second segment containing the remaining hinge, C_{H}2, and C_{H}3 domains of the human immunoglobulin gamma 1 heavy chain constant region was generated by PCR amplification using oligonucleotide primers SEQ ID NO: 34 and SEQ ID NO: 35. The template used for this PCR amplification was from a previously generated human gamma 1 Fc construct that contained codons for amino acid substitutions to reduce FcγRI binding and C1q complement fixation as described in Example 12.

The four protein coding domains were linked by overlap PCR using oligonucleotides SEQ ID NO: 28 and SEQ ID NO: 35. A (Gly₄Ser)₃ peptide linker was inserted between the IL-20RA and CH1 protein domains. The (Gly₄Ser)₃ peptide linker was encoded on the PCR primers SEQ ID NO: 32 and SEQ ID NO: 31. The IL-20RA extracellular domain/ domain human immunoglobulin gamma 1 heavy constant region fusion protein and DNA sequence are shown in SEQ ID NOs: 22 and 23. The predicted mature polypeptide sequence, minus the signal sequence, is SEQ ID NO: 62. The portion of extracellular domain of IL-20RA that was actually used was comprised of SEQ ID NO: 63.

The IL-20RB extracellular domain human immunoglobulin kappa light chain constant region fusion coding segment was cloned into the second MCS while the human IL-20RA extracellular domain human immunoglobulin gamma 1 heavy chain constant region fusion coding segment was cloned into the first MCS of pEZE3. The plasmid was used to transfect CHO cells. The cells were selected in medium without hypoxanthine or thymidine and the transgene was amplified using methotrexate. The presence of protein was assayed by Western blotting using anti human gamma 1 heavy chain constant region and anti human kappa light chain antibodies. N-terminal sequencing revealed that the optimized tPA leader was not completely cleaved. The observed mass indicated that the first residue of the polypeptide sequence to be pyroglutamic acid, and the N-terminal sequence appears to be pyroEEIHAELRRFRRVPCVSGG (SEQ ID NO: 64), the underlined portion being remnants of the tPA leader.

### Example 6

### IL-20 Transgenic Phenotype

Both human and mouse IL-20 were overexpressed in transgenic mice using a variety of promoters. The liver-specific mouse albumin promoter, directing expression of human IL-20, was used initially in an attempt to achieve circulating levels of protein. Subsequent studies were conducted using the keratin 14 (K14) promoter, which primarily targets expression to the epidermis and other stratified squamous epithelia; the mouse metallothionein-1 promoter, which gives a broad expression pattern; and the EµLCK promoter, which drives expression in cells of the lymphoid lineage. Similar results were obtained in all four cases, possibly because these promoters all give rise to circulating levels of IL-20.

In all cases, transgenic pups expressing the IL-20 transgene were smaller than non-transgenic littermates, had a shiny appearance with tight, wrinkled skin and died within the first few days after birth. Pups had milk in their stomachs indicating that they were able to suckle. These mice had swollen extremities, tail, nostril and mouth regions and had difficulty moving. In addition, the mice were frail, lacked visible adipose tissue and had delayed ear and toe development. Low expression levels in liver (less than 100 mRNA molecules/cell) were sufficient for both the neonatal lethality and skin abnormalities. Transgenic mice without a visible phenotype either did not express the transgene, did not express it at detectable levels, or were mosaic.

Histologic analysis of the skin of the IL-20 transgenic mice showed a thickened epidermis, hyperkeratosis and a compact stratum corneum compared to non-transgenic littermates. Serocellular crusts (scabs) were observed occasionally. Electron microscopic (EM) analysis of skin from transgenic mice showed intramitochondrial lipoid inclusions, mottled keratohyaline granules, and relatively few tonofilaments similar to that observed in human psoriatic skin and in mouse skin disease models. In addition, many of the transgenic mice had apoptotic thymic lymphocytes. No other abnormalities were detected by histopathological analysis. These histological and EM results support and extend the observed gross skin alterations.

### Example 7

### Specificity and Affinity of IL-20 for Its Receptor

The specificity and affinity of IL-20 for its receptor was determined using BHK cells stably transfected with IL-20RA, IL-20RB or both receptor subunits. Binding assays using radiolabeled ligand demonstrated that IL-20 bound to BHK transfectants expressing both IL-20RA and IL-20RB but not to untransfected cells nor to transfectants expressing either receptor subunit alone. Binding of ¹²⁵I-labeled IL-20 was eliminated in the presence of 100-fold excess of unlabeled IL-20 but not with 100-fold excess of the unrelated cytokine, IL-21. The binding affinity (k_{D}) of IL-20 to the IL-20RA/IL-20RB heterodimeric receptor was determined to be approximately 1.5 nM.

### Example 8

### IL-20 receptor activation

To determine if IL-20 binding leads to receptor activation, the factor-dependent pre-B cell line BaF3 was co-transfected with IL-20RA and IL-20RB and treated with IL-20 at various concentrations. IL-20 stimulated proliferation in a dose-dependent manner and gave a detectable signal at 1.1 pM, with a half maximal response at 3.4 pM. We note that the IL-20 concentration for the half maximal proliferative response in BaF3 cells is 1000X lower than that for half maximal binding affinity in BHK cells. Possible explanations for this large difference include the use of different cell lines, different receptor expression levels and different assay outputs. IL-20 also stimulated signal transduction in the biologically relevant human keratinocyte cell line HaCaT, which naturally expresses IL-20RA and IL-20RB. Therefore, IL-20 binds and activates the heterodimeric IL-20RA/IL-20RB receptor at concentrations expected for a cytokine. While the negative controls containing untransfected BaF3

### Example 9

### Expression Analysis of IL-20RA and IL-20RB

RT-PCR analysis was performed on a variety of human tissues to determine the expression pattern of IL-20RA and IL-20RB. Both receptor subunits are most highly expressed in skin and testis. The significant result is that IL-20RA and IL-20RB are both expressed in skin, where they have been shown to mediate the IL-20-induced response. Both IL-20RA and IL-20RB are also both expressed in monocytes, lung, ovary, muscle, testis, adrenal gland, heart, salivary gland and placenta. IL-20RA is also in brain, kidney, liver, colon, small intestine, stomach, thyroid, pancreas, uterus and prostate while IL-20RB is not.

### Example 10

### IL-20RA and IL-20RB mRNA are Up-regulated in Psoriasis

*In situ* hybridization was used to determine whether IL-20 receptor expression is altered in psoriasis. Skin samples from four psoriasis patients and three unaffected patients were assayed with probes specific for the two-receptor subunit mRNAs. All four psoriatic skin samples had high levels of IL-20RA and IL-20RB mRNA in keratinocytes whereas normal skin samples did not have detectable levels of either receptor subunit mRNA. Positive signals in psoriatic skin were also observed in mononuclear immune cells and in endothelial cells in a subset of vessels. Therefore, both IL-20RA and IL-20RB are expressed in keratinocytes, immune cells and endothelial cells, the major cell types thought to interact in psoriasis.

### Example 11

### Cloning of mouse IL-20RA

A cross-species hybridization probe was generated which contained the full-length cDNA fragment encoding human IL-20RA. A Southern blot of mouse genomic DNA and Northern blots of mouse RNA were performed to demonstrate that the human IL-20RA cDNA could specifically hybridize to mouse sequences. The Northern blot results indicated that mouse IL-20RA RNA was present in mouse embryo day 15 and 17 as well as heart, brain, lung, liver, kidney, testes, spleen, thymus, liver, stomach, and small intestine.

The human IL-20RA full length DNA hybridization probe was used to screen a mouse genomic library. The library, which was obtained from Clontech (Palo Alto, CA), was generated from an MboI partial digest of mouse genomic DNA and cloned into the BamHI site of Lambda bacteriophage EMBL3 SP6/T7. Positive bacteriophage was plaque purified and bacteriophage DNA was prepared using Promega's Wizard Lambda Preps DNA Purification System. Two genomic restriction enzyme fragments, a 5.7 kb EcoRI fragment and an 8.0 kb SacI fragment, were generated from the positive bacteriophage and subcloned into pBluescript. DNA sequence analysis revealed the presence of 3 exons from the mouse ortholog to human IL-20RA.

PCR primers from the 5' UTR, SEQ ID NO: 40, and 3' UTR, SEQ ID NO: 41, were designed to generate a full-length mouse IL-20RA sequence by PCR amplification. Mouse embryo 15day plus 17 day cDNA was used as the template for the PCR amplification. PCR products were subcloned and sequenced for confirmation. The mouse sequences are SEQ ID NOs: 36 and 37. The mature extracellular domain is comprised of SEQ ID NO: 38.

### Example 12

### Construction of an IL-20 Receptor Heterotetramer

A vector expressing a secreted hIL-20RA/hIL-20B heterodimer was constructed. In this construct, the extracellular domain of hIL-20RA was fused to the heavy chain of IgG gamma 1 (IgGγ1), while the extracellular portion of IL-20RB was fused to human kappa light chain (human κ light chain).

### Construction of IgG gamma 1 and human κ light fusion vectors

The heavy chain of IgGγ1 was cloned into the Zem229R mammalian expression vector (ATCC deposit No. 69447) such that any extracellular portion of a receptor having a 5' EcoRI and 3' NheI site can be cloned in, resulting in an N-terminal extracellular domain-C-terminal IgGγ1 fusion. The IgGγ1 fragment used in this construct was made by using PCR to isolate the IgGγ1 sequence from a Clontech human fetal liver cDNA library as template. A PCR reaction using oligos SEQ ID NO: 42 and SEQ ID NO: 43 was run as follows: 40 cycles of 94° for 60 sec., 53°C for 60 sec., and 72° for 120 sec.; and 72°C for 7 minutes. PCR products were separated by agarose gel electrophoresis and purified using a QiaQuick™ (Qiagen Inc., Valencia, CA) gel extraction kit. The isolated, 990 bp, DNA fragment was digested with MluI and EcoRI (Boerhinger-Mannheim), extracted with QiaQuick™ gel extraction kit and ligated with oligos SEQ ID NO: 44 and SEQ ID NO: 45, which comprise an MluI/EcoRI linker, into Zem229R previously digested with Mlul and EcoRI using standard molecular biology techniques disclosed herein. This generic cloning vector was called Vector#76 hIgGgammal w/ Ch1 #786 Zem229R (Vector #76). The polynucleotide sequence of the extracellular domain of hIL-20RA fused to the heavy chain of IgG gamma 1 is show in SEQ ID NO: 52 and the corresponding polypeptide sequence shown in SEQ ID NO: 53, the mature polypeptide, minus the signal sequence being comprised of SEQ ID NO: 54. The portion of the extracellular domain of IL-20RA used was comprised of SEQ ID NO: 55.

The human κ light chain was cloned in the Zem228R mammalian expression vector (ATCC deposit No. 69446) such that any extracellular portion of a receptor having a 5' EcoRI site and a 3' KpnI site can be cloned in, resulting in an N-terminal extracellular domain-C-terminal human κ light chain fusion. The human κ light chain fragment used in this construct was made by using PCR to isolate the human κ light chain sequence from the same Clontech hFetal Liver cDNA library used above. A PCR reaction was run using oligos SEQ ID NO: 46 and SEQ ID NO: 47. PCR products were separated by agarose gel electrophoresis and purified using a QiaQuick™ (Qiagen) gel extraction kit. The isolated, 315 bp, DNA fragment was digested with MluI and EcoRI (Boerhinger-Mannheim), extracted with QiaQuick™ gel extraction kit and ligated with the MluI/EcoRI linker described above, into Zem228R previously digested with MluI and EcoRI using standard molecular biology techniques disclosed herein. This generic cloning vector was called Vector #77 hκlight #774 Zem228R (Vector #77). The polynucleotide sequence of the extracellular portion of IL-20RB fused to human kappa light chain is shown in SEQ ID NO: 56 and the corresponding polypeptide sequence shown in SEQ ID NO: 57, the mature polypeptide, minus the signal sequence, is comprised of SEQ ID NO: 58. The portion of the extracellular domain of IL-20RB actually used was comprised of SEQ ID NO: 59.

### Insertion of hIL-20RA and IL-20RB extracellular domains into fusion vector constructs

Using the construction vectors above, a construct having human IL-20RA fused to IgGγ1 was made. This construction was done by using PCR to obtain human IL-20RA receptor from hIL-20RA /IgG Vector #102 with oligos SEQ ID NO: 48 and SEQ ID NO: 49 under conditions described as follows: 30 cycles of 94°C for 60 sec., 57°C for 60 sec., and 72°C for 120 sec.; and 72°C for 7 min. The resulting PCR product was digested with EcoRI and NheI, gel purified, as described herein, and ligated into a previously EcoRI and NheI digested and band-purified Vector #76 (above). The resulting vector was sequenced to confirm that the human IL-20Rα /IgG gamma 1 fusion (hIL-20RA /Ch1 IgG) was correct. The hIL-20RA /Ch1 IgG gamma 1 #1825 Zem229R vector was called vector #195. The IL-20RA/Ch1 IgGγ1 sequence thus obtained is depicted by SEQ ID NOs: 52 and 53. N-terminal sequencing indicated the presence of the predicted mature polypeptide sequence of SEQ ID NO: 54.

A separate construct having IL-20RB fused to κ light was also constructed. The IL-20RB/human κ light chain construction was performed as above by PCRing from DR1/7N-4 with oligos SEQ ID NO: 50 and SEQ ID NO: 51, digesting the resulting band with EcoRI and KpnI and then ligating this product into a previously EcoRI and KpnI digested and band-purified Vec#77 (above). The resulting vector was sequenced to confirm that the IL-20RB/ human κ light chain fusion (IL-20RB/κlight) was correct. This IL-20RB//κlight construct is shown by SEQ ID NOs: 56 and 57. N-terminal sequencing of the resultant polypeptide indicated the presence of the predicted mature amino acid sequence comprised of SEQ ID NO: 58. SEQ ID NO:59 is the mature portion of the extracellular domain of IL-20RB used.

### Co-expression of the human IL-20RA and human IL-20RB receptors

Approximately 16µg of each of vectors #194 and #195, above, were co-transfected into BHK-570 cells (ATCC No. CRL-10314) using Lipofectamine™ reagent (Gibco/BRL), as per manufacturer's instructions. The transfected cells were selected for 10 days in DMEM + 5%FBS (Gibco/BRL) containing 1µM of methotrexate (MTX) (Sigma, St. Louis, MO) and 0.5mg/ml G418 (Gibco/BRL) for 10 days. The resulting pool oftransfectants was selected again in 10µM MTX and 0.5mg/ml G418 for 10 days.

The resulting pool of doubly selected cells was used to generate protein. Three factories (Nunc, Denmark) of this pool were used to generate 8 L of serum free conditioned medium. This conditioned media was passed over a 1 ml protein-A column and eluted in (10) 750 microliter fractions. 4 of these fractions found to have the highest concentration were pooled and dialyzed (10 kD MW cutoff) against PBS. Finally, the dialyzed material was analyzed by BCA (Pierce) and found to have a concentration of 317 µg/ml. A total of 951 µg was obtained from this 8 L purification.

### Example 13

### IL-20 binding activates STAT3 in the HaCaT keratinocyte cell line

IL-20 binds cell lines transfected with both subunits of its receptor. However, these cell lines overexpress the IL-20 receptor relative to its normal level and their relevance to the physiological role of IL-20 is unclear. The human HaCaT keratinocyte cell line, which expresses endogenous IL-20RA and IL-20RB was used to examine IL-20 signal transduction in a biologically relevant cell type. HaCaT cells were infected with recombinant adenovirus containing a reporter construct to allow detection of intracellular signaling. The construct consists of the firefly luciferase gene driven by promoter/enhancer sequences comprised of the serum response element (SRE) and signal transducers and activators of transduction elements (STATs). This assay system detects productive ligand-receptor interactions and indicates possible downstream signal transduction components involved in receptor activation. Treatment with IL-20 alone resulted in a dose-dependent increase in luciferase activity with a half maximal response occurring at approximately 2.3 nM. Subsequent luciferase reporter assays using adenovirus vectors containing only the SRE element or only the STAT elements produced detectable reporter activation only through STATs.

To determine if other cytokines act in concert with IL-20, HaCaT cells were treated with IL-20 alone or in combination with a single submaximal dose of EGF, IL-1β, or TNFα. In the presence of each of these three proteins, IL-20 treatment resulted in a dose-dependent increase in luciferase activity. IL-20 in combination with IL-1β results in a half-maximal response at approximately 0.5 nM, about five-fold lower than with IL-20 alone. In addition, activation of the reporter gene is detectable at 0.1 nM IL-20, a dose that is at least tenfold lower than the IL-20 dose required alone.

BHK cells transfected with IL-20RA, IL-20RB or both receptor subunits were used to determine whether receptor pairing was required for IL-20 stimulation of STAT-luciferase. As was the case with binding assays, only cells transfected with both receptor subunits responded to IL-20 and did so with a half-maximal response of 5.7 pM. We note that the IL-20 concentration for the half-maximal response in BHK cells is 400-fold lower than that for half-maximal response in HaCaT cells. It is likely that a lower concentration of IL-20 is needed for half-maximal response in BHK cells, as compared to HaCaT cells, due to higher receptor levels in the BHK IL-20 receptor transfectants.

A nuclear translocation assay was used to identify STAT proteins involved in IL-20 action. Both HaCaT cells, with endogenous IL-20 receptors, and BHK cells transfected with IL-20RA and IL-20RB, were treated with IL-20 protein and translocation of STAT3 and STAT1 transcription factors from the cytoplasm to the nucleus was assayed by immunofluorescence.

In unstimulated HaCaT cells, STAT3 staining was predominantly in the cytosol. Treatment of HaCaT cells with IL-20 resulted in a distinct accumulation of STAT3 in the nucleus. Nuclear translocation of STAT3 in response to increasing concentrations of IL-20 occurred with a half-maximal IL-20 concentration of 7 nM. In contrast to STAT3 translocation, HaCaT cells treated with IL-20 did not show any detectable nuclear accumulation of STAT1.

BHK cells transfected with IL-20RA and IL-20RB were used to confirm that the IL-20 receptor was required for IL-20 stimulation of STAT3 nuclear translocation. In BHK cells lacking the IL-20 receptor, STAT3 remained cytosolic following treatment with IL-20. In contrast, in BHK cells transfected with the IL-20 receptor, STAT3 translocated to the nucleus in response to IL-20. Again, STAT1 remained cytosolic regardless of IL-20 treatment or IL-20 receptor expression. We conclude that the IL-20 receptor is required for IL-20-mediated STAT3 activation.

## Claims

1. A polynucleotide that encodes a receptor comprising an IL-20RA subunit and an IL-20RB subunit, wherein the IL-20RA subunit comprises a polypeptide comprising an amino acid sequence selected from the group consisting of SEQ ID NOs: 12, 38, 55, 63, and 65, and the IL-20RB subunit comprises a polypeptide comprising an amino acid sequence selected from the group consisting of SEQ ID NOs: 15, 59, 61, 67, 68 and 69, and wherein the IL-20RA subunit and the IL-20RB subunit are covalently linked together by a linkage selected from one or more polypeptide linkers and one or more disulphide bonds.

2. The polynucleotide according to Claim 1, wherein the encoded receptor is a soluble receptor.

3. The polynucleotide according to Claim 1, wherein the encoded receptor forms a multimeric, heterodimeric, or heterotetrameric receptor complex.

4. The polynucleotide according to any of Claims 1-3, wherein the encoded receptor further comprises glycosylation or an affinity tag.

5. The polynucleotide according to Claim 4, wherein the affinity tag is a maltose-binding protein or an immunoglobulin domain.

6. The polynucleotide according to any previous claim, wherein the encoded receptor comprises a polypeptide linker to link the IL-20RA and IL-20RB subunits.

7. The polynucleotide according to Claim 6, wherein the encoded linker comprises 100 to 240 amino acid residues.

8. The polynucleotide according to Claim 6, wherein the linker comprises 170 amino acid residues.

9. The polynucleotide according to any previous claim, wherein the polynucleotide encodes a receptor comprising a polypeptide linker fused to the IL-20RA subunit and a polypeptide linker fused to the IL-20RB subunit.

10. The polynucleotide according to Claim 9, wherein each of the encoded polypeptide linkers has at least one cysteine residue, and wherein at least one disulfide bond forms with a cysteine from the polypeptide linker of the IL-20RA subunit and with a cysteine from the polypeptide linker of the IL-20RB subunit.

11. The polynucleotide according to Claim 9 or 10, wherein the encoded receptor comprising the IL-20RA subunit disulfide bonded to an IL-20RB subunit is disulfide bonded to another receptor comprising an IL-20RA subunit disulfide bonded to an IL-20RB subunit.

12. The polynucleotide according to any of Claims 9-11, wherein the polynucleotide encodes the IL-20RA subunit fused to all or a portion of the constant region of a heavy chain of an immunoglobulin (Ig) molecule, and wherein the polynucleotide encodes the IL-20RB subunit fused to all or a portion of the constant region of a light chain of an immunoglobulin molecule.

13. The polynucleotide according to any of Claims 9-11, wherein the polynucleotide encodes the IL-20RB subunit fused to all or a portion of the constant region of a heavy chain of an immunoglobulin (Ig) molecule, and wherein the polynucleotide encodes the IL-20RA subunit fused to all or a portion of the constant region of a light chain of an immunoglobulin molecule.

14. The polynucleotide according to Claim 12 or 13, wherein the light chain and the heavy chain are disulfide bonded together.

15. The polynucleotide according to any of Claims 12-14, wherein the encoded receptor comprises a CH1 domain, a CH2 domain, a CH3 domain, or a CH4 domain.

16. The polynucleotide according to any of Claims 12-15, wherein the encoded receptor comprises a CH1 domain and a CH2 domain.

17. The polynucleotide according to Claim 16, wherein the encoded receptor comprises a CH1 domain, a CH2 domain, and a CH3 domain.

18. The polynucleotide according to any of Claims 9-11, wherein the receptor comprises a hinge domain.

19. The polynucleotide according to Claim 12, wherein the encoded IL-20RA subunit fused to the constant region of the heavy chain comprises an amino acid sequence selected from the group consisting of SEQ ID NOs: 23, 53, 54 and 62, and the encoded IL-20RB subunit fused to the constant region of the light chain of the Ig molecule comprises an amino acid sequence selected from the group consisting of SEQ ID NOs: 21, 57, 58, and 60.

20. The polynucleotide according to Claim 2, wherein the encoded IL-20RA subunit comprises an amino acid selected from group consisting of SEQ ID NOs: 53 and 54, and the encoded IL-20RB subunit comprises an amino acid sequence selected from the group consisting of SEQ ID NOs: 57 and 58.

21. The polynucleotide according to Claim 2, wherein the encoded IL-20RA subunit comprises an amino acid selected from group consisting SEQ ID NOs: 23 and 62, and the IL-20RB subunit comprises an amino acid sequence selected from the group consisting of SEQ ID NOs: 21 and 60.

22. The polynucleotide according to Claim 20 or 21, wherein the encoded soluble receptor comprising an IL-20RA subunit disulfide bonded to an IL-20RB subunit is disulfide bonded to another receptor comprising an IL-20RA subunit disulfide bonded to an IL-20RB subunit.

23. A polynucleotide that encodes a polypeptide comprising the amino acid sequence of SEQ ID NO: 66 and an amino acid sequence selected from the group consisting of SEQ ID NOs: 70 and 71, and wherein the amino acid sequences are covalently joined together by a linkage selected from one or more polypeptide linkers and one or more disulphide bonds.

24. An expression vector comprising the following operably linked elements:
a transcription promoter,
a DNA segment encoding a receptor, wherein said receptor is encoded by a polynucleotide according to any of Claims 1-23; and
a transcription terminator.

25. The expression vector according to Claim 24, wherein the encoded IL-20RB subunit further comprises all or a portion of an immunoglobulin light chain constant region, and wherein the encoded IL-20RA subunit comprises all or a portion of an immunoglobulin heavy chain constant region.

26. The expression vector according to Claim 24, wherein the encoded IL-20RA subunit further comprises all or a portion of an immunoglobulin light chain constant region, and wherein the encoded IL-20RB subunit comprises all or a portion of an immunoglobulin heavy chain constant region.

27. An expression vector comprising the following operably linked elements:
(a) a first transcription promoter; a first DNA segment that encodes a first polypeptide comprising an IL-20RA subunit and all or a portion of an immunoglobulin heavy chain constant region; and a first transcription terminator; and
(b) a second transcription promoter; a second DNA segment that encodes a second polypeptide comprising an IL-20RB subunit and all or a portion of an immunoglobulin light chain constant region; and a second transcription terminator,
wherein the IL-20RA subunit comprises a polypeptide comprising an amino acid sequence selected from the group consisting of SEQ ID NOs: 12, 38, 55, 63, and 65, and the IL-20RB subunit comprises a polypeptide comprising an amino acid sequence selected from the group consisting of SEQ ID NOs: 15, 59, 61, 67, 68 and 69, and wherein the first and second polypeptides form a receptor, wherein said receptor is encoded by a polynucleotide according to any of Claims 1-23.

28. An expression vector comprising the following operably linked elements:
(a) a first transcription promoter; a first DNA segment that encodes a first polypeptide comprising an IL-20RA subunit and all or a portion of an immunoglobulin light chain constant region; and a first transcription terminator; and
(b) a second transcription promoter; a second DNA segment that encodes a second polypeptide comprising an IL-20RB subunit and all or a portion of an immunoglobulin heavy chain constant region; and a second transcription terminator,
wherein the IL-20RA subunit comprises a polypeptide comprising an amino acid sequence selected from the group consisting of SEQ ID NOs: 12, 38, 55, 63, and 65, and the IL-20RB subunit comprises a polypeptide comprising an amino acid sequence selected from the group consisting of SEQ ID NOs: 15, 59, 61, 67, 68 and 69, and wherein the first and second polypeptides form a receptor, wherein said receptor is encoded by a polynucleotide according to any of Claims 1-23.

29. The expression vector according to any of Claims 24-28, wherein the receptor is a soluble receptor.

30. The expression vector according to Claim 29, wherein the receptor forms a multimeric, heterodimeric, or heterotetrameric receptor complex.

31. The expression vector according to Claim 25 or 27, wherein the DNA sequence encoding the IL-20RA subunit encodes an immunoglobulin heavy chain hinge region, wherein the hinge region is joined to the heavy chain constant region domain.

32. The expression vector according to Claim 25 or 27, wherein the DNA sequence encoding IL-20RA subunit further encodes an immunoglobulin variable region.

33. The expression vector according to Claim 26 or 28, wherein the DNA sequence encoding the IL-20RB subunit encodes an immunoglobulin heavy chain hinge region wherein the hinge region is joined to the heavy chain constant region domain.

34. The expression vector according to Claim 26 or 28, wherein the DNA sequence encoding IL-20RB subunit further encodes an immunoglobulin variable region.

35. A cultured cell comprising the expression vector according to any of Claims 24 to 34, wherein the cell expresses a receptor encoded by the vector.

36. A method of producing a receptor comprising culturing a cell according to Claim 35, and isolating the receptor produced by the cell.

## Patentansprüche

1. Polynukeotid, welches einen Rezeptor, umfassend eine IL-20RA-Untereinheit und eine IL-20RB-Untereinheit, kodiert, wobei die IL-20RA-Untereinheit ein Polypeptid, umfassend eine Aminosäuresequenz, welche aus der Gruppe ausgewählt ist, die aus SEQ ID Nr.: 12, 38, 55, 63 und 65 besteht, umfasst und die IL-20RB-Untereinheit ein Polypeptid, umfassend eine Aminosäuresequenz, welche aus der Gruppe ausgewählt ist, die aus SEQ ID Nr.: 15, 59, 61, 67, 68 und 69 besteht, umfasst und wobei die IL-20RA-Untereinheit und die IL-20RB-Untereinheit kovalent zusammen gebunden sind durch eine Bindung, ausgewählt aus einem oder mehreren Polypeptidlinkern und einer oder mehreren Disulfidbindungen.

2. Polynukleotid gemäß Anspruch 1, wobei der kodierte Rezeptor ein löslicher Rezeptor ist.

3. Polynukleotid gemäß Anspruch 1, wobei der kodierte Rezeptor einen multimeren, heterodimeren oder heterotetrameren Rezeptorkomplex bildet.

4. Polynukleotid gemäß einem der Ansprüche 1 bis 3, wobei der kodierte Rezeptor ferner Glycosylierung oder ein Affmitäts-Tag umfasst.

5. Polynukleotid gemäß Anspruch 4, wobei das Affinitäts-Tag ein Maltose-bindendes Protein oder eine Immunglobulindomäne ist.

6. Polynukleotid gemäß einem vorstehenden Anspruch, wobei der kodierte Rezeptor einen Polypeptidlinker zum Verbinden der IL-20RA- und IL-20RB-Untereinheiten umfasst.

7. Polynukleotid gemäß Anspruch 6, wobei der kodierte Linker 100 bis 240 Aminosäurereste umfasst.

8. Polynukleotid gemäß Anspruch 6, wobei der Linker 170 Aminosäurereste umfasst.

9. Polynukleotid gemäß einem vorstehenden Anspruch, wobei das Polynukleotid einen Rezeptor, umfassend einen Polypeptidlinker, welcher an die IL-20RA-Untereinheit fusioniert ist, und einen Polypeptidlinker, welcher an die IL-20RB-Unter-einheit fusioniert ist, kodiert.

10. Polynukleotid gemäß Anspruch 9, wobei jeder der kodierten Polypeptidlinker mindestens einen Cysteinrest aufweist und wobei mindestens eine Disulfidbindung mit einem Cystein von dem Polypeptidlinker der IL-20RA-Untereinheit und mit einem Cystein von dem Polypeptidlinker der IL-20RB-Untereinheit gebildet wird.

11. Polynukleotid gemäß Anspruch 9 oder 10, wobei der kodierte Rezeptor, umfassend die IL-20RA-Untereinheit, welche an eine IL-20RB-Untereinheit disulfidgebunden ist, an einen anderen Rezeptor, umfassend eine IL-20RA-Untereinheit, welche an eine IL-20RB-Untereinheit disulfidgebunden ist, disulfidgebunden ist.

12. Polynukleotid gemäß einem der Ansprüche 9 bis 11, wobei das Polynukleotid die IL-20RA-Untereinheit, fusioniert an die gesamte oder einen Teil der konstanten Region einer schweren Kette eines Immunglobulin (Ig)-Moleküls, kodiert und wobei das Polynukleotid die IL-20RB-Untereinheit, fusioniert an die gesamte oder einen Teil der konstanten Region einer leichten Kette eines Immunglobulinmoleküls, kodiert.

13. Polynukleotid gemäß einem der Ansprüche 9 bis 11, wobei das Polynukleotid die IL-20RB-Untereinheit, fusioniert an die gesamte oder einen Teil der konstanten Region einer schweren Kette eines Immunglobulin (Ig)-Moleküls, kodiert und wobei das Polynukleotid die IL-20RA-Untereinheit, fusioniert an die gesamte oder einen Teil der konstanten Region einer leichten Kette eines Immunglobulinmoleküls, kodiert.

14. Polynukleotid gemäß Anspruch 12 oder 13, wobei die leichte Kette und die schwere Kette zusammen disulfidgebunden sind.

15. Polynukleotid gemäß einem der Ansprüche 12 bis 14, wobei der kodierte Rezeptor eine CH1-Domäne, eine CH2-Domäne, eine CH3-Domäne oder eine CH4-Domäne umfasst.

16. Polynukleotid gemäß einem der Ansprüche 12 bis 15, wobei der kodierte Rezeptor eine CH1-Domäne und eine CH2-Domäne umfasst.

17. Polynukleotid gemäß Anspruch 16, wobei der kodierte Rezeptor eine CH1-Domäne, eine CH2-Domäne und eine CH3-Domäne umfasst.

18. Polynukleotid gemäß einem der Ansprüche 9 bis 11, wobei der Rezeptor eine Hinge-Domäne umfasst.

19. Polynukleotid gemäß Anspruch 12, wobei die kodierte IL-20RA-Untereinheit, fusioniert an die konstante Region der schweren Kette, eine Aminosäuresequenz umfasst, welche aus der Gruppe ausgewählt ist, die aus SEQ ID Nr.: 23, 53, 54 und 62 besteht, und wobei die kodierte IL-20RB-Untereinheit, fusioniert an die konstante Region der leichten Kette des Ig-Moleküls, eine Aminosäuresequenz umfasst, welche aus der Gruppe ausgewählt ist, die aus SEQ ID Nr.: 21, 57, 58 und 60 besteht.

20. Polynukleotid gemäß Anspruch 2, wobei die kodierte IL-20RA-Untereinheit eine Aminosäure umfasst, welche aus der Gruppe ausgewählt ist, die aus SEQ ID Nr.: 53 und 54 besteht, und wobei die kodierte IL-20RB-Untereinheit eine Aminosäuresequenz umfasst, welche aus der Gruppe ausgewählt ist, die aus SEQ ID Nr.: 57 und 58 besteht.

21. Polynukleotid gemäß Anspruch 2, wobei die kodierte IL-20RA-Untereinheit eine Aminosäure umfasst, welche aus der Gruppe ausgewählt ist, die aus SEQ ID Nr.: 23 und 62 besteht, und wobei die IL-20RB-Untereinheit eine Aminosäuresequenz umfasst, welche aus der Gruppe ausgewählt ist, die aus SEQ ID Nr.: 21 und 60 besteht.

22. Polynukleotid gemäß Anspruch 20 oder 21, wobei der kodierte lösliche Rezeptor, umfassend eine IL-20RA-Untereinheit, welche an eine IL-20RB-Untereinheit disulfidgebunden ist, an einen anderen Rezeptor, umfassend eine IL-20RA-Untereinheit, welche an eine IL-20RB-Untereinheit disulfidgebunden ist, disulfidgebunden ist.

23. Polynukleotid, welches ein Polypeptid kodiert, das die Aminosäuresequenz SEQ ID Nr.: 66 und eine Aminosäuresequenz, welche aus der Gruppe ausgewählt ist, die aus SEQ ID Nr.: 70 und 71 besteht, umfasst, und wobei die Aminosäuresequenzen zusammen kovalent gebunden sind durch eine Bindung, welche aus einem oder mehr Polypeptidlinkern und einer oder mehreren Disulfidbindungen ausgewählt ist.

24. Expressionsvektor, umfassend die folgenden funktionell verbundenen Elemente:
einen Transkriptionspromotor,
ein DNA-Segment, welches einen Rezeptor kodiert, wobei der Rezeptor durch ein Polynukleotid gemäß einem der Ansprüche 1 bis 23 kodiert wird; und
einen Transkriptionsterminator.

25. Expressionsvektor gemäß Anspruch 24, wobei die kodierte IL-20RB-Untereinheit ferner die gesamte oder einen Teil der konstanten Region einer leichten Kette eines Immunglobulins umfasst und wobei die kodierte IL-20RA-Untereinheit die gesamte oder einen Teil der konstanten Region einer schweren Kette eines Immunglobulins umfasst.

26. Expressionsvektor gemäß Anspruch 24, wobei die kodierte IL-20RA-Untereinheit ferner die gesamte oder einen Teil der konstanten Region einer leichten Kette eines Immunglobulins umfasst und wobei die kodierte IL-20RB-Untereinheit die gesamte oder einen Teil der konstanten Region einer schweren Kette eines Immunglobulins umfasst.

27. Expressionsvektor, umfassend die folgenden funktionell verbundenen Elemente:
(a) einen ersten Transkriptionspromotor; ein erstes DNA-Segment, welches ein erstes Polypeptid kodiert, das eine IL-20RA-Untereinheit und die gesamte oder einen Teil der konstanten Region einer schweren Kette eines Immunglobulins umfasst; und einen ersten Transkriptionsterminator; und
(b) einen zweiten Transkriptionspromotor; ein zweites DNA-Segment, welches ein zweites Polypeptid kodiert, das eine IL-20RB-Untereinheit und die gesamte oder einen Teil der konstanten Region einer leichten Kette eines Immunglobulins umfasst; und einen zweiten Transkriptionsterminator,
wobei die IL-20RA-Untereinheit ein Polypeptid umfasst, welches eine Aminosäuresequenz umfasst, die aus der Gruppe ausgewählt ist, welche aus SEQ ID Nr.: 12, 38, 55, 63 und 65 besteht, und wobei die IL-20RB-Untereinheit ein Polypeptid umfasst, welches eine Aminosäuresequenz umfasst, die aus der Gruppe ausgewählt ist, welche aus SEQ ID Nr.: 15, 59, 61, 67, 68 und 69 besteht, und wobei die ersten und zweiten Polypeptide einen Rezeptor bilden, wobei der Rezeptor durch ein Polynukleotid gemäß einem der Ansprüche 1 bis 23 kodiert wird.

28. Expressionsvektor, umfassend die folgenden funktionell verbundenen Elemente:
(a) einen ersten Transkriptionspromotor; ein erstes DNA-Segment, welches ein erstes Polypeptid kodiert, das eine IL-20RA-Untereinheit und die gesamte oder einen Teil der konstanten Region einer leichten Kette eines Immunglobulins umfasst; und einen ersten Transkriptionsterminator; und
(b) einen zweiten Transkriptionspromotor; ein zweites DNA-Segment, welches ein zweites Polypeptid kodiert, das eine IL-20RB-Untereinheit und die gesamte oder einen Teil der konstanten Region einer schweren Kette eines Immunglobulins umfasst; und einen zweiten Transkriptionsterminator,
wobei die IL-20RA-Untereinheit ein Polypeptid umfasst, welches eine Aminosäuresequenz umfasst, die aus der Gruppe ausgewählt ist, welche aus SEQ ID Nr.: 12, 38, 55, 63 und 65 besteht, und wobei die IL-20RB-Untereinheit ein Polypeptid umfasst, welches eine Aminosäuresequenz umfasst, die aus der Gruppe ausgewählt ist, welche aus SEQ ID Nr.: 15, 59, 61, 67, 68 und 69 besteht, und wobei die ersten und zweiten Polypeptide einen Rezeptor bilden, wobei der Rezeptor durch ein Polynukleotid gemäß einem der Ansprüche 1 bis 23 kodiert wird.

29. Expressionsvektor gemäß einem der Ansprüche 24 bis 28, wobei der Rezeptor ein löslicher Rezeptor ist.

30. Expressionsvektor gemäß Anspruch 29, wobei der Rezeptor einen multimeren, heterodimeren oder heterotetrameren Rezeptorkomplex bildet.

31. Expressionsvektor gemäß Anspruch 25 oder 27, wobei die DNA-Sequenz, welche die IL-20RA-Untereinheit kodiert, eine Hinge-Region einer schweren Kette eines Immunglobulins kodiert, wobei die Hinge-Region an die Domäne der konstanten Region der schweren Kette gebunden ist.

32. Expressionsvektor gemäß Anspruch 25 oder 27, wobei die DNA-Sequenz, welche eine IL-20RA-Untereinheit kodiert, ferner eine variable Region eines Immunglobulins kodiert.

33. Expressionsvektor gemäß Anspruch 26 oder 28, wobei die DNA-Sequenz, welche die IL-20RB-Untereinheit kodiert, eine Hinge-Region einer schweren Kette eines Immunglobulins kodiert, wobei die Hinge-Region an die Domäne der konstanten Region der schweren Kette gebunden ist.

34. Expressionsvektor gemäß Anspruch 26 oder 28, wobei die DNA-Sequenz, welche eine IL-20RB-Untereinheit kodiert, ferner eine variable Region eines Immunglobulins kodiert.

35. Kultivierte Zelle, umfassend den Expressionsvektor gemäß einem der Ansprüche 24 bis 34, wobei die Zelle einen Rezeptor, welcher durch den Vektor kodiert wird, exprimiert.

36. Verfahren zum Herstellen eines Rezeptors, umfassend das Kultivieren einer Zelle gemäß Anspruch 35 und das Isolieren des durch die Zelle hergestellten Rezeptors.

## Revendications

1. Polynucléotide qui code un récepteur comprenant une sous-unité IL-20RA et une sous-unité IL-20RB, où la sous-unité IL-20RA comprend un polypeptide comprenant une séquence d'aminoacides choisie dans le groupe consistant en SEQ ID NO : 12, 38, 55, 63 et 65, et la sous-unité IL-20RB comprend un polypeptide comprenant une séquence d'aminoacides choisie dans le groupe consistant en SEQ ID NO : 15, 59, 61, 67, 68 et 69, et où la sous-unité IL-20RA et la sous-unité IL-20RB sont liées entre elles par covalence par un lien choisi parmi un ou plusieurs lieurs polypeptidiques et une ou plusieurs liaisons disulfure.

2. Polynucléotide selon la revendication 1 où le récepteur codé est un récepteur soluble.

3. Polynucléotide selon la revendication 1 où le récepteur codé forme un complexe récepteur multimérique, hétérodimérique ou hétérotétramérique.

4. Polynucléotide selon l'une quelconque des revendications 1-3 où le récepteur codé comprend en outre une glycosylation ou un marqueur d'affinité.

5. Polynucléotide selon la revendication 4 où le marqueur d'affinité est une protéine liant le maltose ou un domaine d'immunoglobuline.

6. Polynucléotide selon l'une quelconque des revendications précédentes où le récepteur codé comprend un lieur polypeptidique pour lier les sous-unités IL-20RA et IL-20RB.

7. Polynucléotide selon la revendication 6 où le lieur codé comprend 100 à 240 résidus d'aminoacides.

8. Polynucléotide selon la revendication 6 où le lieur comprend 170 résidus d'aminoacides.

9. Polynucléotide selon l'une quelconque des revendications précédentes où le polynucléotide code un récepteur comprenant un lieur polypeptidique fusionné à la sous-unité IL-20RA et un lieur polypeptidique fusionné à la sous-unité IL-20RB.

10. Polynucléotide selon la revendication 9 où chacun des lieurs polypeptidiques codés a au moins un résidu cystéine, et où au moins une liaison disulfure se forme avec une cystéine du lieur polypeptidique de la sous-unité IL-20RA et avec une cystéine du lieur polypeptidique de la sous-unité IL-20RB.

11. Polynucléotide selon la revendication 9 ou 10 où le récepteur codé comprenant la sous-unité IL-20RA liée par disulfure à une sous-unité IL-20RB est lié par disulfure à un autre récepteur comprenant une sous-unité IL-20RA liée par disulfure à une sous-unité IL-20RB.

12. Polynucléotide selon l'une quelconque des revendications 9-11 où le polynucléotide code la sous-unité IL-20RA fusionnée à tout ou partie de la région constante d'une chaîne lourde d'une molécule d'immunoglobuline (Ig), et où le polynucléotide code la sous-unité IL-20RB fusionnée à tout ou partie de la région constante d'une chaîne légère d'une molécule d'immunoglobuline.

13. Polynucléotide selon l'une quelconque des revendications 9-11 où le polynucléotide code la sous-unité IL-20RB fusionnée à tout ou partie de la région constante d'une chaîne lourde d'une molécule d'immunoglobuline (Ig), et où le polynucléotide code la sous-unité IL-20RA fusionnée à tout ou partie de la région constante d'une chaîne légère d'une molécule d'immunoglobuline.

14. Polynucléotide selon la revendication 12 ou 13 où la chaîne légère et la chaîne lourde sont liées entre elles par disulfure.

15. Polynucléotide selon l'une quelconque des revendications 12-14 où le récepteur codé comprend un domaine CH1, un domaine CH2, un domaine CH3 ou un domaine CH4.

16. Polynucléotide selon l'une quelconque des revendications 12-15 où le récepteur codé comprend un domaine CH1 et un domaine CH2.

17. Polynucléotide selon la revendication 16 où le récepteur codé comprend un domaine CH1, un domaine CH2 et un domaine CH3.

18. Polynucléotide selon l'une quelconque des revendications 9-11 où le récepteur comprend un domaine de charnière.

19. Polynucléotide selon la revendication 12 où la sous-unité IL-20RA codée fusionnée à la région constante de la chaîne lourde comprend une séquence d'aminoacides choisie dans le groupe consistant en SEQ ID NO : 23, 53, 54 et 62, et la sous-unité IL-20RB codée fusionnée à la région constante de la chaîne légère de la molécule d'Ig comprend une séquence d'aminoacides choisie dans le groupe consistant en SEQ ID NO : 21, 57, 58 et 60.

20. Polynucléotide selon la revendication 2 où la sous-unité IL-20RA codée comprend un aminoacide choisi dans le groupe consistant en SEQ ID NO : 53 et 54, et la sous-unité IL-20RB codée comprend une séquence d'aminoacides choisie dans le groupe consistant en SEQ ID NO : 57 et 58.

21. Polynucléotide selon la revendication 2 où la sous-unité IL-20RA codée comprend un aminoacide choisi dans le groupe consistant en SEQ ID NO : 23 et 62, et la sous-unité IL-20RB comprend une séquence d'aminoacides choisie dans le groupe consistant en SEQ ID NO : 21 et 60.

22. Polynucléotide selon la revendication 20 ou 21 où le récepteur soluble codé comprenant une sous-unité IL-20RA liée par disulfure à une sous-unité IL-20RB est lié par disulfure à un autre récepteur comprenant une sous-unité IL-20RA liée par disulfure à une sous-unité IL-20RB.

23. Polynucléotide qui code un polypeptide comprenant la séquence d'aminoacides de SEQ ID NO : 66 et une séquence d'aminoacides choisie dans le groupe consistant en SEQ ID NO : 70 et 71, et où les séquences d'aminoacides sont jointes entre elles par covalence par un lien choisi parmi un ou plusieurs lieurs polypeptidiques et une ou plusieurs liaisons disulfure.

24. Vecteur d'expression comprenant les éléments liés de manière fonctionnelle suivants :
un promoteur de transcription,
un segment d'ADN codant un récepteur, où ledit récepteur est codé par un polynucléotide selon l'une quelconque des revendications 1-23 ; et
un terminateur de transcription.

25. Vecteur d'expression selon la revendication 24 où la sous-unité IL-20RB codée comprend en outre tout ou partie d'une région constante de chaîne légère d'immunoglobuline, et où la sous-unité IL-20RA codée comprend tout ou partie d'une région constante de chaîne lourde d'immunoglobuline.

26. Vecteur d'expression selon la revendication 24 où la sous-unité IL-20RA codée comprend en outre tout ou partie d'une région constante de chaîne légère d'immunoglobuline, et où la sous-unité IL-20RB codée comprend tout ou partie d'une région constante de chaîne lourde d'immunoglobuline.

27. Vecteur d'expression comprenant les éléments liés de manière fonctionnelle suivants :
(a) un premier promoteur de transcription ; un premier segment d'ADN qui code un premier polypeptide comprenant une sous-unité IL-20RA et tout ou partie d'une région constante de chaîne lourde d'immunoglobuline ; et un premier terminateur de transcription ; et
(b) un second promoteur de transcription ; un second segment d'ADN qui code un second polypeptide comprenant une sous-unité IL-20RB et tout ou partie d'une région constante de chaîne légère d'immunoglobuline ; et un second terminateur de transcription,
où la sous-unité IL-20RA comprend un polypeptide comprenant une séquence d'aminoacides choisie dans le groupe consistant en SEQ ID NO : 12, 38, 55, 63 et 65, et la sous-unité IL-20RB comprend un polypeptide comprenant une séquence d'aminoacides choisie dans le groupe consistant en SEQ ID NO : 15, 59, 61, 67, 68 et 69, et où les premier et second polypeptides forment un récepteur, où ledit récepteur est codé par un polynucléotide selon l'une quelconque des revendications 1-23.

28. Vecteur d'expression comprenant les éléments liés de manière fonctionnelle suivants :
(a) un premier promoteur de transcription ; un premier segment d'ADN qui code un premier polypeptide comprenant une sous-unité IL-20RA et tout ou partie d'une région constante de chaîne légère d'immunoglobuline ; et un premier terminateur de transcription ; et
(b) un second promoteur de transcription ; un second segment d'ADN qui code un second polypeptide comprenant une sous-unité IL-20RB et tout ou partie d'une région constante de chaîne lourde d'immunoglobuline ; et un second terminateur de transcription,
où la sous-unité IL-20RA comprend un polypeptide comprenant une séquence d'aminoacides choisie dans le groupe consistant en SEQ ID NO : 12, 38, 55, 63 et 65, et la sous-unité IL-20RB comprend un polypeptide comprenant une séquence d'aminoacides choisie dans le groupe consistant en SEQ ID NO : 15, 59, 61, 67, 68 et 69, et où les premier et second polypeptides forment un récepteur, où ledit récepteur est codé par un polynucléotide selon l'une quelconque des revendications 1-23.

29. Vecteur d'expression selon l'une quelconque des revendications 24-28 où le récepteur est un récepteur soluble.

30. Vecteur d'expression selon la revendication 29 où le récepteur forme un complexe récepteur multimérique, hétérodimérique ou hétérotétramérique.

31. Vecteur d'expression selon la revendication 25 ou 27 où la séquence d'ADN codant la sous-unité IL-20RA code une région charnière de chaîne lourde d'immunoglobuline, où la région charnière est jointe au domaine de région constante de chaîne lourde.

32. Vecteur d'expression selon la revendication 25 ou 27 où la séquence d'ADN codant la sous-unité IL-20RA code en outre une région variable d'immunoglobuline.

33. Vecteur d'expression selon la revendication 26 ou 28 où la séquence d'ADN codant la sous-unité IL-20RB code une région charnière de chaîne lourde d'immunoglobuline, où la région charnière est jointe au domaine de région constante de chaîne lourde.

34. Vecteur d'expression selon la revendication 26 ou 28 où la séquence d'ADN codant la sous-unité IL-20RB code en outre une région variable d'immunoglobuline.

35. Cellule cultivée comprenant le vecteur d'expression selon l'une quelconque des revendications 24 à 34, où la cellule exprime un récepteur codé par le vecteur.

36. Procédé de production d'un récepteur comprenant la culture d'une cellule selon la revendication 35 et l'isolement du récepteur produit par la cellule.


### SEQUENCE LISTING

<110> ZymoGenetics, Inc.
<120> Soluble Interleukin-20 Receptor
<130> 99-107PC
<150> 09/471,774
   <151> 1999-12-23
<150> 60/213,416
   <151> 2000-06-22
<160> 72
<170> FastSEQ for Windows Version 3.0
<210> 1
   <211> 176
   <212> PRT
   <213> Homo sapiens
<400> 1
<210> 2
   <211> 152
   <212> PRT
   <213> Homo sapiens
<400> 2
   Leu Lys Thr Leu Asn Leu Gly Ser Cys Val Ile Ala Thr Asn Leu
   Gln
   1 5 10 15
   Glu Ile Arg Asn Gly Phe Ser Asp Ile Arg Gly Ser Val Gln Ala
   Lys
   20 25 30
   Asp Gly Asn Ile Asp Ile Arg Ile Leu Arg Arg Thr Glu Ser Leu
   Gln
   35 40 45
   Asp Thr Lys Pro Ala Asn Arg Cys Cys Leu Leu Arg His Leu Leu
   Arg
   50 55 60
   Leu Tyr Leu Asp Arg Val Phe Lys Asn Tyr Gln Thr Pro Asp His
   Tyr
   65 70 75
   80
   Thr Leu Arg Lys Ile Ser Ser Leu Ala Asn Ser Phe Leu Thr Ile
   Lys
   85 90 95
   Lys Asp Leu Arg Leu Cys His Ala His Met Thr Cys His Cys Gly
   Glu
   100 105 110
   Glu Ala Met Lys Lys Tyr Ser Gln Ile Leu Ser His Phe Glu Lys
   Leu
   115 120 125
   Glu Pro Gln Ala Ala Val Val Lys Ala Leu Gly Glu Leu Asp Ile
   Leu
   130 135 140
   Leu Gln Trp Met Glu Glu Thr Glu
   145 150
<210> 3
   <211> 151
   <212> PRT
   <213> Homo sapiens
<400> 3
   Met Lys Ala Ser Ser Leu Ala Phe Ser Leu Leu Ser Ala Ala Phe
   Tyr
   1 5 10 15
   Leu Leu Trp Thr Pro Ser Thr Gly Leu Lys Thr Leu Asn Leu Gly
   Ser
   20 25 30
   Cys Val Ile Ala Thr Asn Leu Gln Glu Ile Arg Asn Gly Phe Ser Asp
   35 40 45
   Ile Arg Gly Ser Val Gln Ala Lys Asp Gly Asn Ile Asp Ile Arg Ile
   50 55 60
   Leu Arg Arg Thr Glu Ser Leu Gln Asp Thr Lys Pro Ala Asn Arg Cys
   65 70 75 80
   Cys Leu Leu Arg His Leu Leu Arg Leu Tyr Leu Asp Arg Val Phe Lys
   85 90 95
   Asn Tyr Gln Thr Pro Asp His Tyr Thr Leu Arg Lys Ile Ser Ser Leu
   100 105 110
   Ala Asn Ser Phe Leu Thr Ile Lys Lys Asp Leu Arg Leu Cys Leu Glu
   115 120 125
   Pro Gln Ala Ala Val Val Lys Ala Leu Gly Glu Leu Asp Ile Leu Leu
   130 135 140
   Gln Trp Met Glu Glu Thr Glu
   145 150
<210> 4
   <211> 127
   <212> PRT
   <213> Homo sapiens
<400> 4
   Leu Lys Thr Leu Asn Leu Gly Ser Cys Val Ile Ala Thr Asn Leu Gln
   1 5 10 15
   Glu Ile Arg Asn Gly Phe Ser Asp Ile Arg Gly Ser Val Gln Ala Lys
   20 25 30
   Asp Gly Asn Ile Asp Ile Arg Ile Leu Arg Arg Thr Glu Ser Leu Gln
   35 40 45
   Asp Thr Lys Pro Ala Asn Arg Cys Cys Leu Leu Arg His Leu Leu Arg
   50 55 60
   Leu Tyr Leu Asp Arg Val Phe Lys Asn Tyr Gln Thr Pro Asp His Tyr
   65 70 75 80
   Thr Leu Arg Lys Ile Ser Ser Leu Ala Asn Ser Phe Leu Thr Ile Lys
   85 90 95
   Lys Asp Leu Arg Leu Cys Leu Glu Pro Gln Ala Ala Val Val Lys Ala
   100 105 110
   Leu Gly Glu Leu Asp Ile Leu Leu Gln Trp Met Glu Glu Thr Glu
   115 120 125
<210> 5
   <211> 176
   <212> PRT
   <213> Mus musculis
<400> 5
   Met Lys Gly Phe Gly Leu Ala Phe Gly Leu Phe Ser Ala Val Gly Phe
   1 5 10 15
   Leu Leu Trp Thr Pro Leu Thr Gly Leu Lys Thr Leu His Leu Gly Ser
   20 25 30
   Cys Val Ile Thr Ala Asn Leu Gln Ala Ile Gln Lys Glu Phe Ser Glu
   35 40 45
   Ile Arg Asp Ser Val Gln Ala Glu Asp Thr Asn Ile Asp Ile Arg Ile
   50 55 60
   Leu Arg Thr Thr Glu Ser Leu Lys Asp Ile Lys Ser Leu Asp Arg Cys
   65 70 75 80
   Cys Phe Leu Arg His Leu Val Arg Phe Tyr Leu Asp Arg Val Phe Lys
   85 90 95
   Val Tyr Gln Thr Pro Asp His His Thr Leu Arg Lys Ile Ser Ser Leu
   100 105 110
   Ala Asn Ser Phe Leu Ile Ile Lys Lys Asp Leu Ser Val Cys His Ser
   115 120 125
   His Met Ala Cys His Cys Gly Glu Glu Ala Met Glu Lys Tyr Asn Gln
   130 135 140
   Ile Leu Ser His Phe Ile Glu Leu Glu Leu Gln Ala Ala Val Val Lys
   145 150 155 160
   Ala Leu Gly Glu Leu Gly Ile Leu Leu Arg Trp Met Glu Glu Met Leu
   165 170 175
<210> 6
   <211> 152
   <212> PRT
   <213> Mus musculis
<400> 6
   Leu Lys Thr Leu His Leu Gly Ser Cys Val Ile Thr Ala Asn Leu Gln
   1 5 10 15
   Ala Ile Gln Lys Glu Phe Ser Glu Ile Arg Asp Ser Val Gln Ala Glu
                     20                      25                      30
   Asp Thr Asn Ile Asp Ile Arg Ile Leu Arg Thr Thr Glu Ser Leu
   Lys
               35                      40                      45
   Asp Ile Lys Ser Leu Asp Arg Cys Cys Phe Leu Arg His Leu Val
   Arg
           50                      55                      60
   Phe Tyr Leu Asp Arg Val Phe Lys Val Tyr Gln Thr Pro Asp His
   His
   65                      70                      75
   80
   Thr Leu Arg Lys Ile Ser Ser Leu Ala Asn Ser Phe Leu Ile Ile
   Lys
                   85                      90                      95
   Lys Asp Leu Ser Val Cys His Ser His Met Ala Cys His Cys Gly
   Glu
               100                     105                     110
   Glu Ala Met Glu Lys Tyr Asn Gln Ile Leu Ser His Phe Ile Glu
   Leu
           115                     120                     125
   Glu Leu Gln Ala Ala Val Val Lys Ala Leu Gly Glu Leu Gly Ile
   Leu
       130                     135                     140
   Leu Arg Trp Met Glu Glu Met Leu
   145                 150
<210> 7
   <211> 144
   <212> PRT
   <213> Mus musculis
<400> 7
   Cys Val Ile Thr Ala Asn Leu Gln Ala Ile Gln Lys Glu Phe Ser
   Glu
   1               5                   10                  15
   Ile Arg Asp Ser Val Gln Ala Glu Asp Thr Asn Ile Asp Ile Arg
   Ile
               20                  25                  30
   Leu Arg Thr Thr Glu Ser Leu Lys Asp Ile Lys Ser Leu Asp Arg
   Cys
           35                  40                  45
   Cys Phe Leu Arg His Leu Val Arg Phe Tyr Leu Asp Arg Val Phe
   Lys
       50                  55                  60
   Val Tyr Gln Thr Pro Asp His His Thr Leu Arg Lys Ile Ser Ser
   Leu
   65                  70                  75
   80
   Ala Asn Ser Phe Leu Ile Ile Lys Lys Asp Leu Ser Val Cys His
   Ser
                   85                  90                  95
   His Met Ala Cys His Cys Gly Glu Glu Ala Met Glu Lys Tyr Asn
   Gln
               100                 105                 110
   Ile Leu Ser His Phe Ile Glu Leu Glu Leu Gln Ala Ala Val Val Lys
   115 120 125
   Ala Leu Gly Glu Leu Gly Ile Leu Leu Arg Trp Met Glu Glu Met Leu
   130 135 140
<210> 8
   <211> 154
   <212> PRT
   <213> Mus musculis
<400> 8
   Met Lys Gly Phe Gly Leu Ala Phe Gly Leu Phe Ser Ala Val Gly Phe
   1 5 10 15
   Leu Leu Trp Thr Pro Leu Thr Gly Leu Lys Thr Leu His Leu Gly Ser
   20 25 30
   Cys Val Ile Thr Ala Asn Leu Gln Ala Ile Gln Lys Glu Phe Ser Glu
   35 40 45
   Ile Arg Asp Ser Val Ser Leu Asp Arg Cys Cys Phe Leu Arg His Leu
   50 55 60
   Val Arg Phe Tyr Leu Asp Arg Val Phe Lys Val Tyr Gln Thr Pro Asp
   65 70 75 80
   His His Thr Leu Arg Lys Ile Ser Ser Leu Ala Asn Ser Phe Leu Ile
   85 90 95
   Ile Lys Lys Asp Leu Ser Val Cys His Ser His Met Ala Cys His Cys
   100 105 110
   Gly Glu Glu Ala Met Glu Lys Tyr Asn Gln Ile Leu Ser His Phe Ile
   115 120 125
   Glu Leu Glu Leu Gln Ala Ala Val Val Lys Ala Leu Gly Glu Leu Gly
   130 135 140
   Ile Leu Leu Arg Trp Met Glu Glu Met Leu
   145 150
<210> 9
   <211> 130
   <212> PRT
   <213> Homo sapiens
<400> 9
   Leu Lys Thr Leu His Leu Gly Ser Cys Val Ile Thr Ala Asn Leu
   Gln
    1               5                   10                  15
   Ala Ile Gln Lys Glu Phe Ser Glu Ile Arg Asp Ser Val Ser Leu
   Asp
               20                  25                  30
   Arg Cys Cys Phe Leu Arg His Leu Val Arg Phe Tyr Leu Asp Arg
   Val
           35                  40                  45
   Phe Lys Val Tyr Gln Thr Pro Asp His His Thr Leu Arg Lys Ile
   Ser
       50                  55                  60
   Ser Leu Ala Asn Ser Phe Leu Ile Ile Lys Lys Asp Leu Ser Val
   Cys
   65                  70                  75
   80
   His Ser His Met Ala Cys His Cys Gly Glu Glu Ala Met Glu Lys
   Tyr
                   85                  90                  95
   Asn Gln Ile Leu Ser His Phe Ile Glu Leu Glu Leu Gln Ala Ala
   Val
               100                 105                 110
   Val Lys Ala Leu Gly Glu Leu Gly Ile Leu Leu Arg Trp Met Glu
   Glu
           115                 120                 125
   Met Leu
       130
<210> 10
   <211> 3516
   <212> DNA
   <213> Homo sapiens
<220>
   <221> CDS
   <222> (237)...(1895)
<400> 10
<210> 11
   <211> 553
   <212> PRT
   <213> Homo sapiens
<400> 11
<210> 12
   <211> 221
   <212> PRT
   <213> Homo sapiens
<400> 12
   Val Pro Cys Val Ser Gly Gly Leu Pro Lys Pro Ala Asn Ile Thr
   Phe
    1               5                   10                  15
   Leu Ser Ile Asn Met Lys Asn Val Leu Gln Trp Thr Pro Pro Glu
   Gly
                    20                  25                  30
   Leu Gln Gly Val Lys Val Thr Tyr Thr Val Gln Tyr Phe Ile Tyr
   Gly
                35                  40                  45
   Gln Lys Lys Trp Leu Asn Lys Ser Glu Cys Arg Asn Ile Asn Arg
   Thr
        50                  55                  60
   Tyr Cys Asp Leu Ser Ala Glu Thr Ser Asp Tyr Glu His Gln Tyr
   Tyr
    65                  70                  75
   80
   Ala Lys Val Lys Ala Ile Trp Gly Thr Lys Cys Ser Lys Trp Ala
   Glu
                    85                  90                  95
   Ser Gly Arg Phe Tyr Pro Phe Leu Glu Thr Gln Ile Gly Pro Pro
   Glu
                100                 105                 110
   Val Ala Leu Thr Thr Asp Glu Lys Ser Ile Ser Val Val Leu Thr
   Ala
            115                 120                 125
   Pro Glu Lys Trp Lys Arg Asn Pro Glu Asp Leu Pro Val Ser Met
   Gln
        130                 135                 140
   Gln Ile Tyr Ser Asn Leu Lys Tyr Asn Val Ser Val Leu Asn Thr
   Lys
    145                 150                 155
   160
   Ser Asn Arg Thr Trp Ser Gln Cys Val Thr Asn His Thr Leu Val
   Leu
                    165                 170                 175
   Thr Trp Leu Glu Pro Asn Thr Leu Tyr Cys Val His Val Glu Ser
   Phe
                180                 185                 190
   Val Pro Gly Pro Pro Arg Arg Ala Gln Pro Ser Glu Lys Gln Cys
   Ala
            195                 200                 205
   Arg Thr Leu Lys Asp Gln Ser Ser Glu Phe Lys Ala Lys
       210                 215                 220
<210> 13
   <211> 971
   <212> DNA
   <213> Homo sapiens
<220>
   <221> CDS
   <222> (18)...(950)
<400> 13
<210> 14
   <211> 311
   <212> PRT
   <213> Homo sapiens
<400> 14
   Met Gln Thr Phe Thr Met Val Leu Glu Glu Ile Trp Thr Ser Leu Phe
   1 5 10 15
   Met Trp Phe Phe Tyr Ala Leu Ile Pro Cys Leu Leu Thr Asp Glu Val
   20 25 30
   Ala Ile Leu Pro Ala Pro Gln Asn Leu Ser Val Leu Ser Thr Asn Met
   35 40 45
   Lys His Leu Leu Met Trp Ser Pro Val Ile Ala Pro Gly Glu Thr Val
   50 55 60
   Tyr Tyr Ser Val Glu Tyr Gln Gly Glu Tyr Glu Ser Leu Tyr Thr Ser
   65 70 75 80
   His Ile Trp Ile Pro Ser Ser Trp Cys Ser Leu Thr Glu Gly Pro Glu
   85 90 95
   Cys Asp Val Thr Asp Asp Ile Thr Ala Thr Val Pro Tyr Asn Leu Arg
   100 105 110
   Val Arg Ala Thr Leu Gly Ser Gln Thr Ser Ala Trp Ser Ile Leu Lys
   115 120 125
   His Pro Phe Asn Arg Asn Ser Thr Ile Leu Thr Arg Pro Gly Met Glu
   130 135 140
   Ile Thr Lys Asp Gly Phe His Leu Val Ile Glu Leu Glu Asp Leu Gly
   145 150 155 160
   Pro Gln Phe Glu Phe Leu Val Ala Tyr Trp Arg Arg Glu Pro Gly Ala
   165 170 175
   Glu Glu His Val Lys Met Val Arg Ser Gly Gly Ile Pro Val His Leu
   180 185 190
   Glu Thr Met Glu Pro Gly Ala Ala Tyr Cys Val Lys Ala Gln Thr Phe
   195 200 205
   Val Lys Ala Ile Gly Arg Tyr Ser Ala Phe Ser Gln Thr Glu Cys Val
   210 215 220
   Glu Val Gln Gly Glu Ala Ile Pro Leu Val Leu Ala Leu Phe Ala Phe
   225 230 235 240
   Val Gly Phe Met Leu Ile Leu Val Val Val Pro Leu Phe Val Trp Lys
   245 250 255
   Met Gly Arg Leu Leu Gln Tyr Ser Cys Cys Pro Val Val Val Leu Pro
   260 265 270
   Asp Thr Leu Lys Ile Thr Asn Ser Pro Gln Lys Leu Ile Ser Cys Arg
   275 280 285
   Arg Glu Glu Val Asp Ala Cys Ala Thr Ala Val Met Ser Pro Glu Glu
   290 295 300
   Leu Leu Arg Ala Trp Ile Ser
   305 310
<210> 15
   <211> 203
   <212> PRT
   <213> Homo sapiens
<400> 15
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75
   80
   Asn Leu Arg Val Arg Ala Thr Leu Gly Ser Gln Thr Ser Ala Trp
   Ser
   85 90 95
   Ile Leu Lys His Pro Phe Asn Arg Asn Ser Thr Ile Leu Thr Arg
   Pro
   100 105 110
   Gly Met Glu Ile Thr Lys Asp Gly Phe His Leu Val Ile Glu Leu
   Glu
   115 120 125
   Asp Leu Gly Pro Gln Phe Glu Phe Leu Val Ala Tyr Trp Arg Arg
   Glu
   130 135 140
   Pro Gly Ala Glu Glu His Val Lys Met Val Arg Ser Gly Gly Ile
   Pro
   145 150 155
   160
   Val His Leu Glu Thr Met Glu Pro Gly Ala Ala Tyr Cys Val Lys
   Ala
   165 170 175
   Gln Thr Phe Val Lys Ala Ile Gly Arg Tyr Ser Ala Phe Ser Gln
   Thr
   180 185 190
   Glu Cys Val Glu Val Gln Gly Glu Ala Ile Pro
   195 200
<210> 16
   <211> 33
   <212> DNA
   <213> Homo sapiens
<400> 16
   gcgaattcga gtctaccaaa tgcagacttt cac
   33
<210> 17
   <211> 32
   <212> DNA
   <213> Homo sapiens
<400> 17
   cgctcgagcc ttccgcaaac ctatgagatc ca
   32
<210> 18
   <211> 1379
   <212> DNA
   <213> Homo sapiens
<220>
   <221> CDS
   <222> (132)...(1034)
<400> 18
<210> 19
   <211> 301
   <212> PRT
   <213> Homo sapiens
<400> 19
   Met Gln Thr Phe Thr Met Val Leu Glu Glu Ile Trp Thr Ser Leu Phe
   1 5 10 15
   Met Trp Phe Phe Tyr Ala Leu Ile Pro Cys Leu Leu Thr Asp Glu Val
   20 25 30
   Ala Ile Leu Pro Ala Pro Gln Asn Leu Ser Val Leu Ser Thr Asn Met
   35 40 45
   Lys His Leu Leu Met Trp Ser Pro Val Ile Ala Pro Gly Glu Thr Val
   50 55 60
   Tyr Tyr Ser Val Glu Tyr Gln Gly Glu Tyr Glu Ser Leu Tyr Thr Ser
   65 70 75 80
   His Ile Trp Ile Pro Ser Ser Trp Cys Ser Leu Thr Glu Gly Pro Glu
   85 90 95
   Cys Asp Val Thr Asp Asp Ile Thr Ala Thr Val Pro Tyr Asn Leu Arg
   100 105 110
   Val Arg Ala Thr Leu Gly Ser Gln Thr Ser Ala Trp Ser Ile Leu Lys
   115 120 125
   His Pro Phe Asn Arg Asn Ser Thr Ile Leu Thr Arg Pro Gly Met Glu
   130 135 140
   Ile Pro Lys His Gly Phe His Leu Val Ile Glu Leu Glu Asp Leu Gly
   145 150 155 160
   Pro Gln Phe Glu Phe Leu Val Ala Tyr Trp Thr Arg Glu Pro Gly Ala
   165 170 175
   Glu Glu His Val Lys Met Val Arg Ser Gly Gly Ile Pro Val His Leu
   180 185 190
   Glu Thr Met Glu Pro Gly Ala Ala Tyr Cys Val Lys Ala Gln Thr Phe
   195 200 205
   Val Lys Ala Ile Gly Arg Tyr Ser Ala Phe Ser Gln Thr Glu Cys Val
   210 215 220
   Glu Val Gln Gly Glu Ala Ile Pro Leu Val Leu Ala Leu Phe Ala Phe
   225 230 235 240
   Val Gly Phe Met Leu Ile Leu Val Val Val Pro Leu Phe Val Trp Lys
   245 250 255
   Met Gly Arg Leu Leu Gln Tyr Ser Cys Cys Pro Val Val Val Leu Pro
   260 265 270
   Asp Thr Leu Lys Ile Thr Asn Ser Pro Gln Val Asn Gln Leu Gln Lys
   275 280 285
   Gly Gly Gly Gly Cys Leu Cys His Gly Cys Asp Val Ser
   290 295 300
<210> 20
   <211> 1081
   <212> DNA
   <213> Homo sapiens
<220>
   <221> CDS
   <222> (9)...(1067)
<400> 20
<210> 21
   <211> 352
   <212> PRT
   <213> Homo sapiens
<400> 21
   Met Gln Thr Phe Thr Met Val Leu Glu Glu Ile Trp Thr Ser Leu Phe
   1 5 10 15
   Met Trp Phe Phe Tyr Ala Leu Ile Pro Cys Leu Leu Thr Asp Glu Val
   20 25 30
   Ala Ile Leu Pro Ala Pro Gln Asn Leu Ser Val Leu Ser Thr Asn Met
   35 40 45
   Lys His Leu Leu Met Trp Ser Pro Val Ile Ala Pro Gly Glu Thr Val
   50 55 60
   Tyr Tyr Ser Val Glu Tyr Gln Gly Glu Tyr Glu Ser Leu Tyr Thr Ser
   65 70 75 80
   His Ile Trp Ile Pro Ser Ser Trp Cys Ser Leu Thr Glu Gly Pro Glu
   85 90 95
   Cys Asp Val Thr Asp Asp Ile Thr Ala Thr Val Pro Tyr Asn Leu Arg
   100 105 110
   Val Arg Ala Thr Leu Gly Ser Gln Thr Ser Ala Trp Ser Ile Leu Lys
   115 120 125
   His Pro Phe Asn Arg Asn Ser Thr Ile Leu Thr Arg Pro Gly Met
   Glu
   130 135 140
   Ile Pro Lys His Gly Phe His Leu Val Ile Glu Leu Glu Asp Leu
   Gly
   145 150 155
   160
   Pro Gln Phe Glu Phe Leu Val Ala Tyr Trp Thr Arg Glu Pro Gly
   Ala
   165 170 175
   Glu Glu His Val Lys Met Val Arg Ser Gly Gly Ile Pro Val His
   Leu
   180 185 190
   Glu Thr Met Glu Pro Gly Ala Ala Tyr Cys Val Lys Ala Gln Thr
   Phe
   195 200 205
   Val Lys Ala Ile Gly Arg Tyr Ser Ala Phe Ser Gln Thr Glu Cys
   Val
   210 215 220
   Glu Val Gln Gly Glu Ala Gly Gly Gly Gly Ser Gly Gly Gly Gly
   Ser
   225 230 235
   240
   Gly Gly Gly Gly Ser Arg Thr Val Ala Ala Pro Ser Val Phe Ile
   Phe
   245 250 255
   Pro Pro Ser Asp Glu Gln Leu Lys Ser Gly Thr Ala Ser Val Val
   Cys
   260 265 270
   Leu Leu Asn Asn Phe Tyr Pro Arg Glu Ala Lys Val Gln Trp Lys
   Val
   275 280 285
   Asp Asn Ala Leu Gln Ser Gly Asn Ser Gln Glu Ser Val Thr Glu
   Gln
   290 295 300
   Asp Ser Lys Asp Ser Thr Tyr Ser Leu Ser Ser Thr Leu Thr Leu
   Ser
   305 310 315
   320
   Lys Ala Asp Tyr Glu Lys His Lys Val Tyr Ala Cys Glu Val Thr
   His
   325 330 335
   Gln Gly Leu Ser Ser Pro Val Thr Lys Ser Phe Asn Arg Gly Glu
   Cys
   340 345 350
<210> 22
   <211> 1801
   <212> DNA
   <213> Homo sapiens
<220>
   <221> CDS
   <222> (8)...(1789)
<400> 22
<210> 23
   <211> 594
   <212> PRT
   <213> Homo sapiens
<400> 23
<210> 24
   <211> 29
   <212> DNA
   <213> Homo sapiens
<400> 24
   ggccggccat gcagactttc acaatggtt 29
<210> 25
   <211> 52
   <212> DNA
   <213> homo sapiens
<400> 25
   tccgctaccg ccgcctccac tgccaccacc tccggcctct ccttgcacct cc
   52
<210> 26
   <211> 53
   <212> DNA
   <213> Homo sapiens
<400> 26
   gtggaggcgg cggtagcgga ggcggtggca gtcgaactgt ggctgcacca tct
   53
<210> 27
   <211> 38
   <212> DNA
   <213> Homo sapiens
<400> 27
   ggcgcgcctc tagattaaca ctctcccctg ttgaagct
   38
<210> 28
   <211> 30
   <212> DNA
   <213> Homo sapiens
<400> 28
   gtcgaccatg gatgcaatga agagagggct
   30
<210> 29
   <211> 30
   <212> DNA
   <213> Homo sapiens
<400> 29
   cacagggaac tctacggaag cgtctcaact
   30
<210> 30
   <211> 33
   <212> DNA
   <213> Homo sapiens
<400> 30
   cttccgtaga gttccctgtg tctctggtgg ttt
   33
<210> 31
   <211> 53
   <212> DNA
   <213> Homo sapiens
<400> 31
   gccagagcca cctccgcctg aaccgcctcc accttgatct ttcaaagtcc tgg
   53
<210> 32
   <211> 51
   <212> DNA
   <213> Homo sapiens
<400> 32
   caggcggagg tggctctggc ggtggcggat cggcctccac caagggccca t
   51
<210> 33
   <211> 20'
   <212> DNA
   <213> Homo sapiens
<400> 33
   ctgggcacgg tgggcatgtg
   20
<210> 34
   <211> 20
   <212> DNA
   <213> Homo sapiens
<400> 34
   cacatgccca ccgtgcccag
   20
<210> 35
   <211> 31
   <212> DNA
   <213> Homo sapiens
<400> 35
   agatctagat tatttacccg gagacaggga g
   31
<210> 36
   <211> 1806
   <212> DNA
   <213> Mus musculus
<220>
   <221> CDS
   <222> (38)...(1675)
<400> 36
<210> 37
   <211> 546
   <212> PRT
   <213> Mus musculus
<400> 37
   Met His Thr Pro Gly Thr Pro Ala Pro Gly His Pro Asp Pro Pro Pro
   1 5 10 15
   Leu Leu Leu Leu Thr Leu Leu Leu Leu Leu Ala Ala Ser Gly Arg Ala
   20 25 30
   Val Pro Cys Val Phe Cys Gly Leu Pro Lys Pro Thr Asn Ile Thr Phe
   35 40 45
   Leu Ser Ile Asn Met Lys Asn Val Leu His Trp Asn Pro Pro Glu Ser
   50 55 60
   Leu His Gly Val Glu Val Thr Tyr Thr Val Gln Tyr Phe Ile Tyr Gly
   65 70 75 80
   Gln Lys Lys Trp Leu Asn Ala Ser Lys Cys Gly Ser Ile Asn Arg Thr
   85 90 95
   Tyr Cys Asp Leu Ser Val Glu Thr Ser Asp Tyr Glu His Gln Phe Tyr
   100 105 110
   Ala Lys Val Lys Ala Ile Trp Glu Ala Arg Cys Ser Glu Trp Ala Glu
   115 120 125
   Thr Glu Arg Phe Tyr Pro Phe Leu Glu Thr Gln Val Ser Pro Pro Glu
   130 135 140
   Ile Ala Leu Thr Thr Gly Glu Lys Ser Ile Ser Ile Ala Leu Thr Ala
   145 150 155 160
   Pro Glu Lys Trp Lys Arg Asn Pro Gln Asp His Thr Val Ser Met Gln
   165 170 175
   Gln Ile Tyr Pro Asn Leu Lys Tyr Asn Val Ser Val Tyr Asn Thr Lys
   180 185 190
   Ser Arg Arg Thr Trp Ser Gln Cys Val Thr Asn Ser Thr Leu Val Leu
   195 200 205
   Ser Trp Leu Glu Pro Asn Thr Leu Tyr Cys Val His Val Glu Ser Leu
   210 215 220
   Val Pro Gly Pro Pro Arg Leu Pro Met Pro Ser Gln Lys Gln Cys Ile
   225 230 235 240
   Ser Thr Leu Glu Val Gln Thr Ser Ala Trp Lys Ala Lys Val Ile
   Phe
   245 250 255
   Trp Tyr Val Phe Leu Thr Ser Val Ile Val Phe Leu Phe Ser Ala
   Ile
   260 265 270
   Gly Tyr Leu Val Tyr Arg Tyr Ile His Val Gly Lys Glu Lys His
   Pro
   275 280 285
   Ala Asn Leu Val Leu Ile Tyr Arg Asn Glu Ile Gly Thr Arg Val
   Phe
   290 295 300
   Glu Pro Thr Glu Thr Ile Thr Leu Asn Phe Ile Thr Phe Ser Met
   Leu
   305 310 315
   320
   Asp Asp Thr Lys Ile Ser Pro Lys Asp Met Asn Leu Leu Asp Lys
   Ser
   325 330 335
   Ser Asp Asp Ile Ser Val Asn Asp Pro Glu His Asn Glu Ala Trp
   Glu
   340 345 350
   Pro His Trp Glu Glu Val Glu Gly Gln His Leu Gly Cys Ser Ser
   His
   355 360 365
   Leu Met Asp Ala Val Cys Gly Ala Glu Gln Arg Asp Gly Asp Thr
   Ser
   370 375 380
   Leu Thr Gln His Gly Trp Leu Asn Ser Thr Ile Pro Thr Gly Glu
   Thr
   385 390 395
   400
   Asp Thr Glu Pro Gln Tyr Lys Val Leu Ser Asp Phe Tyr Gly Glu
   Gly
   405 410 415
   Glu Ile Gln Leu Ser Cys Glu Pro Glu Glu Ala Ala Arg Thr Glu
   Lys
   420 425 430
   Ile Ser Glu Pro Leu Val Thr Ser Ala Asn Leu Asp Pro Gln Leu
   Glu
   435 440 445
   Asp Leu His His Leu Gly Gln Glu His Thr Val Ser Glu Asp Gly
   Pro
   450 455 460
   Glu Glu Glu Thr Ser Ile Thr Val Val Asp Trp Asp Pro Gln Thr
   Gly
   465 470 475
   480
   Arg Leu Cys Ile Pro Ser Leu Pro Ile Phe Gly Arg Asp Pro Glu
   Asn
   485 490 495
   Tyr Gly His Tyr Glu Arg Asp Gln Leu Leu Glu Gly Gly Leu Leu
   Ser
   500 505 510
   Arg Leu Tyr Glu Asn Gln Ala Pro Asp Lys Pro Glu Lys Glu Asn Glu
   515 520 525
   Asn Cys Leu Thr Arg Phe Met Glu Glu Trp Gly Leu His Val Gln Met
   530 535 540
   Glu Ser
   545
<210> 38
   <211> 217
   <212> PRT
   <213> Mus musculus
<400> 38
   Val Pro Cys Val Phe Cys Gly Leu Pro Lys Pro Thr Asn Ile Thr Phe
   1 5 10 15
   Leu Ser Ile Asn Met Lys Asn Val Leu His Trp Asn Pro Pro Glu Ser
   20 25 30
   Leu His Gly Val Glu Val Thr Tyr Thr Val Gln Tyr Phe Ile Tyr Gly
   35 40 45
   Gln Lys Lys Trp Leu Asn Ala Ser Lys Cys Gly Ser Ile Asn Arg Thr
   50 55 60
   Tyr Cys Asp Leu Ser Val Glu Thr Ser Asp Tyr Glu His Gln Phe Tyr
   65 70 75 80
   Ala Lys Val Lys Ala Ile Trp Glu Ala Arg Cys Ser Glu Trp Ala Glu
   85 90 95
   Thr Glu Arg Phe Tyr Pro Phe Leu Glu Thr Gln Val Ser Pro Pro Glu
   100 105 110
   Ile Ala Leu Thr Thr Gly Glu Lys Ser Ile Ser Ile Ala Leu Thr Ala
   115 120 125
   Pro Glu Lys Trp Lys Arg Asn Pro Gln Asp His Thr Val Ser Met Gln
   130 135 140
   Gln Ile Tyr Pro Asn Leu Lys Tyr Asn Val Ser Val Tyr Asn Thr Lys
   145 150 155 160
   Ser Arg Arg Thr Trp Ser Gln Cys Val Thr Asn Ser Thr Leu Val Leu
   165 170 175
   Ser Trp Leu Glu Pro Asn Thr Leu Tyr Cys Val His Val Glu Ser Leu
   180 185 190
   Val Pro Gly Pro Pro Arg Leu Pro Met Pro Ser Gln Lys Gln Cys Ile
   195 200 205
   Ser Thr Leu Glu Val Gln Thr Ser Ala
   210 215
<210> 39
   <211> 514
   <212> PRT
   <213> Mus musculus
<400> 39
   Val Pro Cys Val Phe Cys Gly Leu Pro Lys Pro Thr Asn Ile Thr Phe
   1 5 10 15
   Leu Ser Ile Asn Met Lys Asn Val Leu His Trp Asn Pro Pro Glu Ser
   20 25 30
   Leu His Gly Val Glu Val Thr Tyr Thr Val Gln Tyr Phe Ile Tyr Gly
   35 40 45
   Gln Lys Lys Trp Leu Asn Ala Ser Lys Cys Gly Ser Ile Asn Arg Thr
   50 55 60
   Tyr Cys Asp Leu Ser Val Glu Thr Ser Asp Tyr Glu His Gln Phe Tyr
   65 70 75 80
   Ala Lys Val Lys Ala Ile Trp Glu Ala Arg Cys Ser Glu Trp Ala Glu
   85 90 95
   Thr Glu Arg Phe Tyr Pro Phe Leu Glu Thr Gln Val Ser Pro Pro Glu
   100 105 110
   Ile Ala Leu Thr Thr Gly Glu Lys Ser Ile Ser Ile Ala Leu Thr Ala
   115 120 125
   Pro Glu Lys Trp Lys Arg Asn Pro Gln Asp His Thr Val Ser Met Gln
   130 135 140
   Gln Ile Tyr Pro Asn Leu Lys Tyr Asn Val Ser Val Tyr Asn Thr Lys
   145 150 155 160
   Ser Arg Arg Thr Trp Ser Gln Cys Val Thr Asn Ser Thr Leu Val Leu
   165 170 175
   Ser Trp Leu Glu Pro Asn Thr Leu Tyr Cys Val His Val Glu Ser Leu
   180 185 190
   Val Pro Gly Pro Pro Arg Leu Pro Met Pro Ser Gln Lys Gln Cys Ile
   195 200 205
   Ser Thr Leu Glu Val Gln Thr Ser Ala Trp Lys Ala Lys Val Ile Phe
   210 215 220
   Trp Tyr Val Phe Leu Thr Ser Val Ile Val Phe Leu Phe Ser Ala
   Ile
   225 230 235
   240
   Gly Tyr Leu Val Tyr Arg Tyr Ile His Val Gly Lys Glu Lys His
   Pro
   245 250 255
   Ala Asn Leu Val Leu Ile Tyr Arg Asn Glu Ile Gly Thr Arg Val
   Phe
   260 265 270
   Glu Pro Thr Glu Thr Ile Thr Leu Asn Phe Ile Thr Phe Ser Met
   Leu
   275 280 285
   Asp Asp Thr Lys Ile Ser Pro Lys Asp Met Asn Leu Leu Asp Lys
   Ser
   290 295 300
   Ser Asp Asp Ile Ser Val Asn Asp Pro Glu His Asn Glu Ala Trp
   Glu
   305 310 315
   320
   Pro His Trp Glu Glu Val Glu Gly Gln His Leu Gly Cys Ser Ser
   His
   325 330 335
   Leu Met Asp Ala Val Cys Gly Ala Glu Gln Arg Asp Gly Asp Thr
   Ser
   340 345 350
   Leu Thr Gln His Gly Trp Leu Asn Ser Thr Ile Pro Thr Gly Glu
   Thr
   355 360 365
   Asp Thr Glu Pro Gln Tyr Lys Val Leu Ser Asp Phe Tyr Gly Glu
   Gly
   370 375 380
   Glu Ile Gln Leu Ser Cys Glu Pro Glu Glu Ala Ala Arg Thr Glu
   Lys
   385 390 395
   400
   Ile Ser Glu Pro Leu Val Thr Ser Ala Asn Leu Asp Pro Gln Leu
   Glu
   405 410 415
   Asp Leu His His Leu Gly Gln Glu His Thr Val Ser Glu Asp Gly
   Pro
   420 425 430
   Glu Glu Glu Thr Ser Ile Thr Val Val Asp Trp Asp Pro Gln Thr
   Gly
   435 440 445
   Arg Leu Cys Ile Pro Ser Leu Pro Ile Phe Gly Arg Asp Pro Glu
   Asn
   450 455 460
   Tyr Gly His Tyr Glu Arg Asp Gln Leu Leu Glu Gly Gly Leu Leu
   Ser
   465 470 475
   480
   Arg Leu Tyr Glu Asn Gln Ala Pro Asp Lys Pro Glu Lys Glu Asn
   Glu
   485 490 495
   Asn Cys Leu Thr Arg Phe Met Glu Glu Trp Gly Leu His Val Gln
   Met
   500 505 510
   Glu Ser
<210> 40
   <211> 18
   <212> DNA
   <213> Mus musculus
<400> 40
   cgccgcgttc ccgagatg
   18
<210> 41
   <211> 24
   <212> DNA
   <213> Mus musculus
<400> 41
   ggatgaggca gggctgacaa agtt
   24
<210> 42
   <211> 36
   <212> DNA
   <213> Homo sapiens
<400> 42
   acttgtggaa ttcgctagca ccaagggccc atcggt
   36
<210> 43
   <211> 32
   <212> DNA
   <213> Homo sapiens
<400> 43
   gcctagaacg cgttcattta cccggagaca gg
   32
<210> 44
   <211> 8
   <212> DNA
   <213> Homo sapiens
<400> 44
   aattgaga
   8
<210> 45
   <211> 8
   <212> DNA
   <213> Homo sapiens
<400> 45
   cgcgtctc
   8
<210> 46
   <211> 37
   <212> DNA
   <213> Homo sapiens
<400> 46
   gtcacttgaa ttcggtaccg cctctgttgt gtgcctg
   37
<210> 47
   <211> 32
   <212> DNA
   <213> Homo sapiens
<400> 47
   gacctgaacg cgtctaacac tctcccctgt tg
   32
<210> 48
   <211> 38
   <212> DNA
   <213> Homo sapiens
<400> 48
   tcagtcggaa ttcgcagaag ccatgcgggc tcccggcc
   38
<210> 49
   <211> 35
   <212> DNA
   <213> Homo sapiens
<400> 49
   ctgtgacgct agcctctgat gattgatctt tcaaa
   35
<210> 50
   <211> 43
   <212> DNA
   <213> Homo sapiens
<400> 50 <210> 51
   <211> 86
   <212> DNA
   <213> Homo sapiens
<400> 51
<210> 52
   <211> 1720
   <212> DNA
   <213> Homo sapiens
<220>
   <221> CDS
   <222> (1)...(1713)
<400> 52
<210> 53
   <211> 571
   <212> PRT
   <213> Homo sapiens
<400> 53
   Met Arg Ala Pro Gly Arg Pro Ala Leu Arg Pro Leu Leu Leu Leu
   Leu
    1              5                   10                  15
   Leu Ala Ala Pro Trp Gly Arg Ala Val Pro Cys Val Ser Gly Gly
   Leu
               20                  25                  30
   Pro Lys Pro Ala Asn Ile Thr Phe Leu Ser Ile Asn Met Lys Asn
   Val
           35                  40                  45
   Leu Gln Trp Thr Pro Pro Glu Gly Leu Gln Gly Val Lys Val Thr
   Tyr
       50                  55                  60
   Thr Val Gln Tyr Phe Ile Tyr Gly Gln Lys Lys Trp Leu Asn Lys
   Ser
   65                  70                  75
   80
   Glu Cys Arg Asn Ile Asn Arg Thr Tyr Cys Asp Leu Ser Ala Glu
   Thr
                   85                  90                  95
   Ser Asp Tyr Glu His Gln Tyr Tyr Ala Lys Val Lys Ala Ile Trp
   Gly
               100                 105                 110
   Thr Lys Cys Ser Lys Trp Ala Glu Ser Gly Arg Phe Tyr Pro Phe
   Leu
           115                 120                 125
   Glu Thr Gln Ile Gly Pro Pro Glu Val Ala Leu Thr Thr Asp Glu
   Lys
       130                 135                 140
   Ser Ile Ser Val Val Leu Thr Ala Pro Glu Lys Trp Lys Arg Asn Pro
   145 150 155 160
   Glu Asp Leu Pro Val Ser Met Gln Gln Ile Tyr Ser Asn Leu Lys Tyr
   165 170 175
   Asn Val Ser Val Leu Asn Thr Lys Ser Asn Arg Thr Trp Ser Gln Cys
   180 185 190
   Val Thr Asn His Thr Leu Val Leu Thr Trp Leu Glu Pro Asn Thr Leu
   195 200 205
   Tyr Cys Val His Val Glu Ser Phe Val Pro Gly Pro Pro Arg Arg Ala
   210 215 220
   Gln Pro Ser Glu Lys Gln Cys Ala Arg Thr Leu Lys Asp Gln Ser Ser
   225 230 235 240
   Glu Ala Ser Thr Lys Gly Pro Ser Val Phe Pro Leu Ala Pro Ser Ser
   245 250 255
   Lys Ser Thr Ser Gly Gly Thr Ala Ala Leu Gly Cys Leu Val Lys Asp
   260 265 270
   Tyr Phe Pro Glu Pro Val Thr Val Ser Trp Asn Ser Gly Ala Leu Thr
   275 280 285
   Ser Gly Val His Thr Phe Pro Ala Val Leu Gln Ser Ser Gly Leu Tyr
   290 295 300
   Ser Leu Ser Ser Val Val Thr Val Pro Ser Ser Ser Leu Gly Thr Gln
   305 310 315 320
   Thr Tyr Ile Cys Asn Val Asn His Lys Pro Ser Asn Thr Lys Val Asp
   325 330 335
   Lys Lys Val Glu Pro Lys Ser Cys Asp Lys Thr His Thr Cys Pro Pro
   340 345 350
   Cys Pro Ala Pro Glu Leu Leu Gly Gly Pro Ser Val Phe Leu Phe Pro
   355 360 365
   Pro Lys Pro Lys Asp Thr Leu Met Ile Ser Arg Thr Pro Glu Val Thr
   370 375 380
   Cys Val Val Val Asp Val Ser His Glu Asp Pro Glu Val Lys Phe Asn
   385 390 395 400
   Trp Tyr Val Asp Gly Val Glu Val His Asn Ala Lys Thr Lys Pro Arg
   405 410 415
   Glu Glu Gln Tyr Asn Ser Thr Tyr Arg Val Val Ser Val Leu Thr Val
   420 425 430
   Leu His Gln Asp Trp Leu Asn Gly Lys Glu Tyr Lys Cys Lys Val Ser
   435 440 445
   Asn Lys Ala Leu Pro Ala Pro Ile Glu Lys Thr Ile Ser Lys Ala Lys
   450 455 460
   Gly Gln Pro Arg Glu Pro Gln Val Tyr Thr Leu Pro Pro Ser Arg Asp
   465 470 475 480
   Glu Leu Thr Lys Asn Gln Val Ser Leu Thr Cys Leu Val Lys Gly Phe
   485 490 495
   Tyr Pro Ser Asp Ile Ala Val Glu Trp Glu Ser Asn Gly Gln Pro Glu
   500 505 510
   Asn Asn Tyr Lys Thr Thr Pro Pro Val Leu Asp Ser Asp Gly Ser Phe
   515 520 525
   Phe Leu Tyr Ser Lys Leu Thr Val Asp Lys Ser Arg Trp Gln Gln Gly
   530 535 540
   Asn Val Phe Ser Cys Ser Val Met His Glu Ala Leu His Asn His Tyr
   545 550 555 560
   Thr Gln Lys Ser Leu Ser Leu Ser Pro Gly Lys
   565 570
<210> 54
   <211> 547
   <212> PRT
   <213> Homo sapiens
<400> 54
   Val Pro Cys Val Ser Gly Gly Leu Pro Lys Pro Ala Asn Ile Thr Phe
   1 5 10 15
   Leu Ser Ile Asn Met Lys Asn Val Leu Gln Trp Thr Pro Pro Glu Gly
   20 25 30
   Leu Gln Gly Val Lys Val Thr Tyr Thr Val Gln Tyr Phe Ile Tyr Gly
   35 40 45
   Gln Lys Lys Trp Leu Asn Lys Ser Glu Cys Arg Asn Ile Asn Arg Thr
   50 55 60
   Tyr Cys Asp Leu Ser Ala Glu Thr Ser Asp Tyr Glu His Gln Tyr Tyr
   65 70 75 80
   Ala Lys Val Lys Ala Ile Trp Gly Thr Lys Cys Ser Lys Trp Ala Glu
   85 90 95
   Ser Gly Arg Phe Tyr Pro Phe Leu Glu Thr Gln Ile Gly Pro Pro Glu
   100 105 110
   Val Ala Leu Thr Thr Asp Glu Lys Ser Ile Ser Val Val Leu Thr Ala
   115 120 125
   Pro Glu Lys Trp Lys Arg Asn Pro Glu Asp Leu Pro Val Ser Met Gln
   130 135 140
   Gln Ile Tyr Ser Asn Leu Lys Tyr Asn Val Ser Val Leu Asn Thr Lys
   145 150 155 160
   Ser Asn Arg Thr Trp Ser Gln Cys Val Thr Asn His Thr Leu Val Leu
   165 170 175
   Thr Trp Leu Glu Pro Asn Thr Leu Tyr Cys Val His Val Glu Ser Phe
   180 185 190
   Val Pro Gly Pro Pro Arg Arg Ala Gln Pro Ser Glu Lys Gln Cys Ala
   195 200 205
   Arg Thr Leu Lys Asp Gln Ser Ser Glu Ala Ser Thr Lys Gly Pro Ser
   210 215 220
   Val Phe Pro Leu Ala Pro Ser Ser Lys Ser Thr Ser Gly Gly Thr Ala
   225 230 235 240
   Ala Leu Gly Cys Leu Val Lys Asp Tyr Phe Pro Glu Pro Val Thr Val
   245 250 255
   Ser Trp Asn Ser Gly Ala Leu Thr Ser Gly Val His Thr Phe Pro Ala
   260 265 270
   Val Leu Gln Ser Ser Gly Leu Tyr Ser Leu Ser Ser Val Val Thr Val
   275 280 285
   Pro Ser Ser Ser Leu Gly Thr Gln Thr Tyr Ile Cys Asn Val Asn His
   290 295 300
   Lys Pro Ser Asn Thr Lys Val Asp Lys Lys Val Glu Pro Lys Ser Cys
   305 310 315 320
   Asp Lys Thr His Thr Cys Pro Pro Cys Pro Ala Pro Glu Leu Leu Gly
   325 330 335
   Gly Pro Ser Val Phe Leu Phe Pro Pro Lys Pro Lys Asp Thr Leu Met
   340 345 350
   Ile Ser Arg Thr Pro Glu Val Thr Cys Val Val Val Asp Val Ser His
   355 360 365
   Glu Asp Pro Glu Val Lys Phe Asn Trp Tyr Val Asp Gly Val Glu Val
   370 375 380
   His Asn Ala Lys Thr Lys Pro Arg Glu Glu Gln Tyr Asn Ser Thr Tyr
   385 390 395 400
   Arg Val Val Ser Val Leu Thr Val Leu His Gln Asp Trp Leu Asn Gly
   405 410 415
   Lys Glu Tyr Lys Cys Lys Val Ser Asn Lys Ala Leu Pro Ala Pro Ile
   420 425 430
   Glu Lys Thr Ile Ser Lys Ala Lys Gly Gln Pro Arg Glu Pro Gln Val
   435 440 445
   Tyr Thr Leu Pro Pro Ser Arg Asp Glu Leu Thr Lys Asn Gln Val Ser
   450 455 460
   Leu Thr Cys Leu Val Lys Gly Phe Tyr Pro Ser Asp Ile Ala Val Glu
   465 470 475 480
   Trp Glu Ser Asn Gly Gln Pro Glu Asn Asn Tyr Lys Thr Thr Pro Pro
   485 490 495
   Val Leu Asp Ser Asp Gly Ser Phe Phe Leu Tyr Ser Lys Leu Thr Val
   500 505 510
   Asp Lys Ser Arg Trp Gln Gln Gly Asn Val Phe Ser Cys Ser Val Met
   515 520 525
   His Glu Ala Leu His Asn His Tyr Thr Gln Lys Ser Leu Ser Leu Ser
   530 535 540
   Pro Gly Lys
   545
<210> 55
   <211> 217
   <212> PRT
   <213> Homo sapiens
<400> 55
   Val Pro Cys Val Ser Gly Gly Leu Pro Lys Pro Ala Asn Ile Thr Phe
   1 5 10 15
   Leu Ser Ile Asn Met Lys Asn Val Leu Gln Trp Thr Pro Pro Glu Gly
   20 25 30
   Leu Gln Gly Val Lys Val Thr Tyr Thr Val Gln Tyr Phe Ile Tyr Gly
   35 40 45
   Gln Lys Lys Trp Leu Asn Lys Ser Glu Cys Arg Asn Ile Asn Arg Thr
   50 55 60
   Tyr Cys Asp Leu Ser Ala Glu Thr Ser Asp Tyr Glu His Gln Tyr Tyr
   65 70 75 80
   Ala Lys Val Lys Ala Ile Trp Gly Thr Lys Cys Ser Lys Trp Ala Glu
   85 90 95
   Ser Gly Arg Phe Tyr Pro Phe Leu Glu Thr Gln Ile Gly Pro Pro Glu
   100 105 110
   Val Ala Leu Thr Thr Asp Glu Lys Ser Ile Ser Val Val Leu Thr Ala
   115 120 125
   Pro Glu Lys Trp Lys Arg Asn Pro Glu Asp Leu Pro Val Ser Met Gln
   130 135 140
   Gln Ile Tyr Ser Asn Leu Lys Tyr Asn Val Ser Val Leu Asn Thr Lys
   145 150 155 160
   Ser Asn Arg Thr Trp Ser Gln Cys Val Thr Asn His Thr Leu Val Leu
   165 170 175
   Thr Trp Leu Glu Pro Asn Thr Leu Tyr Cys Val His Val Glu Ser Phe
   180 185 190
   Val Pro Gly Pro Pro Arg Arg Ala Gln Pro Ser Glu Lys Gln Cys Ala
   195 200 205
   Arg Thr Leu Lys Asp Gln Ser Ser Glu
   210 215
<210> 56
   <211> 1011
   <212> DNA
   <213> Homo sapiens
<220>
   <221> CDS
   <222> (1)...(1008)
<400> 56
<210> 57
   <211> 336
   <212> PRT
   <213> Homo sapiens
<400> 57
   Met Gln Thr Phe Thr Met Val Leu Glu Glu Ile Trp Thr Ser Leu Phe
   1 5 10 15
   Met Trp Phe Phe Tyr Ala Leu Ile Pro Cys Leu Leu Thr Asp Glu Val
   20 25 30
   Ala Ile Leu Pro Ala Pro Gln Asn Leu Ser Val Leu Ser Thr Asn Met
   35 40 45
   Lys His Leu Leu Met Trp Ser Pro Val Ile Ala Pro Gly Glu Thr Val
   50 55 60
   Tyr Tyr Ser Val Glu Tyr Gln Gly Glu Tyr Glu Ser Leu Tyr Thr Ser
   65 70 75 80
   His Ile Trp Ile Pro Ser Ser Trp Cys Ser Leu Thr Glu Gly Pro Glu
   85 90 95
   Cys Asp Val Thr Asp Asp Ile Thr Ala Thr Val Pro Tyr Asn Leu Arg
   100 105 110
   Val Arg Ala Thr Leu Gly Ser Gln Thr Ser Ala Trp Ser Ile Leu Lys
   115 120 125
   His Pro Phe Asn Arg Asn Ser Thr Ile Leu Thr Arg Pro Gly Met Glu
   130 135 140
   Ile Thr Lys Asp Gly Phe His Leu Val Ile Glu Leu Glu Asp Leu Gly
   145 150 155 160
   Pro Gln Phe Glu Phe Leu Val Ala Tyr Trp Arg Arg Glu Pro Gly Ala
   165 170 175
   Glu Glu His Val Lys Met Val Arg Ser Gly Gly Ile Pro Val His Leu
   180 185 190
   Glu Thr Met Glu Pro Gly Ala Ala Tyr Cys Val Lys Ala Gln Thr Phe
   195 200 205
   Val Lys Ala Ile Gly Arg Tyr Ser Ala Phe Ser Gln Thr Glu Cys Val
   210 215 220
   Glu Val Gln Gly Glu Ala Thr Val Ala Ala Pro Ser Val Phe Ile Phe
   225 230 235 240
   Pro Pro Ser Asp Glu Gln Leu Lys Ser Gly Thr Ala Ser Val Val Cys
   245 250 255
   Leu Leu Asn Asn Phe Tyr Pro Arg Glu Ala Lys Val Gln Trp Lys Val
   260 265 270
   Asp Asn Ala Leu Gln Ser Gly Asn Ser Gln Glu Ser Val Thr Glu Gln
   275 280 285
   Asp Ser Lys Asp Ser Thr Tyr Ser Leu Ser Ser Thr Leu Thr Leu Ser
   290 295 300
   Lys Ala Asp Tyr Glu Lys His Lys Val Tyr Ala Cys Glu Val Thr His
   305 310 315 320
   Gln Gly Leu Ser Ser Pro Val Thr Lys Ser Phe Asn Arg Gly Glu Cys
   325 330 335
<210> 58
   <211> 307
   <212> PRT
   <213> Homo sapiens
<400> 58
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Thr Lys Asp Gly Phe His Leu Val Ile Glu Leu Glu
   115 120 125
   Asp Leu Gly Pro Gln Phe Glu Phe Leu Val Ala Tyr Trp Arg Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala Thr Val Ala Ala Pro Ser Val
   195 200 205
   Phe Ile Phe Pro Pro Ser Asp Glu Gln Leu Lys Ser Gly Thr Ala Ser
   210 215 220
   Val Val Cys Leu Leu Asn Asn Phe Tyr Pro Arg Glu Ala Lys Val Gln
   225 230 235 240
   Trp Lys Val Asp Asn Ala Leu Gln Ser Gly Asn Ser Gln Glu Ser Val
   245 250 255
   Thr Glu Gln Asp Ser Lys Asp Ser Thr Tyr Ser Leu Ser Ser Thr Leu
   260 265 270
   Thr Leu Ser Lys Ala Asp Tyr Glu Lys His Lys Val Tyr Ala Cys Glu
   275 280 285
   Val Thr His Gln Gly Leu Ser Ser Pro Val Thr Lys Ser Phe Asn Arg
   290 295 300
   Gly Glu Cys
   305
<210> 59
   <211> 201
   <212> PRT
   <213> Homo sapiens
<400> 59
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Thr Lys Asp Gly Phe His Leu Val Ile Glu Leu Glu
   115 120 125
   Asp Leu Gly Pro Gln Phe Glu Phe Leu Val Ala Tyr Trp Arg Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala
   195 200
<210> 60
   <211> 323
   <212> PRT
   <213> Homo sapiens
<400> 60
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Pro Lys His Gly Phe His Leu Val Ile Glu Leu Glu
   115 120 125
   Asp Leu Gly Pro Gln Phe Glu Phe Leu Val Ala Tyr Trp Thr Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala Gly Gly Gly Gly Ser Gly Gly
   195 200 205
   Gly Gly Ser Gly Gly Gly Gly Ser Arg Thr Val Ala Ala Pro Ser Val
   210 215 220
   Phe Ile Phe Pro Pro Ser Asp Glu Gln Leu Lys Ser Gly Thr Ala Ser
   225 230 235 240
   Val Val Cys Leu Leu Asn Asn Phe Tyr Pro Arg Glu Ala Lys Val Gln
   245 250 255
   Trp Lys Val Asp Asn Ala Leu Gln Ser Gly Asn Ser Gln Glu Ser Val
   260 265 270
   Thr Glu Gln Asp Ser Lys Asp Ser Thr Tyr Ser Leu Ser Ser Thr Leu
   275 280 285
   Thr Leu Ser Lys Ala Asp Tyr Glu Lys His Lys Val Tyr Ala Cys Glu
   290 295 300
   Val Thr His Gln Gly Leu Ser Ser Pro Val Thr Lys Ser Phe Asn Arg
   305 310 315 320
   Gly Glu Cys
<210> 61
   <211> 201
   <212> PRT
   <213> Homo sapiens
<400> 61
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Pro Lys His Gly Phe His Leu Val Ile Glu Leu Glu
   115 120 125
   Asp Leu Gly Pro Gln Phe Glu Phe Leu Val Ala Tyr Trp Thr Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala
   195 200
<210> 62
   <211> 559
   <212> PRT
   <213> Homo sapiens
<400> 62
   Val Pro Cys Val Ser Gly Gly Leu Pro Lys Pro Ala Asn Ile Thr
   Phe
   1 5 10 15
   Leu Ser Ile Asn Met Lys Asn Val Leu Gln Trp Thr Pro Pro Glu
   Gly
   20 25 30
   Leu Gln Gly Val Lys Val Thr Tyr Thr Val Gln Tyr Phe Ile Tyr
   Gly
   35 40 45
   Gln Lys Lys Trp Leu Asn Lys Ser Glu Cys Arg Asn Ile Asn Arg
   Thr
   50 55 60
   Tyr Cys Asp Leu Ser Ala Glu Thr Ser Asp Tyr Glu His Gln Tyr
   Tyr
   65 70 75
   80
   Ala Lys Val Lys Ala Ile Trp Gly Thr Lys Cys Ser Lys Trp Ala
   Glu
   85 90 95
   Ser Gly Arg Phe Tyr Pro Phe Leu Glu Thr Gln Ile Gly Pro Pro
   Glu
   100 105 110
   Val Ala Leu Thr Thr Asp Glu Lys Ser Ile Ser Val Val Leu Thr
   Ala
   115 120 125
   Pro Glu Lys Trp Lys Arg Asn Pro Glu Asp Leu Pro Val Ser Met
   Gln
   130 135 140
   Gln Ile Tyr Ser Asn Leu Lys Tyr Asn Val Ser Val Leu Asn Thr
   Lys
   145 150 155
   160
   Ser Asn Arg Thr Trp Ser Gln Cys Val Thr Asn His Thr Leu Val
   Leu
   165 170 175
   Thr Trp Leu Glu Pro Asn Thr Leu Tyr Cys Val His Val Glu Ser
   Phe
   180 185 190
   Val Pro Gly Pro Pro Arg Arg Ala Gln Pro Ser Glu Lys Gln Cys
   Ala
   195 200 205
   Arg Thr Leu Lys Asp Gln Gly Gly Gly Gly Ser Gly Gly Gly Gly
   Ser
   210 215 220
   Gly Gly Gly Gly Ser Ala Ser Thr Lys Gly Pro Ser Val Phe Pro
   Leu
   225 230 235
   240
   Ala Pro Ser Ser Lys Ser Thr Ser Gly Gly Thr Ala Ala Leu Gly
   Cys
   245 250 255
   Leu Val Lys Asp Tyr Phe Pro Glu Pro Val Thr Val Ser Trp Asn
   Ser
   260 265 270
   Gly Ala Leu Thr Ser Gly Val His Thr Phe Pro Ala Val Leu Gln
   Ser
   275 280 285
   Ser Gly Leu Tyr Ser Leu Ser Ser Val Val Thr Val Pro Ser Ser
   Ser
   290 295 300
   Leu Gly Thr Gln Thr Tyr Ile Cys Asn Val Asn His Lys Pro Ser
   Asn
   305 310 315
   320
   Thr Lys Val Asp Lys Lys Val Glu Pro Lys Ser Cys Asp Lys Thr
   His
   325 330 335
   Thr Cys Pro Pro Cys Pro Ala Pro Glu Ala Glu Gly Ala Pro Ser
   Val
   340 345 350
   Phe Leu Phe Pro Pro Lys Pro Lys Asp Thr Leu Met Ile Ser Arg
   Thr
   355 360 365
   Pro Glu Val Thr Cys Val Val Val Asp Val Ser His Glu Asp Pro
   Glu
   370 375 380
   Val Lys Phe Asn Trp Tyr Val Asp Gly Val Glu Val His Asn Ala
   Lys
   385 390 395
   400
   Thr Lys Pro Arg Glu Glu Gln Tyr Asn Ser Thr Tyr Arg Val Val
   Ser
   405 410 415
   Val Leu Thr Val Leu His Gln Asp Trp Leu Asn Gly Lys Glu Tyr
   Lys
   420 425 430
   Cys Lys Val Ser Asn Lys Ala Leu Pro Ser Ser Ile Glu Lys Thr
   Ile
   435 440 445
   Ser Lys Ala Lys Gly Gln Pro Arg Glu Pro Gln Val Tyr Thr Leu
   Pro
   450 455 460
   Pro Ser Arg Asp Glu Leu Thr Lys Asn Gln Val Ser Leu Thr Cys
   Leu
   465 470 475
   480
   Val Lys Gly Phe Tyr Pro Ser Asp Ile Ala Val Glu Trp Glu Ser
   Asn
   485 490 495
   Gly Gln Pro Glu Asn Asn Tyr Lys Thr Thr Pro Pro Val Leu Asp
   Ser
   500 505 510
   Asp Gly Ser Phe Phe Leu Tyr Ser Lys Leu Thr Val Asp Lys Ser
   Arg
   515 520 525
   Trp Gln Gln Gly Asn Val Phe Ser Cys Ser Val Met His Glu Ala Leu
   530 535 540
   His Asn His Tyr Thr Gln Lys Ser Leu Ser Leu Ser Pro Gly Lys
   545 550 555
<210> 63
   <211> 214
   <212> PRT
   <213> Homo sapiens
<400> 63
   Val Pro Cys Val Ser Gly Gly Leu Pro Lys Pro Ala Asn Ile Thr Phe
   1 5 10 15
   Leu Ser Ile Asn Met Lys Asn Val Leu Gln Trp Thr Pro Pro Glu Gly
   20 25 30
   Leu Gln Gly Val Lys Val Thr Tyr Thr Val Gln Tyr Phe Ile Tyr Gly
   35 40 45
   Gln Lys Lys Trp Leu Asn Lys Ser Glu Cys Arg Asn Ile Asn Arg Thr
   50 55 60
   Tyr Cys Asp Leu Ser Ala Glu Thr Ser Asp Tyr Glu His Gln Tyr Tyr
   65 70 75 80
   Ala Lys Val Lys Ala Ile Trp Gly Thr Lys Cys Ser Lys Trp Ala Glu
   85 90 95
   Ser Gly Arg Phe Tyr Pro Phe Leu Glu Thr Gln Ile Gly Pro Pro Glu
   100 105 110
   Val Ala Leu Thr Thr Asp Glu Lys Ser Ile Ser Val Val Leu Thr Ala
   115 120 125
   Pro Glu Lys Trp Lys Arg Asn Pro Glu Asp Leu Pro Val Ser Met Gln
   130 135 140
   Gln Ile Tyr Ser Asn Leu Lys Tyr Asn Val Ser Val Leu Asn Thr Lys
   145 150 155 160
   Ser Asn Arg Thr Trp Ser Gln Cys Val Thr Asn His Thr Leu Val Leu
   165 170 175
   Thr Trp Leu Glu Pro Asn Thr Leu Tyr Cys Val His Val Glu Ser Phe
   180 185 190
   Val Pro Gly Pro Pro Arg Arg Ala Gln Pro Ser Glu Lys Gln Cys Ala
   195 200 205
   Arg Thr Leu Lys Asp Gln
   210
<210> 64
   <211> 19
   <212> PRT
   <213> Homo sapiens
<400> 64
   Glu Glu Ile His Ala Glu Leu Arg Arg Phe Arg Arg Val Pro Cys Val
   1 5 10 15
   Ser Gly Gly
<210> 65
   <211> 207
   <212> PRT
   <213> Homo sapiens
<400> 65
   Leu Pro Lys Pro Ala Asn Ile Thr Phe Leu Ser Ile Asn Met Lys
   Asn
    1               5                   10                  15
   Val Leu Gln Trp Thr Pro Pro Glu Gly Leu Gln Gly Val Lys Val
   Thr
                20                  25                  30
   Tyr Thr Val Gln Tyr Phe Ile Tyr Gly Gln Lys Lys Trp Leu Asn
   Lys
            35                  40                  45
   Ser Glu Cys Arg Asn Ile Asn Arg Thr Tyr Cys Asp Leu Ser Ala
   Glu
        50                  55                  60
   Thr Ser Asp Tyr Glu His Gln Tyr Tyr Ala Lys Val Lys Ala Ile
   Trp
    65                  70                  75
   80
   Gly Thr Lys Cys Ser Lys Trp Ala Glu Ser Gly Arg Phe Tyr Pro
   Phe
                    85                  90                  95
   Leu Glu Thr Gln Ile Gly Pro Pro Glu Val Ala Leu Thr Thr Asp
   Glu
                100                 105                 110
   Lys Ser Ile Ser Val Val Leu Thr Ala Pro Glu Lys Trp Lys Arg
   Asn
            115                 120                 125
   Pro Glu Asp Leu Pro Val Ser Met Gln Gln Ile Tyr Ser Asn Leu
   Lys
        130                 135                 140
   Tyr Asn Val Ser Val Leu Asn Thr Lys Ser Asn Arg Thr Trp Ser
   Gln
    145                 150                 155
   160
   Cys Val Thr Asn His Thr Leu Val Leu Thr Trp Leu Glu Pro Asn
   Thr
                    165                 170                 175
   Leu Tyr Cys Val His Val Glu Ser Phe Val Pro Gly Pro Pro Arg
   Arg
                180                 185                 190
   Ala Gln Pro Ser Glu Lys Gln Cys Ala Arg Thr Leu Lys Asp Gln
           195                 200                 205
<210> 66
   <211> 150
   <212> PRT
   <213> Homo sapiens
<400> 66
   Cys Arg Asn Ile Asn Arg Thr Tyr Cys Asp Leu Ser Ala Glu Thr Ser
   1 5 10 15
   Asp Tyr Glu His Gln Tyr Tyr Ala Lys Val Lys Ala Ile Trp Gly Thr
   20 25 30
   Lys Cys Ser Lys Trp Ala Glu Ser Gly Arg Phe Tyr Pro Phe Leu Glu
   35 40 45
   Thr Gln Ile Gly Pro Pro Glu Val Ala Leu Thr Thr Asp Glu Lys Ser
   50 55 60
   Ile Ser Val Val Leu Thr Ala Pro Glu Lys Trp Lys Arg Asn Pro Glu
   65 70 75 80
   Asp Leu Pro Val Ser Met Gln Gln Ile Tyr Ser Asn Leu Lys Tyr Asn
   85 90 95
   Val Ser Val Leu Asn Thr Lys Ser Asn Arg Thr Trp Ser Gln Cys Val
   100 105 110
   Thr Asn His Thr Leu Val Leu Thr Trp Leu Glu Pro Asn Thr Leu Tyr
   115 120 125
   Cys Val His Val Glu Ser Phe Val Pro Gly Pro Pro Arg Arg Ala Gln
   130 135 140
   Pro Ser Glu Lys Gln Cys
   145 150
<210> 67
   <211> 196
   <212> PRT
   <213> Homo sapiens
<400> 67
   Leu Pro Ala Pro Gln Asn Leu Ser Val Leu Ser Thr Asn Met Lys His
   1 5 10 15
   Leu Leu Met Trp Ser Pro Val Ile Ala Pro Gly Glu Thr Val Tyr Tyr
   20 25 30
   Ser Val Glu Tyr Gln Gly Glu Tyr Glu Ser Leu Tyr Thr Ser His
   Ile
   35 40 45
   Trp Ile Pro Ser Ser Trp Cys Ser Leu Thr Glu Gly Pro Glu Cys
   Asp
   50 55 60
   Val Thr Asp Asp Ile Thr Ala Thr Val Pro Tyr Asn Leu Arg Val
   Arg
   65 70 75
   80
   Ala Thr Leu Gly Ser Gln Thr Ser Ala Trp Ser Ile Leu Lys His
   Pro
   85 90 95
   Phe Asn Arg Asn Ser Thr Ile Leu Thr Arg Pro Gly Met Glu Ile
   Thr
   100 105 110
   Lys Asp Gly Phe His Leu Val Ile Glu Leu Glu Asp Leu Gly Pro
   Gln
   115 120 125
   Phe Glu Phe Leu Val Ala Tyr Trp Arg Arg Glu Pro Gly Ala Glu
   Glu
   130 135 140
   His Val Lys Met Val Arg Ser Gly Gly Ile Pro Val His Leu Glu
   Thr
   145 150 155
   160
   Met Glu Pro Gly Ala Ala Tyr Cys Val Lys Ala Gln Thr Phe Val
   Lys
   165 170 175
   Ala Ile Gly Arg Tyr Ser Ala Phe Ser Gln Thr Glu Cys Val Glu
   Val
   180 185 190
   Gln Gly Glu Ala
   195
<210> 68
   <211> 203
   <212> PRT
   <213> Homo sapiens
<400> 68
   Asp Glu Val Ala Ile Leu Pro Ala Pro Gln Asn Leu Ser Val Leu
   Ser
   1 5 10 15
   Thr Asn Met Lys His Leu Leu Met Trp Ser Pro Val Ile Ala Pro
   Gly
   20 25 30
   Glu Thr Val Tyr Tyr Ser Val Glu Tyr Gln Gly Glu Tyr Glu Ser
   Leu
   35 40 45
   Tyr Thr Ser His Ile Trp Ile Pro Ser Ser Trp Cys Ser Leu Thr
   Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Pro Lys His Gly Phe His Leu Val Ile Glu Leu Glu
   115 120 125
   Asp Leu Gly Pro Gln Phe Glu Phe Leu Val Ala Tyr Trp Thr Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala Ile Pro
   195 200
<210> 69
   <211> 196
   <212> PRT
   <213> Homo sapiens
<400> 69
   Leu Pro Ala Pro Gln Asn Leu Ser Val Leu Ser Thr Asn Met Lys His
   1 5 10 15
   Leu Leu Met Trp Ser Pro Val Ile Ala Pro Gly Glu Thr Val Tyr Tyr
   20 25 30
   Ser Val Glu Tyr Gln Gly Glu Tyr Glu Ser Leu Tyr Thr Ser His Ile
   35 40 45
   Trp Ile Pro Ser Ser Trp Cys Ser Leu Thr Glu Gly Pro Glu Cys Asp
   50 55 60
   Val Thr Asp Asp Ile Thr Ala Thr Val Pro Tyr Asn Leu Arg Val Arg
   65 70 75 80
   Ala Thr Leu Gly Ser Gln Thr Ser Ala Trp Ser Ile Leu Lys His Pro
   85 90 95
   Phe Asn Arg Asn Ser Thr Ile Leu Thr Arg Pro Gly Met Glu Ile Pro
   100 105 110
   Lys His Gly Phe His Leu Val Ile Glu Leu Glu Asp Leu Gly Pro Gln
   115 120 125
   Phe Glu Phe Leu Val Ala Tyr Trp Thr Arg Glu Pro Gly Ala Glu Glu
   130 135 140
   His Val Lys Met Val Arg Ser Gly Gly Ile Pro Val His Leu Glu Thr
   145 150 155 160
   Met Glu Pro Gly Ala Ala Tyr Cys Val Lys Ala Gln Thr Phe Val Lys
   165 170 175
   Ala Ile Gly Arg Tyr Ser Ala Phe Ser Gln Thr Glu Cys Val Glu Val
   180 185 190
   Gln Gly Glu Ala
   195
<210> 70
   <211> 135
   <212> PRT
   <213> Homo sapiens
<400> 70
   Cys Ser Leu Thr Glu Gly Pro Glu Cys Asp Val Thr Asp Asp Ile Thr
   1 5 10 15
   Ala Thr Val Pro Tyr Asn Leu Arg Val Arg Ala Thr Leu Gly Ser Gln
   20 25 30
   Thr Ser Ala Trp Ser Ile Leu Lys His Pro Phe Asn Arg Asn Ser Thr
   35 40 45
   Ile Leu Thr Arg Pro Gly Met Glu Ile Thr Lys Asp Gly Phe His Leu
   50 55 60
   Val Ile Glu Leu Glu Asp Leu Gly Pro Gln Phe Glu Phe Leu Val Ala
   65 70 75 80
   Tyr Trp Arg Arg Glu Pro Gly Ala Glu Glu His Val Lys Met Val Arg
   85 90 95
   Ser Gly Gly Ile Pro Val His Leu Glu Thr Met Glu Pro Gly Ala Ala
   100 105 110
   Tyr Cys Val Lys Ala Gln Thr Phe Val Lys Ala Ile Gly Arg Tyr Ser
   115 120 125
   Ala Phe Ser Gln Thr Glu Cys
   130 135
<210> 71
   <211> 135
   <212> PRT
   <213> Homo sapiens
<400> 71
   Cys Ser Leu Thr Glu Gly Pro Glu Cys Asp Val Thr Asp Asp Ile Thr
   1               5                   10                  15
   Ala Thr Val Pro Tyr Asn Leu Arg Val Arg Ala Thr Leu Gly Ser Gln
               20                  25                  30
   Thr Ser Ala Trp Ser Ile Leu Lys His Pro Phe Asn Arg Asn Ser Thr
           35                  40                  45
   Ile Leu Thr Arg Pro Gly Met Glu Ile Pro Lys His Gly Phe His Leu
       50                  55                  60
   Val Ile Glu Leu Glu Asp Leu Gly Pro Gln Phe Glu Phe Leu Val Ala
   65                  70                  75                  80
   Tyr Trp Thr Arg Glu Pro Gly Ala Glu Glu His Val Lys Met Val Arg
                   85                  90                  95
   Ser Gly Gly Ile Pro Val His Leu Glu Thr Met Glu Pro Gly Ala Ala
               100                 105                 110
   Tyr Cys Val Lys Ala Gln Thr Phe Val Lys Ala Ile Gly Arg Tyr Ser
           115                 120                 125
   Ala Phe Ser Gln Thr Glu Cys
       130                 135
<210> 72
   <211> 15
   <212> PRT
   <213> Homo sapiens
<400> 72
   Gly Gly Gly Gly Ser Gly Gly Gly Gly Ser Gly Gly Gly Gly Ser
   1               5                   10                  15